# EUROPÄISCHE PATENTSCHRIFT

(11) **EP 2 651 862 B1**
(45) Veröffentlichungstag und Bekanntmachung des Hinweises auf die Patenterteilung: **10.05.2017**
(21) Anmeldenummer: 11791208.9
(22) Anmeldetag: 17.11.2011
(51) Int. Cl.: C07C 15/28, C07C 43/263, C07D 251/24, C09K 11/06, H01L 51/50, H05B 33/14

(54) **SUBSTITUIERTE TETRAARYLBENZOLE**
SUBSTITUTED TETRAARYLBENZENES
TÉTRAARYLBENZÈNES SUBSTITUÉS

(30) Priorität: 13.12.2010 DE 102010054316
(43) Veröffentlichungstag der Anmeldung: 23.10.2013
(73) Patentinhaber: Merck Patent GmbH, 64293 Darmstadt (DE)
(72) Erfinder: ANÉMIAN, Rémi, Manouk, Seoul 657-169 (KR); LUDEMANN, Aurélie, 60322 Frankfurt am Main (DE); EBERLE, Thomas, 76829 Landau (DE); HOEGER, Sigurd, 53121 Bonn (DE); REIS, Eva, Maria, 64747 Breuberg (DE); BOBBE, Vanessa, 53229 Bonn (DE)
(86) Internationale Anmeldenummer: PCT/EP2011/005810
(87) Internationale Veröffentlichungsnummer: WO 2012/079678

(56) Entgegenhaltungen:
- WO-A1-2009/054253
- WO-A2-02/36641
- WO-A2-2011/108902
- JP-A- 2005 302 657
- MUNAWAR HUSSAIN ET AL: "Synthesis of Aryl-Substituted Pyrimidines by Site-Selective Suzuki-Miyura Cross-Coupling Reactions of 2,4,5,6-Tetrachloropyrimidine", ADVANCED SYNTHESIS & CATALYSIS, Bd. 352, Nr. 9, 1. Juni 2010 (2010-06-01), Seiten 1429-1433, XP55017742, ISSN: 1615-4150, DOI: 10.1002/adsc.201000020
- YASUHIRO FUJIOKA ET AL: "Syntheses and spectral characteristics of seven polyphenyls containing highly branched para-phenylene ring(s).", CHEMICAL & PHARMACEUTICAL BULLETIN, Bd. 33, Nr. 1, 1. Januar 1985 (1985-01-01) , Seiten 22-29, XP55017743, ISSN: 0009-2363, DOI: 10.1248/cpb.33.22
- LUKAS HINTERMANN ET AL: "The AZARYPHOS Family of Ligands for Ambifunctional Catalysis: Syntheses and Use in Ruthenium-Catalyzed anti-Markovnikov Hydration of Terminal Alkynes", CHEMISTRY - A EUROPEAN JOURNAL, Bd. 15, Nr. 29, 20. Juli 2009 (2009-07-20) , Seiten 7167-7179, XP55017744, ISSN: 0947-6539, DOI: 10.1002/chem.200900563
- DATABASE CA [Online] CHEMICAL ABSTRACTS SERVICE, COLUMBUS, OHIO, US; 1969, STRZELECKA, HELENA ET AL: "Pyrylium salts. VI. Formation and the nature of a bipyrylium cation. 2,2',4,6,6'-Pentaphenyl-3,4'-bipyrylium diperchlorate", XP002668186, gefunden im STN Database accession no. 1969:57581 & STRZELECKA, HELENA ET AL: "Pyrylium salts. VI. Formation and the nature of a bipyrylium cation. 2,2',4,6,6'-Pentaphenyl-3,4'-bipyrylium diperchlorate", BULLETIN DE LA SOCIETE CHIMIQUE DE FRANCE , (10), 4122-3 CODEN: BSCFAS; ISSN: 0037-8968, 1968,

## Beschreibung

Die vorliegende Erfindung betrifft organische Elektrolumineszenzvorrichtungen sowie Materialien für die Verwendung in organischen Elektrolumineszenzvorrichtungen.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei neben fluoreszierenden Emittern zunehmend metallorganische Komplexe eingesetzt, die Phosphoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich. Generell gibt es sowohl bei OLEDs, die Singulettemission zeigen, wie auch bei OLEDs, die Triplettemission zeigen, immer noch Verbesserungsbedarf, insbesondere im Hinblick auf Effizienz, Betriebsspannung und Lebensdauer. Dies gilt insbesondere für OLEDs, welche im kürzerwelligen Bereich, also grün und insbesondere blau, emittieren.
Die Eigenschaften von OLEDs werden nicht nur durch die eingesetzten Die Eigenschaften von OLEDs werden nicht nur durch die eingesetzten Emitter bestimmt. Hier sind insbesondere auch die anderen verwendeten Materialien, wie Host- und Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können somit auch zu deutlichen Verbesserungen der OLED-Eigenschaften führen.

Gemäß dem Stand der Technik werden unter anderem Ketone (z. B. gemäß WO 2004/093207 oder WO 2010/006680) oder Phosphinoxide (z. B. gemäß WO 2005/003253) als Matrixmaterialien für phosphoreszierende Emitter verwendet. Weitere Matrixmaterialien gemäß dem Stand der Technik repräsentieren Triazine (bspw. WO 2008/056746, EP 0906947, EP 0908787, EP 0906948).

Für fluoreszierende OLEDs werden gemäß dem Stand der Technik vor allem kondensierte Aromaten, insbesondere Anthracenderivate, als Host-Materialien vor allem für blau emittierende Elektrolumineszenzvorrichtungen verwendet, z. B. 9,10-Bis(2-naphthyl)anthracen (US 5935721). In WO 03/095445 und in CN 1362464 werden 9,10-Bis-(1-naphthyl)anthracen-Derivate für die Verwendung in OLEDs offenbart. Weitere Anthracenderivate sind in WO 01/076323, in WO 01/021729, in WO 2004/013073, in WO 2004/018588, in WO 2003/087023 oder in WO 2004/018587 offenbart. Host-Materialien, basierend auf Arylsubstituierten Pyrenen und Chrysenen, werden in WO 2004/016575 offenbart. Host-Materialien basierend auf Benzanthracenderivaten werden in WO 2008/145239 offenbart. Es ist für hochwertige Anwendungen wünschenswert, verbesserte Host-Materialien zur Verfügung zu haben.

Allerdings besteht bei Verwendung dieser Host- und Matrixmaterialien ebenso wie bei anderen Host- und Matrixmaterialien noch Verbesserungsbedarf, insbesondere in Bezug auf die Effizienz und die Lebensdauer der Vorrichtung.

Obwohl die auf kleinen Molekülen basierenden OLEDs (SMOLEDs) teilweise recht gute Effizienzen, Lebensdauern und/oder Betriebsspannung zeigen, sind thermische Aufdampfungsmethoden im Vakuum nötig, die auf eine bestimmte Device-Größe beschränkt sind. Für die Massenproduktion und für größere Displays ist es allerdings wünschenswert, die organischen Materialien aus Lösung aufzubringen, z.B. mittels Spin-coating- oder Inkjet-Verfahren, wodurch zusätzlich die Produktionskosten gesenkt werden können. Zumeist werden lichtemittierende Polymere, Oligomere und/oder Dendrimere verwendet, um elektrolumineszierende Vorrichtungen aus Lösung zu prozessieren. Diese Verbindungen zeigen oft eine gute Löslichkeit in organischen aromatischen Lösemitteln und weisen gute Filmbildungseigenschaften auf. Eine weitere Möglichkeit die Verarbeitbarkeit zu verbessern, besteht darin, lange Alkyl-Ketten als löslichkeitsvermittelnde Gruppen in ein Molekül einzubauen. Leider weisen die aus Lösung prozessierten Vorrichtungen unter Verwendung von Polymeren, Oligomeren und/oder Dendrimeren oder Molekülen mit Alkyl-Ketten meist eine schlechtere Performance auf, als vergleichbare kleine Moleküle, was Effizienz, Lebensdauer und Betriebsspannung betrifft.

Aufgabe der vorliegenden Erfindung ist die Bereitstellung von Verbindungen, welche sich für den Einsatz in einer fluoreszierenden oder phosphoreszierenden OLED eignen, beispielsweise als Host- und/oder Matrixmaterial oder als Lochtransport-/Elektronenblockiermaterial bzw. Exzitonenblockiermaterial oder als Elektronentransport- bzw. Lochblockiermaterial, und welche bei Verwendung in einer OLED zu guten Device-Eigenschaften führen, sowie die Bereitstellung der entsprechenden elektronischen Vorrichtung.
£Eine weitere Aufgabe der vorliegenden Erfindung besteht darin, die Eine weitere Aufgabe der vorliegenden Erfindung besteht darin, die Moleküle bereitzustellen, die eine verbesserte Löslichkeit aufweisen und daher bei der Herstellung einer lichtemittierenden Vorrichtung aus Lösung prozessiert werden können Noch eine weitere Aufgabe der vorliegenden Erfindung besteht in der Bereitstellung von Molekülen, die sich besonders gut dazu eignen, lichtemittierende Vorrichtungen aus der Gasphase herzustellen, d.h. Moleküle bereitzustellen, die besonders gut aufgedampft werden können.
Überraschend wurde gefunden, dass bestimmte, unten näher beschriebene Verbindungen diese Aufgaben lösen und zu guten Eigenschaften der organischen Elektrolumineszenzvorrichtung führen, insbesondere hinsichtlich der Lebensdauer, der Effizienz und der Betriebsspannung. Elektronische Vorrichtungen, insbesondere organische Elektrolumineszenzvorrichtungen, welche derartige Verbindungen enthalten, sowie die entsprechenden bevorzugten Verbindungen sind daher der Gegenstand der vorliegenden Erfindung.

Gegenstand der vorliegenden Erfindung sind Verbindungen gemäß Anspruch 1.

"Vernetzbare Gruppe" in Sinne der vorliegenden Erfindung bedeutet eine funktionelle Gruppe, die in der Lage ist, irreversibel zu reagieren. Dadurch wird ein vernetztes Material gebildet, das unlöslich ist. Die Vernetzung kann gewöhnlich durch Wärme oder durch UV-, Mikrowellen-, Röntgen-oder Elektronenstrahlung unterstützt werden. Durch die hohe Stabilität des erfindungsgemäßen Polymers kommt es bei der Vernetzung zu weniger Nebenproduktbildung. Zudem vernetzen die vernetzbaren Gruppen im erfindungsgemäßen Polymer sehr leicht, so dass geringere Energiemengen für die Vernetzung erforderlich sind (z.B. < 200°C bei der thermischen Vernetzung).

Beispiele für vernetzbare Gruppen Q sind Einheiten, die eine Doppelbindung, eine Dreifachbindung, eine Vorstufe, die zu einer in situ Bildung einer Doppel- bzw. Dreifachbindung in der Lage ist, oder einen heterocyclischen additionspolymerisierbaren Rest enthalten. Bevorzugte Reste Q umfassen Vinyl, Alkenyl, vorzugsweise Ethenyl und Propenyl, C₄₋₂₀-Cycloalkenyl, Azid, Oxiran, Oxetan, Di(hydrocarbyl)amino, Cyanatester, Hydroxy, Glycidylether, C₁₋₁₀-Alkylacrylat, C₁₋₁₀-Alkylmethacrylat, Alkenyloxy, vorzugsweise Ethenyloxy, Perfluoralkenyloxy, vorzugsweise Perfluorethenyloxy, Alkinyl, vorzugsweise Ethinyl, Maleimid, Tri(C₁₋₄)-alkylsiloxy und Tri(C₁₋₄)-alkylsilyl. Besonders bevorzugt ist Vinyl und Alkenyl.

Eine Arylgruppe im Sinne dieser Erfindung enthält 6 bis 40 C-Atome; eine Heteroarylgruppe im Sinne dieser Erfindung enthält 1 bis 39 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 60 C-Atome im Ringsystem. Ein heteroaromatisches Ringsystem im Sinne dieser Erfindung enthält 1 bis 59 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verknüpft sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe unterbrochen sind. Weiterhin sollen Systeme, in denen mehrere Aryl- und/oder Heteroarylgruppen durch eine Einfachbindung miteinander verknüpft sind, wie z. B. Biphenyl, Terphenyl oder Bipyridin, als aromatisches bzw. heteroaromatisches Ringsystem verstanden werden.

Im Rahmen der vorliegenden Erfindung werden unter einem aliphatischen Kohlenwasserstoffrest bzw. einer Alkylgruppe bzw. einer Alkenyl- oder Alkinylgruppe, die typischerweise 1 bis 40 oder auch 1 bis 20 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch R¹C=CR¹, C=C, Si(R¹)₂, Ge(R¹)₂, Sn(R¹)₂, C=O, C=S, C=Se, C=NR¹, P(=O)(R¹), SO, SO₂, NR¹, O, S oder CONR¹ ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 aromatischen Ringatomen, welches noch jeweils mit den oben genannten Resten R¹ oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Weiterhin bevorzugte Ausführungsformen der Verbindungen sind dabei solche, bei denen neben dem Rest Z nur ein Rest R¹ im Molekül vorkommt, wobei der Rest R in jeder möglichen Position der Ringe auftreten kann. Insbesondere bevorzugt sind dabei die Verbindungen der Formel (30) bis (52).

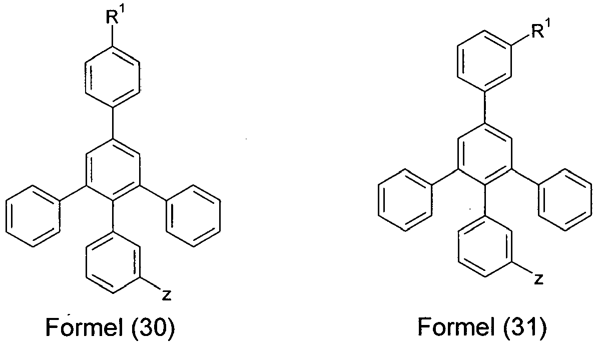

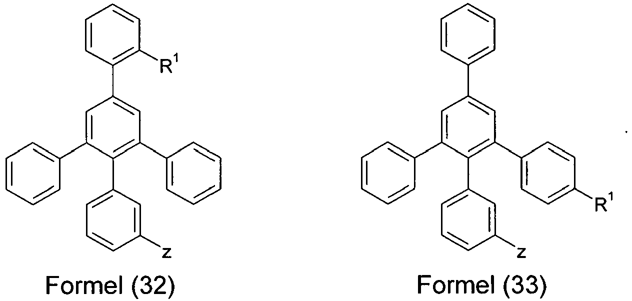

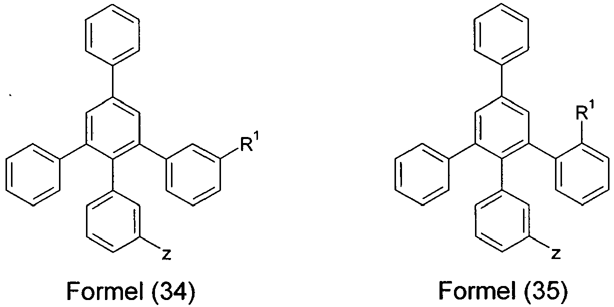

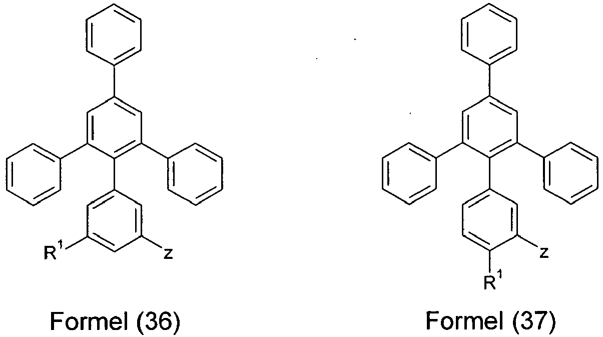

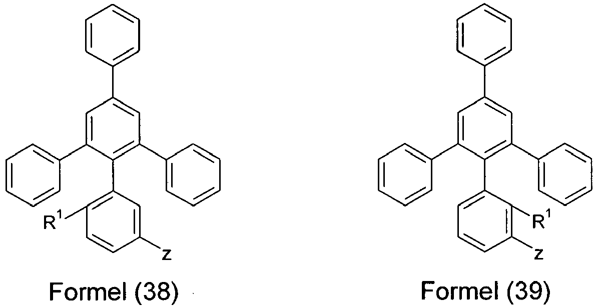

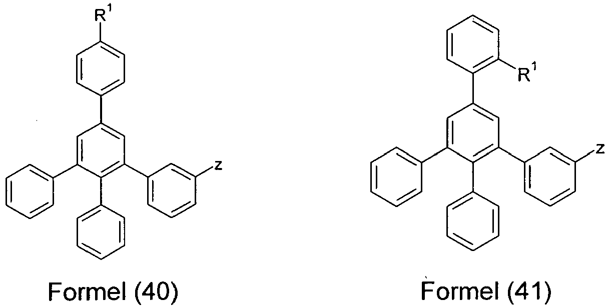

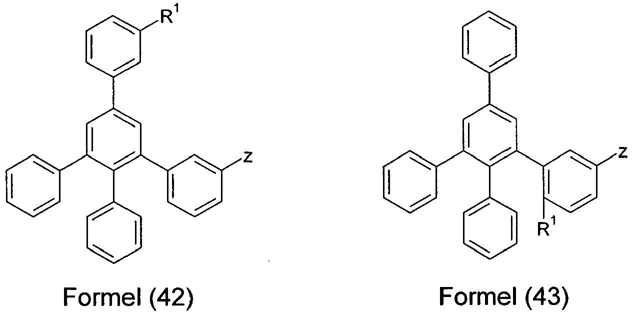

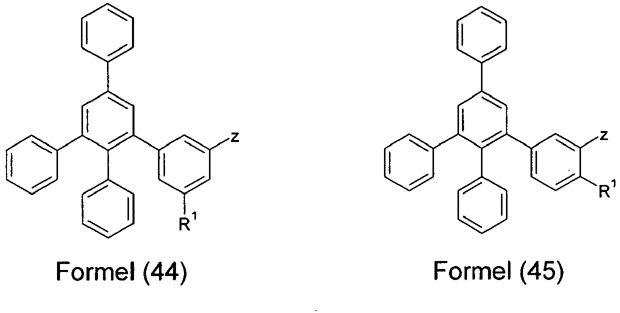

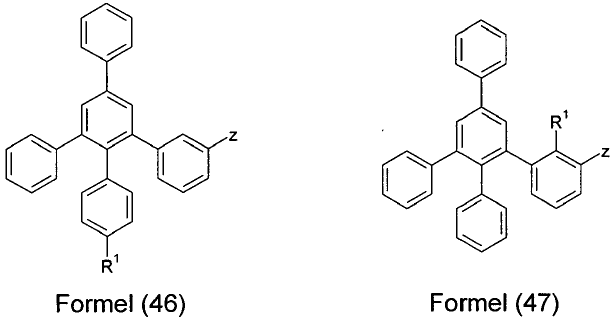

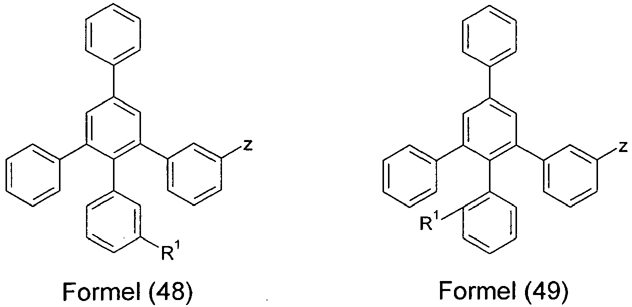

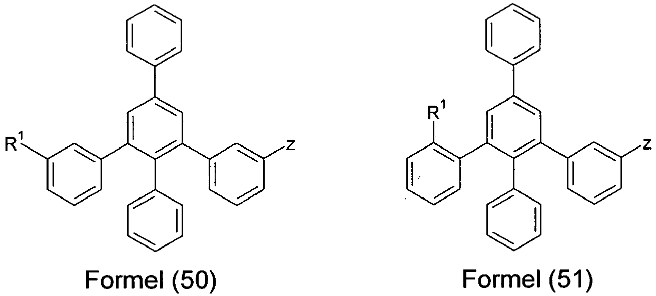

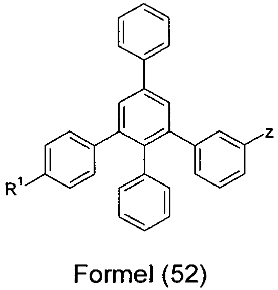

In einer insbesondere bevorzugten Ausführungsform der vorliegenden Erfindung enthält die Verbindung der Formeln (27) oder (28) nur einen Substituenten Z, der sich immer in meta Position zum zentralen Ring befindet (s. Formeln (63) und (64))

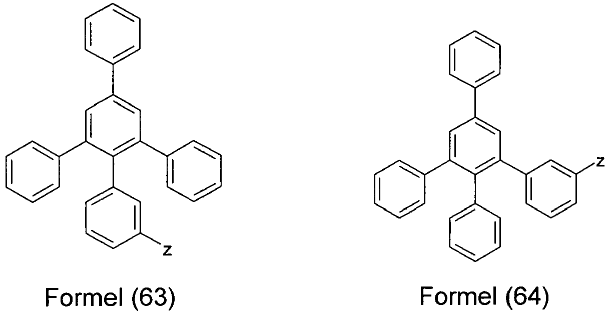

In einer bevorzugten Ausführungsform der vorliegenden Erfindung ist R¹ ist gleich oder verschieden bei jedem Auftreten H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C=C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy-, Arylalkoxy- oder Heteroaryloxy-gruppe mit 5 bis 60 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R² substituiert sein kann, oder eine Kombination aus zwei oder mehr dieser Gruppen.

Weiterhin bevorzugt im Sinne der vorliegenden Erfindung ist, wenn R¹ gleich oder verschieden bei jedem Auftreten H, D, F, Cl, Br, I, N(R²)₂, CN, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C=C, Si(R²)₂, Sn(R²)₂, C=O, C=S, C=NR², P(=O)(R²), SO₂, NR², O, S oder CONR² ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy-, Arylalkoxy- oder Heteroaryloxygruppe mit 5 bis 60 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R² substituiert sein kann, oder eine Kombination aus zwei oder mehr dieser Gruppen ist.

Wenn die Verbindung der Formeln (1) bis (65) als Triplettmatrixmaterial, insbesondere für Emitter, die grünes oder blaues Licht emittieren, eingesetzt wird und der Rest R¹ für ein aromatisches bzw. heteroaromatisches Ringsystem steht, so ist es bevorzugt, wenn dieser keine Arylgruppen mit mehr als zwei kondensierten Arylringen enthält. Diese Bevorzugung erklärt sich aus dem niedrigen Triplett-Niveau von Arylgruppen mit mehr als zwei kondensierten Arylringen, wodurch sich derartige Verbindungen weniger als Triplettmatrixmaterial eignen. Besonders bevorzugt enthält das aromatische bzw. heteroaromatische Ringsystem keine kondensierten Arylgruppen. Bevorzugte aromatische bzw. heteroaromatische Ringsysteme R¹ sind für die Verwendung als Triplettmatrixmaterial daher aufgebaut aus jeweils einer oder mehreren der Gruppen Benzol, Pyridin, Pyrimidin, Pyridazin, Pyrazin, Triazin, Pyrrol, Thiophen, Furan, Naphthalin, Chinolin, Isochinolin, Chinoxalin, Indol, Benzothiophen oder Benzofuran, welches jeweils mit einem oder mehreren Resten R² substituiert sein kann. Besonders bevorzugte Gruppen Z sind aufgebaut aus jeweils einer oder mehreren Gruppen Benzol, Pyridin, Pyrimidin, Pyridazin, Pyrazin oder Triazin, welches jeweils mit einem oder mehreren Resten R² substituiert sein kann, insbesondere Benzol, welches mit einem oder mehreren Resten R² substituiert sein kann. Weitere bevorzugte Gruppen R¹ sind für die Verwendung als Triplettmatrixmaterial Triphenylen und Carbazol. Ebenso bevorzugt sind Kombinationen der als bevorzugt aufgeführten Aryl- und Heteroarylgruppen. Wird die Verbindung gemäß Formel (1) in einer anderen Funktion verwendet, beispielsweise als Singulett Hostmaterial und/oder Elektronentransportmaterial, so können bevorzugte Gruppen R¹ auch größere kondensierte Aryl- oder Heteroarylgruppen enthalten, beispielsweise Anthracen, Pyren oder Perylen, welches jeweils mit einem oder mehreren Resten R² substituiert sein kann.

Der Rest Z ist ausgewählt aus den Formeln (99) bis (117) und (121) bis (289), wobei die angegebenen Formeln ihrerseits mit einem oder mehreren Resten R³ substituiert sein können, die bei jedem Auftreten gleich oder verschieden sein können und mit der Maßgabe, dass wenigstens einer der Reste Z eine aromatische oder heteroaromatische Gruppe mit 5 bis 60 aromatischen Ringatomen sein muss.. 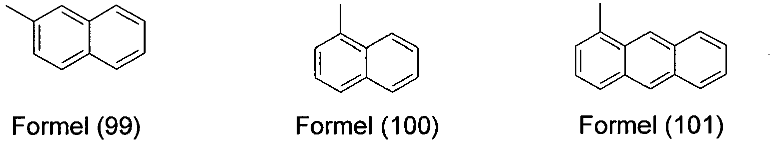 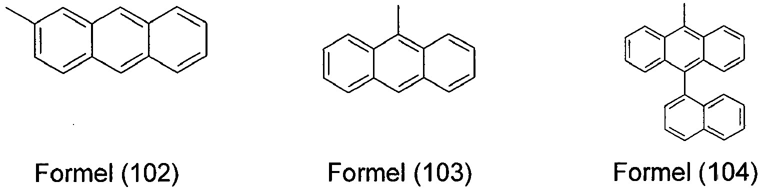 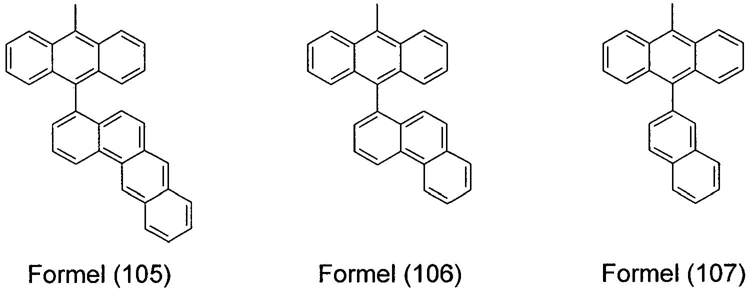 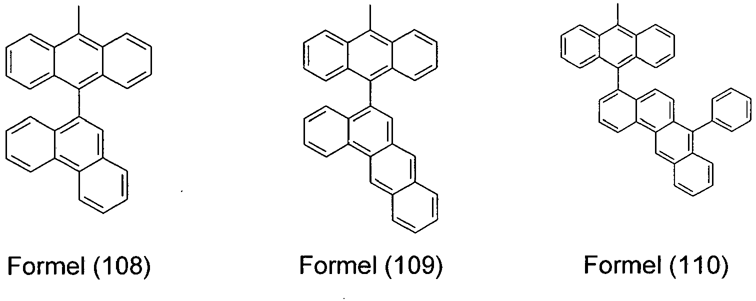 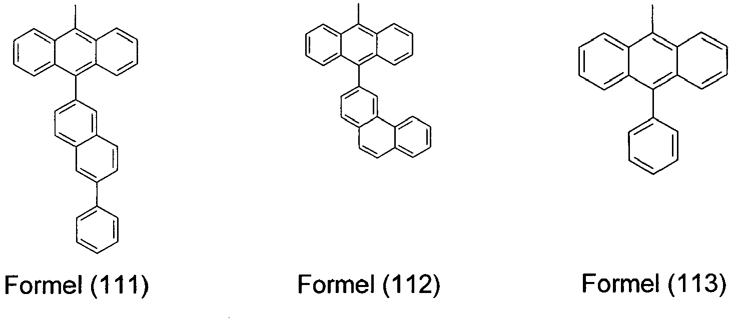 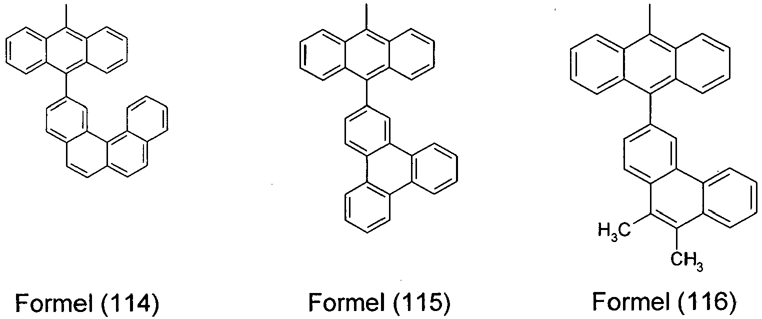 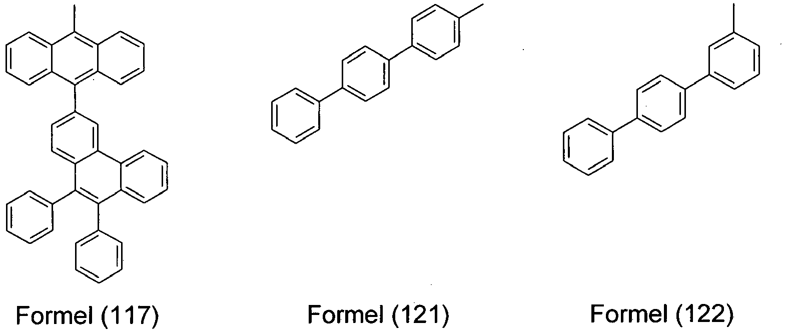 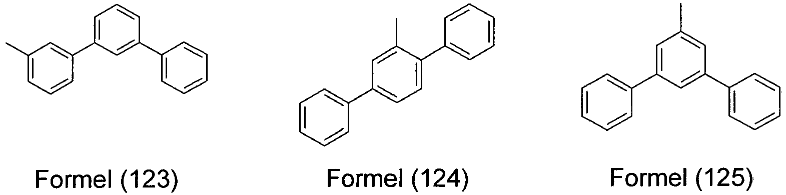 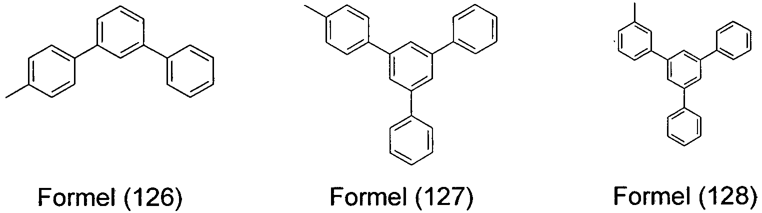 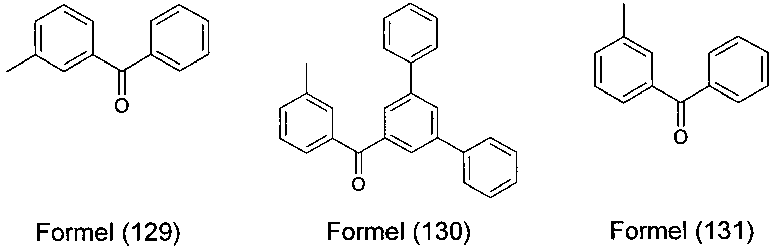 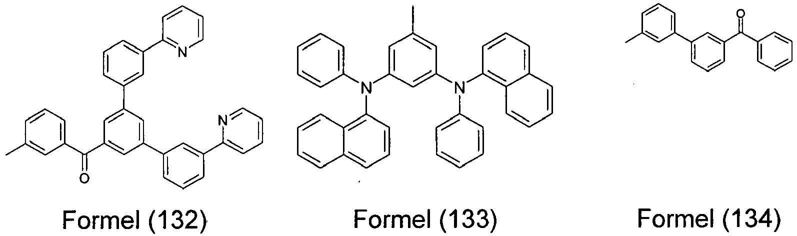 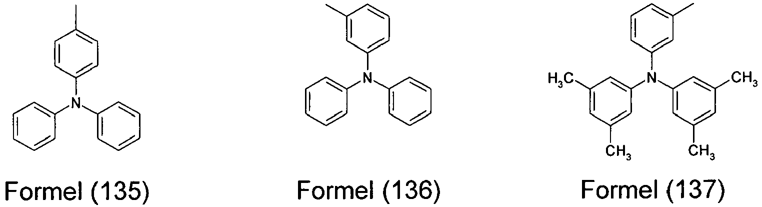 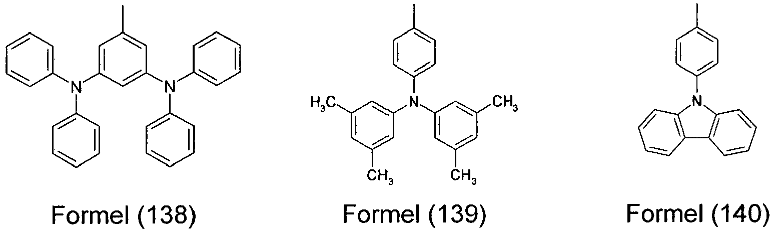 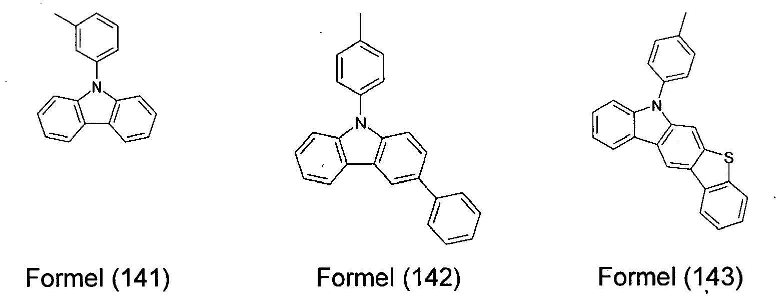 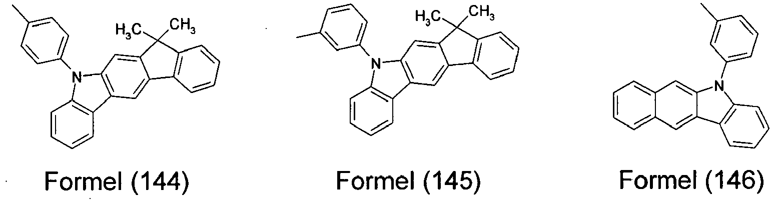 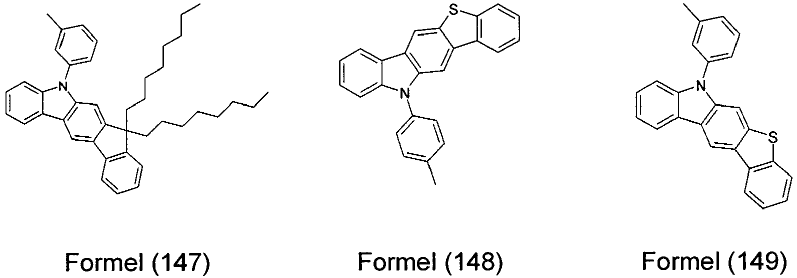 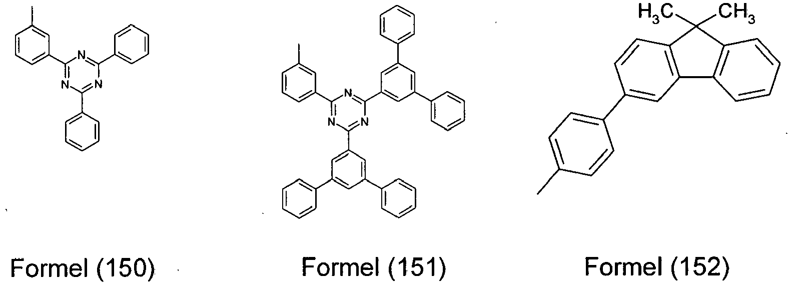 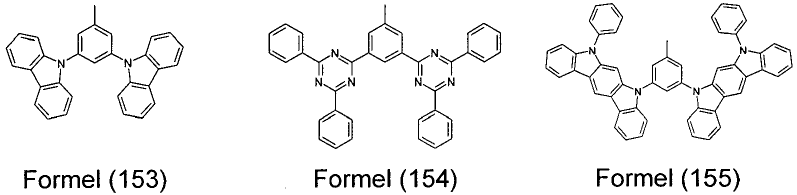 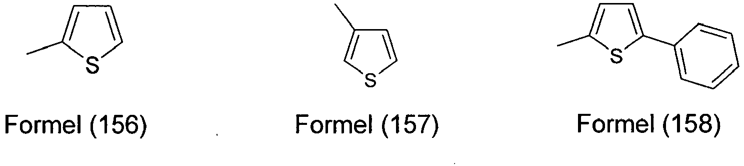 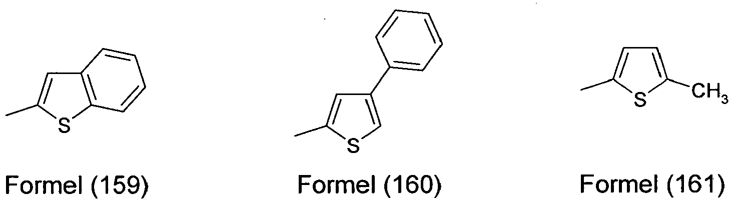 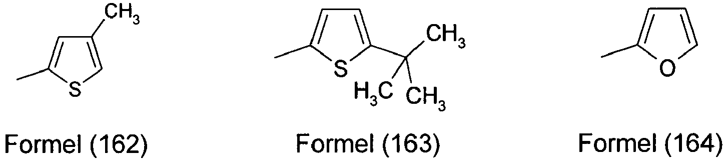 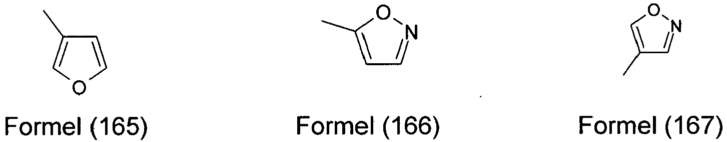 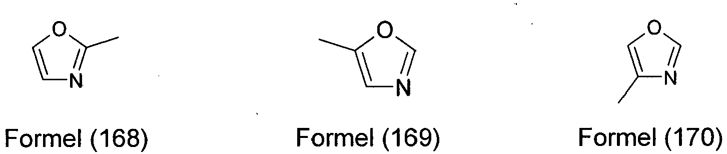 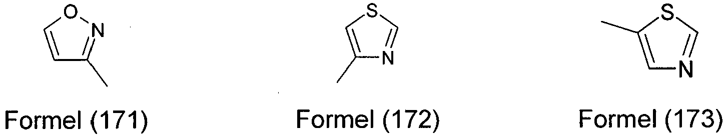 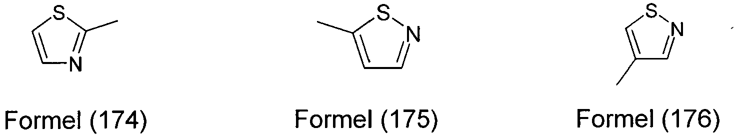 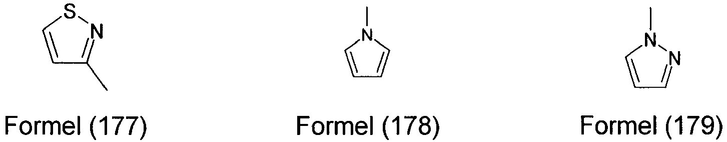 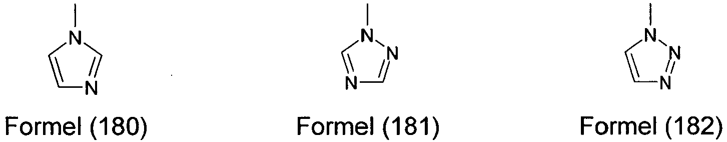 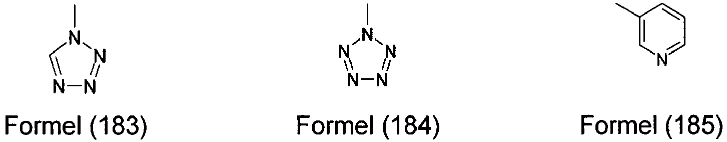 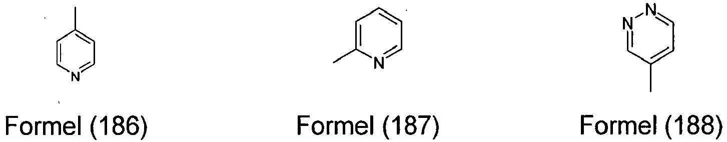 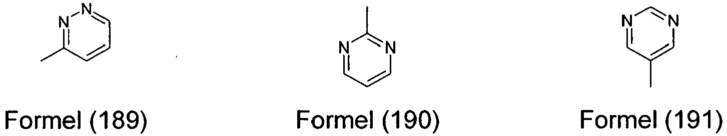 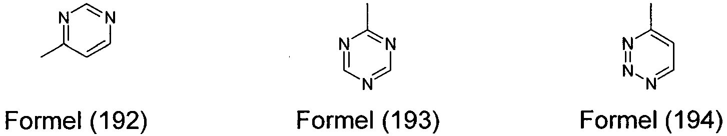 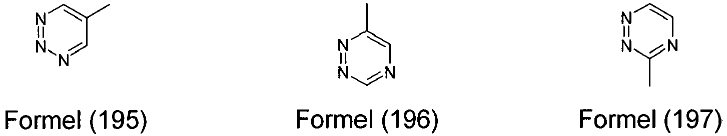 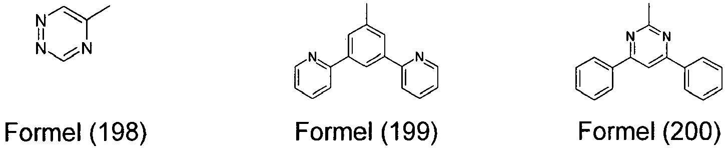 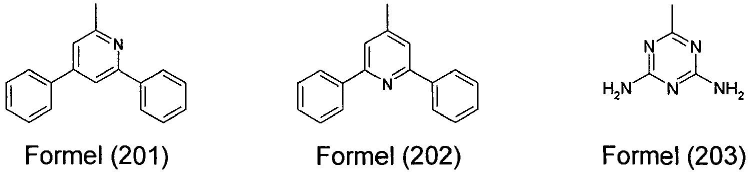 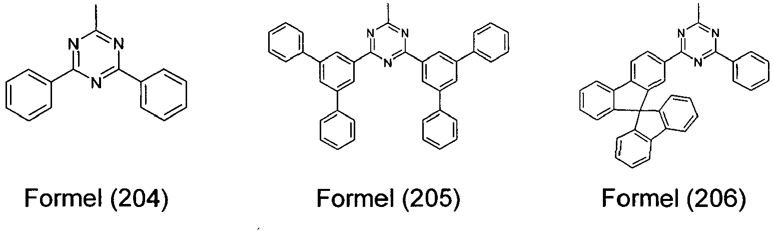 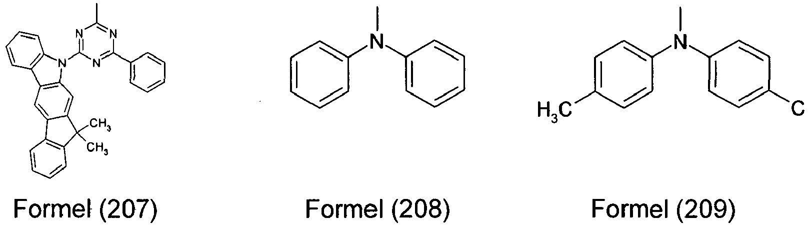 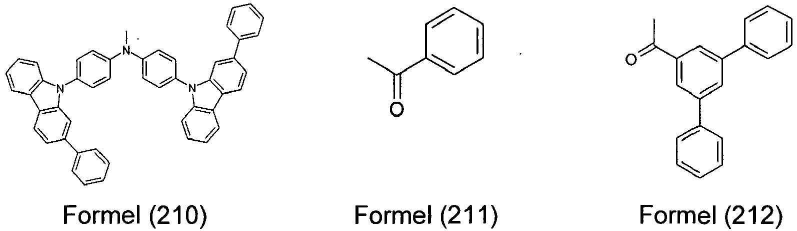 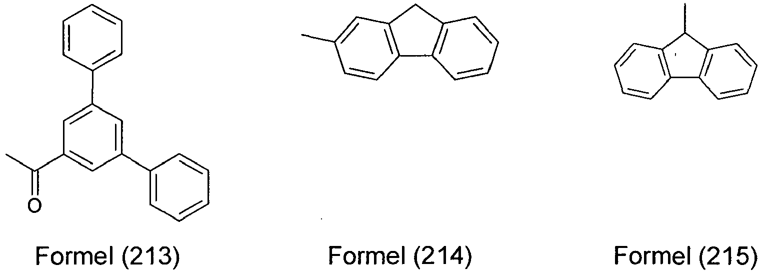 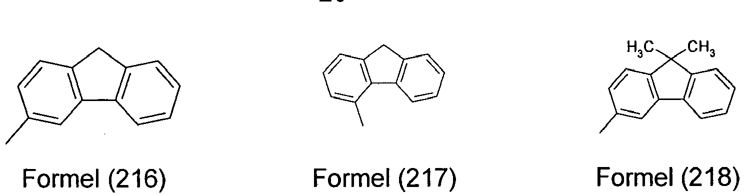 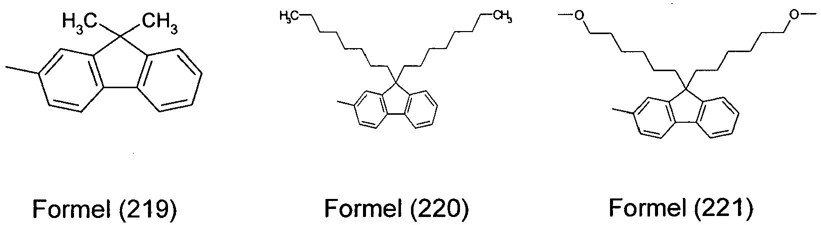 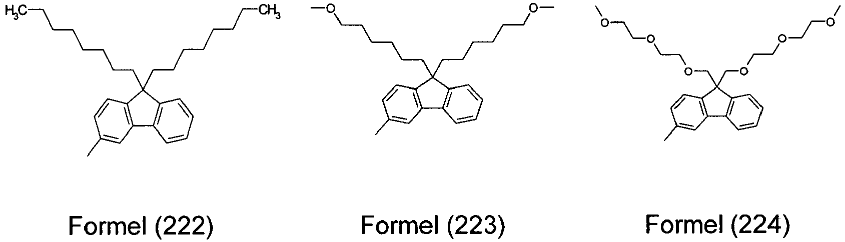 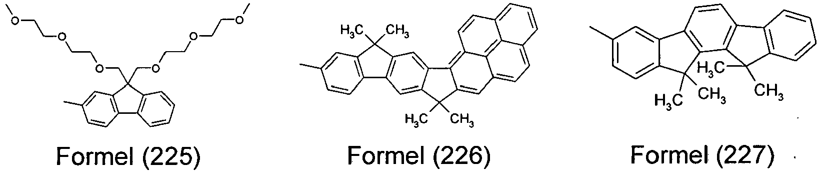 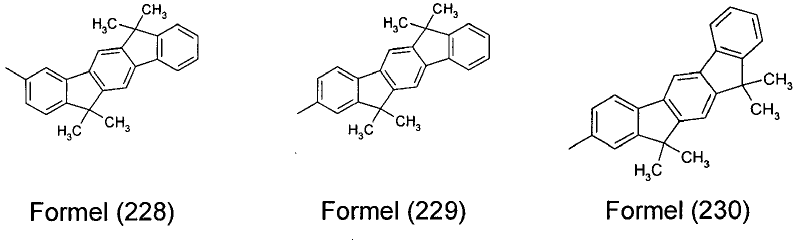 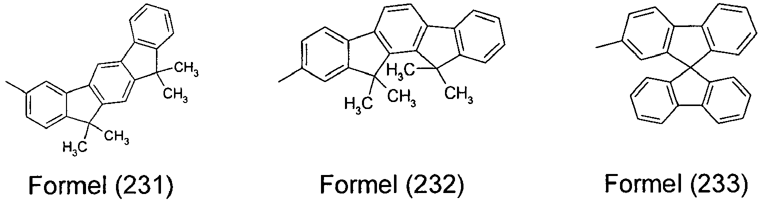 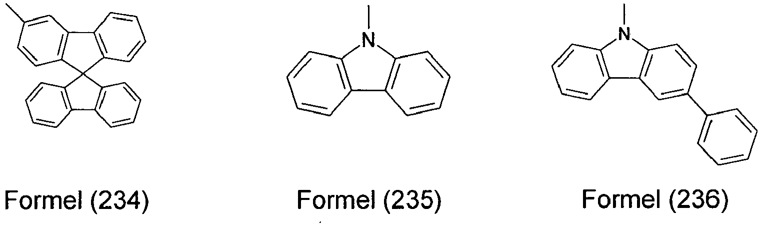 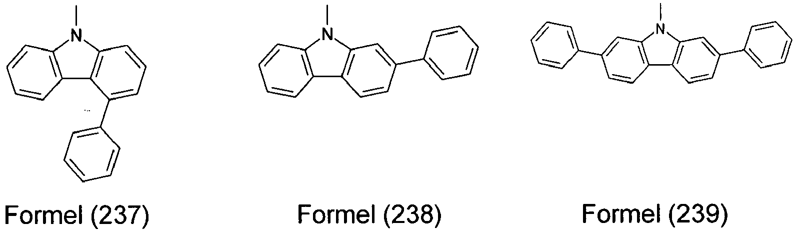 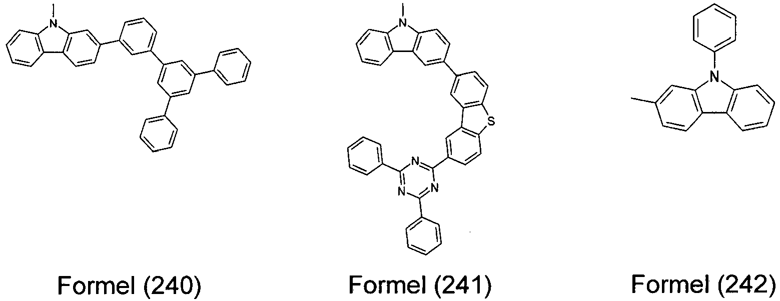 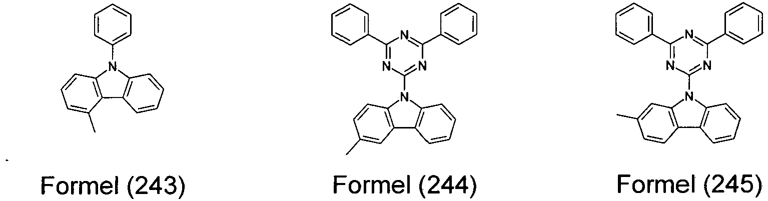 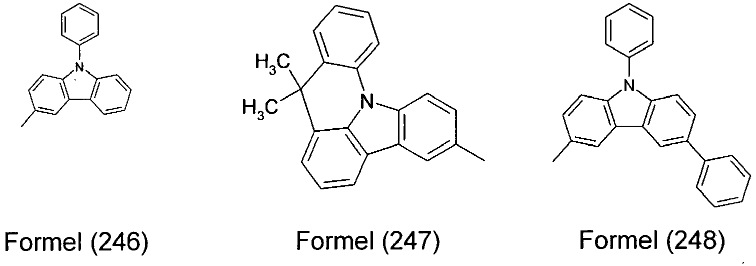 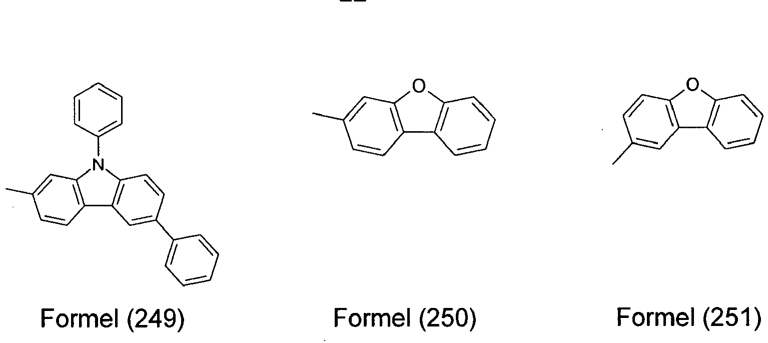 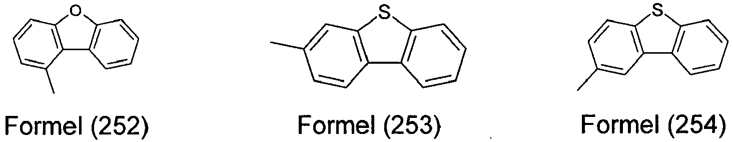 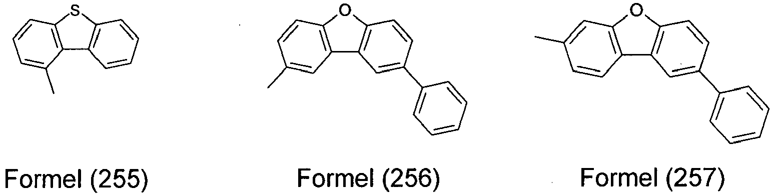 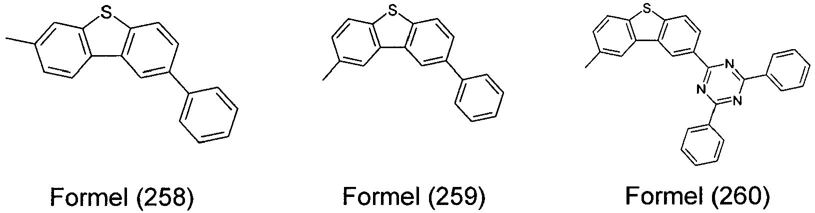 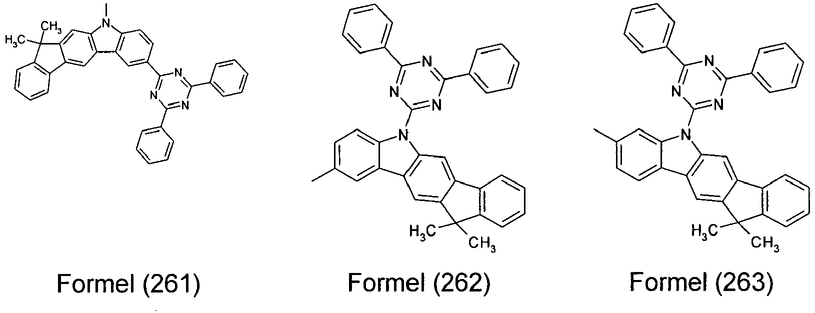 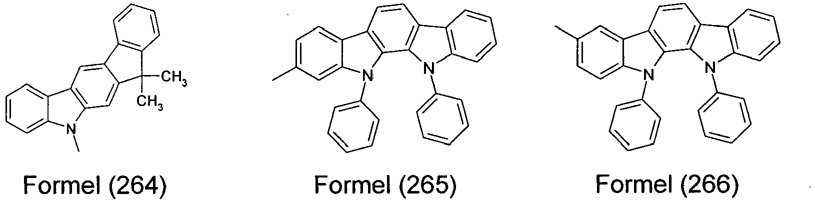 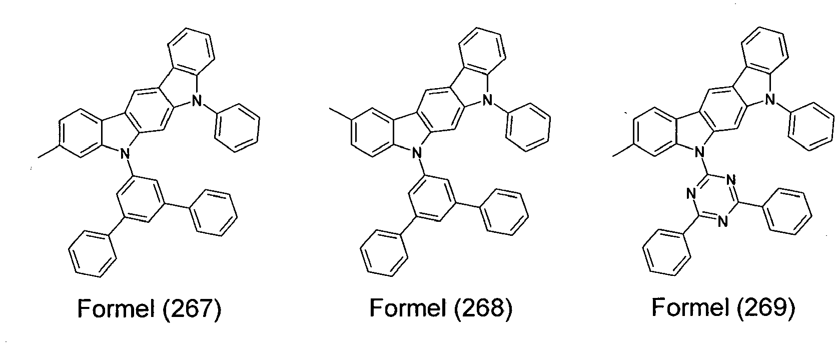 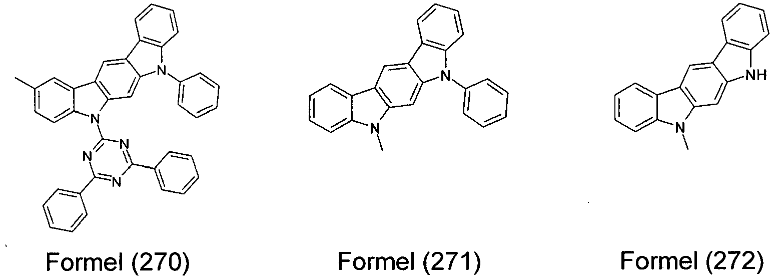 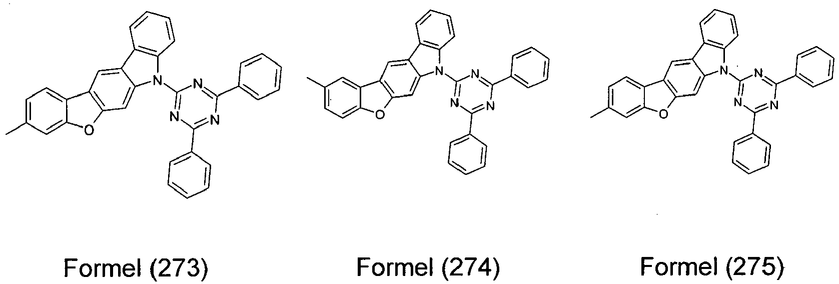 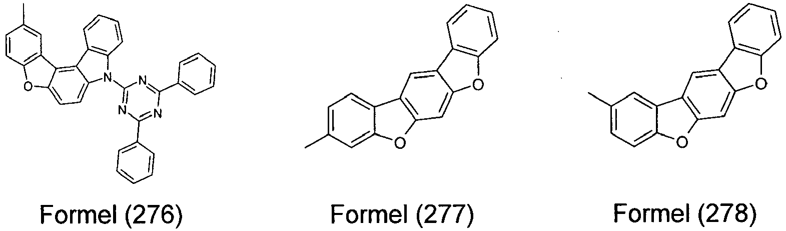 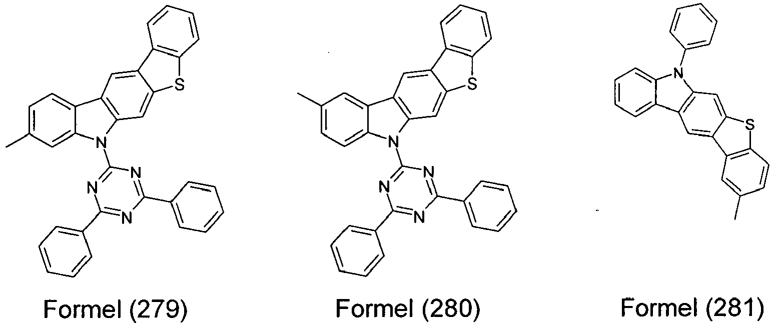 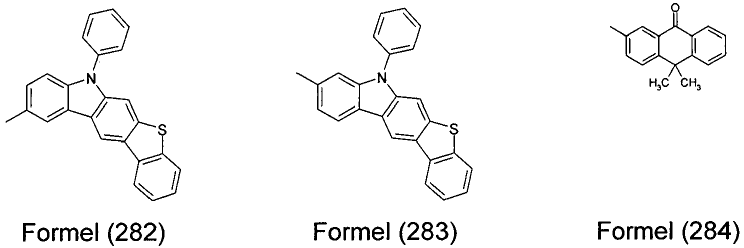 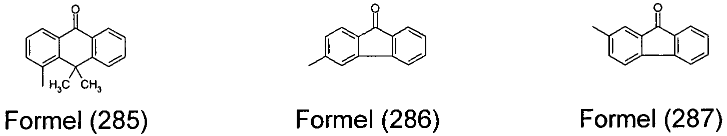 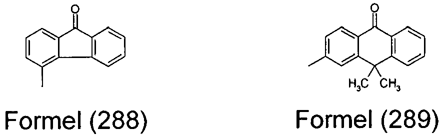 wobei der Bindungsstrich die Position der Verknüpfung des Restes Z kennzeichnet.

Die Verbindungen der folgenden Formel (1) können auch als Monomere der allgemeinen Formel (290) zur Herstellung von Oligomeren, Dendrimeren und Polymeren verwendet werden. Dabei kann das Polymer enthaltend die Verbindung nach Formel (1) sowohl ein Homo- als auch ein Copolymer sein. 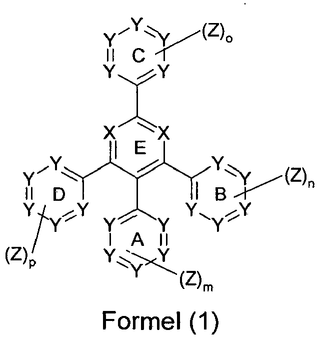 wobei für die verwendeten Symbole und Indizes gilt:
- X: ist bei jedem Auftreten gleich oder verschieden CH oder N;
- Y: ist bei jedem Auftreten gleich oder verschieden CR¹, N, P oder PR¹₂;
- n: ist eine ganze Zahl von 0 bis 5, wobei wenn n größer oder gleich 1 ist die n Substituenten Z an Y = CR¹ gebunden sind und hierbei jeweils den Rest R¹ ersetzten;
- m: ist eine ganze Zahl von 0 bis 5, wobei wenn m größer oder gleich 1 ist die m Substituenten Z an Y = CR¹ gebunden sind und hierbei jeweils den Rest R¹ ersetzten;
- o: ist eine ganze Zahl von 0 bis 5, wobei wenn o größer oder gleich 1 ist die o Substituenten Z an Y = CR¹ gebunden sind und hierbei jeweils den Rest R¹ ersetzten;
- p: ist eine ganze Zahl von 0 bis 5 , wobei wenn p größer oder gleich 1 ist die p Substituenten Z an Y = CR¹ gebunden sind und hierbei jeweils den Rest R¹ ersetzten;
wobei die folgende Bedingung erfüllt sein muss: m+n+o+p=1, 2, 3 oder 4;
und wobei wenigstens einer der Ringe A, B, C oder D im meta Position mit einem Substituenten Z substituiert sein muss.

Die Monomere weisen eine Struktur der folgenden Formel (290) auf 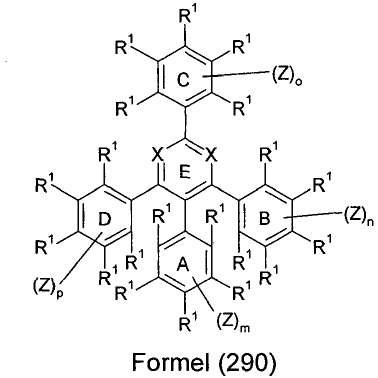
wobei für die verwendeten Symbole und Indizes obige Definitionen (s. Formel (1)) gelten
und wenigstens einer der Ringe A, B, C oder D im meta Position mit einem Substituenten Z substituiert sein muss
und mit der Maßgabe, dass wenigstens einer der Reste Z eine aromatische oder heteroaromatische Gruppe mit 5 bis 60 aromatischen Ringatomen sein muss
und wobei der Substituent bzw. die Substituenten Z jeweils den bzw. die Reste R¹ ersetzen und
zwei oder mehr der Reste R¹ gleich oder verschieden funktionelle Gruppen sind, die unter Bedingungen der C-C- bzw. C-N-Verknüpfungen polymerisieren.

Vorzugsweise sind die funktionellen Gruppen ausgewählt aus Cl, Br, I, O-Tosylat, O-Triflat, O-SO₂R², B(OR²)₂ und Sn(R²)₃, besonders bevorzugt aus Br, I und B(OR²)₂, wobei R² bei jedem Auftreten gleich oder verschieden H, ein aliphatischer oder aromatischer Kohlenwasserstoffrest mit 1 bis 20 C-Atomen ist, und wobei zwei oder mehrere Reste R² auch miteinander ein Ringsystem bilden können.

Die Monomere können weiterhin, wie oben beschrieben, vernetzbare Gruppen Q enthalten, so dass die Polymere enthaltend die Monomere der Formel (290) vernetzt werden können.

Die Synthese der Verbindungen nach Formel (1) mit X = CH erfolgt durch Palladium-katalysierte Kreuz-Kupplungen an den entsprechend halogenierten Grundkörper mit der allgemeinen Formel (291). 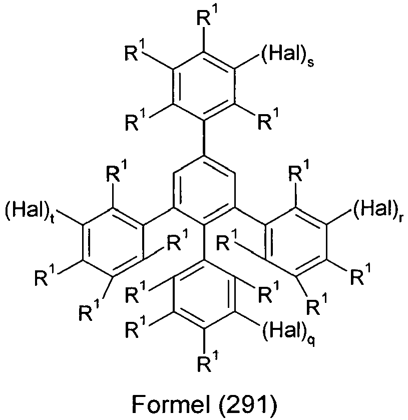 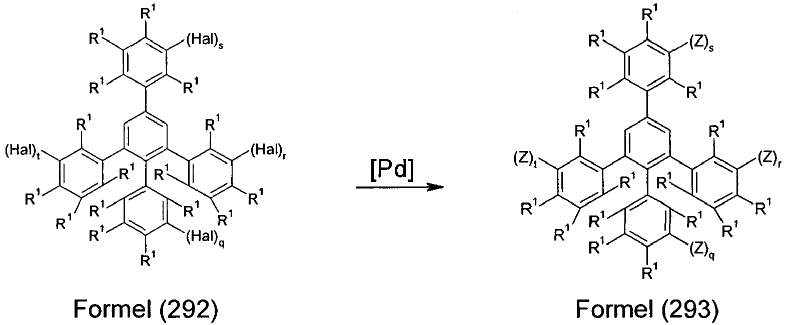 wobei für die verwendeten Symbole und Indizes obige Definitionen gelten. Die Grundkörper lassen sich nach allgemeinen Verfahren entsprechend dem folgenden Schema aus Pyrylium-Salzen (Journal f. Prakt. Chemie 1987, 329, 6, 975-984) darstellen.

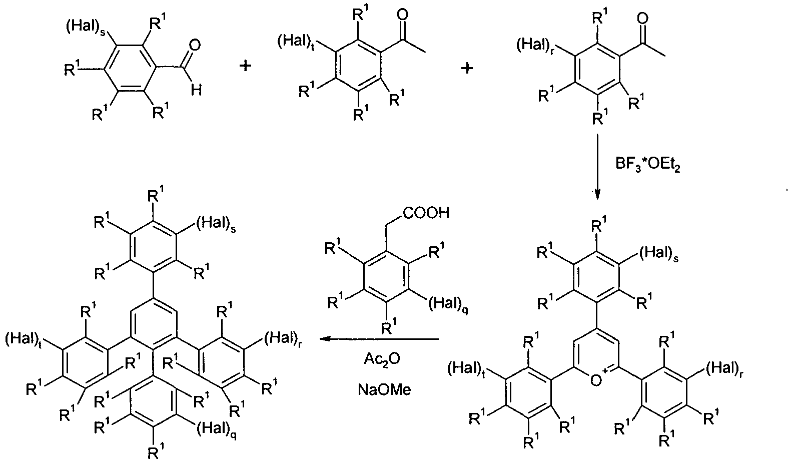

Die Synthese symmetrisch substituierter Verbindungen der Formel (1) mit X = N erfolgt durch Palladium-katalysierte Suzuki-Miyaura-Kupplungen an kommerziell erhältliches 2,4,5,6-Tetrachlorpyrimidin (Adv. Synth. Catal. 2010, 352, 1429-1433).

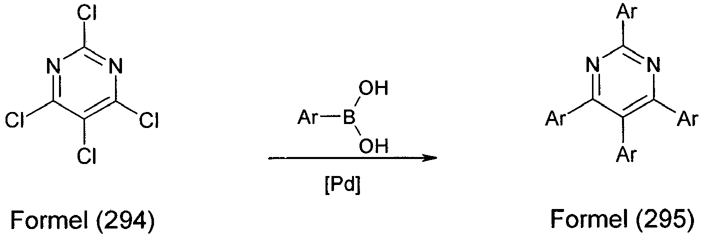

Die Synthese unsymmetrisch substituierter Verbindungen der Formel (1) mit X = N erfolgt bevorzugt durch Bortrifluorid-katalysierte [2+2'+2']-Cycloaddition von Alkinen und Nitrilen (Synthesis 1983, 9, 717-718) nach dem folgenden Schema:

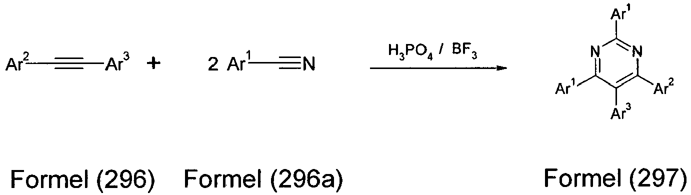

Eine weitere Möglichkeit unsymmetrisch substituierte Verbindungen der Formel (1) mit X = N herzustellen, ist das in Tetrahedron Letters 2005, 46, 1663-1665 beschriebene und in dem folgenden Schema skizzierte Herstellverfahren. 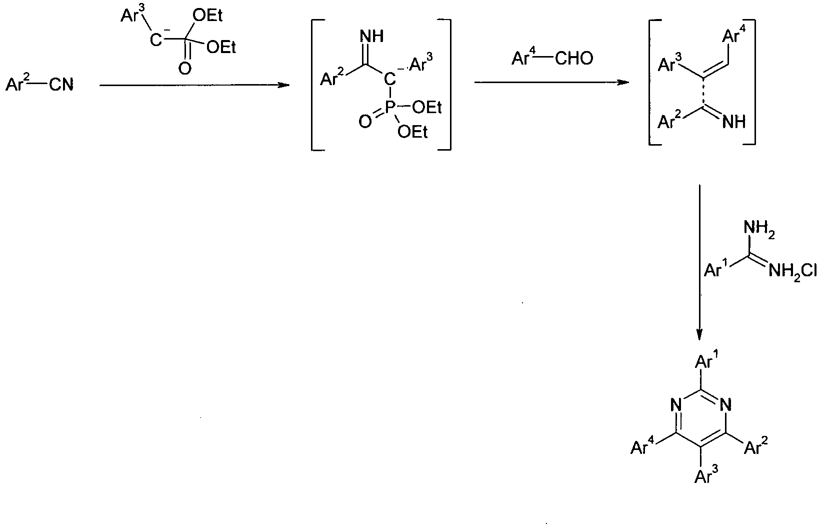 wobei die Reste Ar, Ar¹, Ar², Ar³ und Ar⁴ für die substituierten oder unsubstituierten aromatischen Reste A, B, C und D der Verbindungen der Formeln (1) bis (15) stehen.

Die vorliegenden Erfindung bezieht sich daher auch auf die Herstellung der erfindungsgemäßen Verbindungen nach einem der vorstehenden Verfahren.

Beispiele für Verbindungen gemäß den oben aufgeführten Ausführungsformen, wie sie bevorzugt in organischen elektronischen Vorrichtungen eingesetzt werden können, sind die Verbindungen der folgenden Strukturen (298) bis (425).

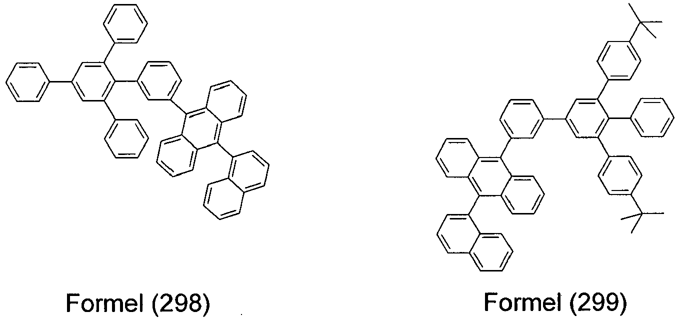

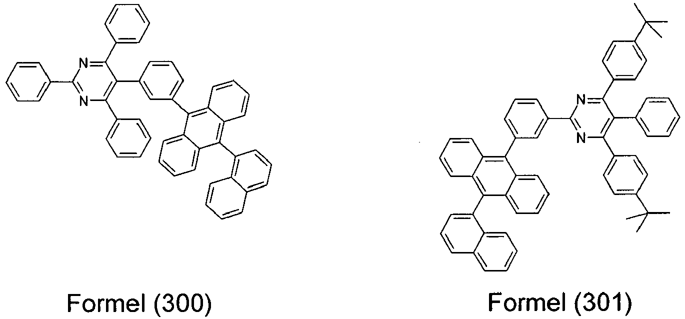

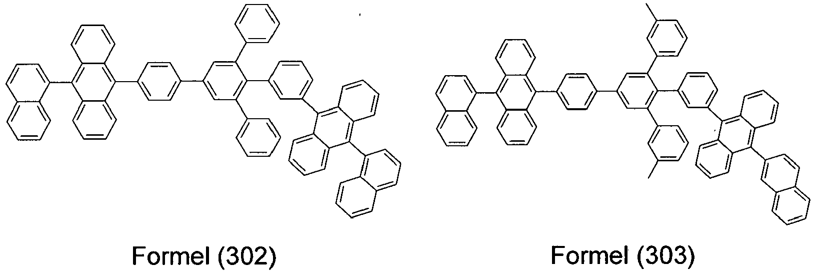

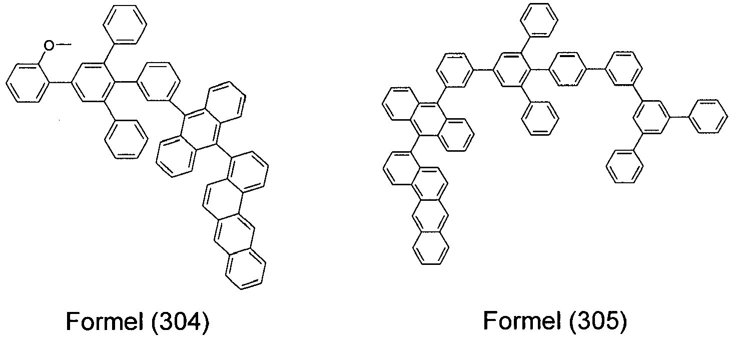

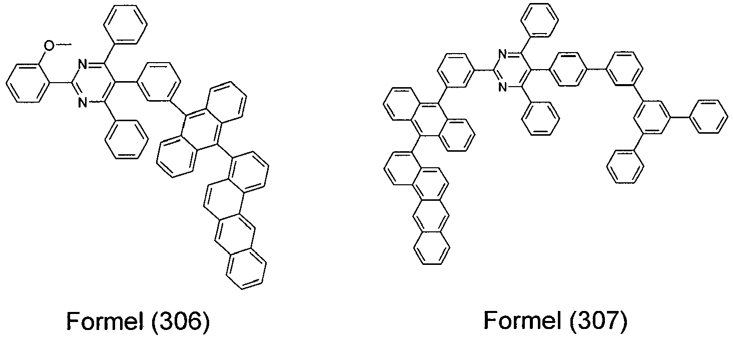

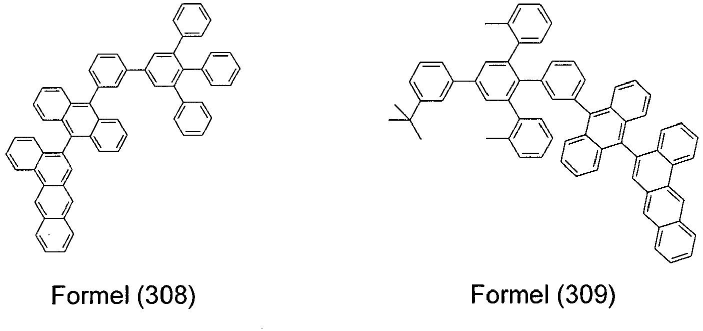

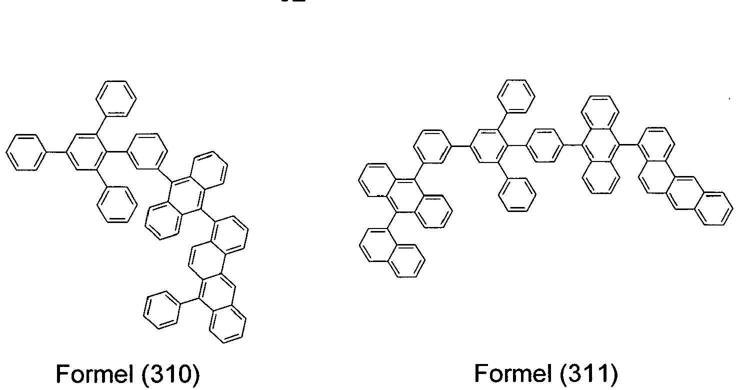

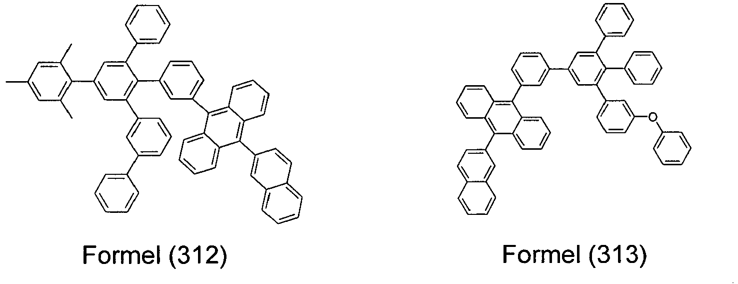

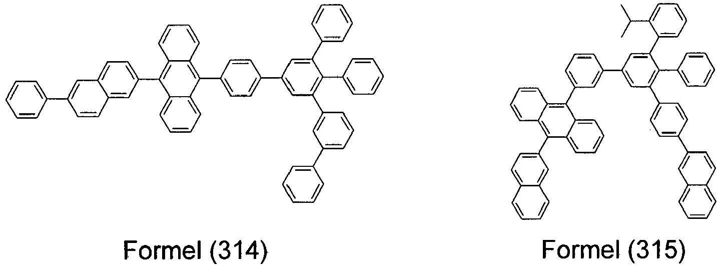

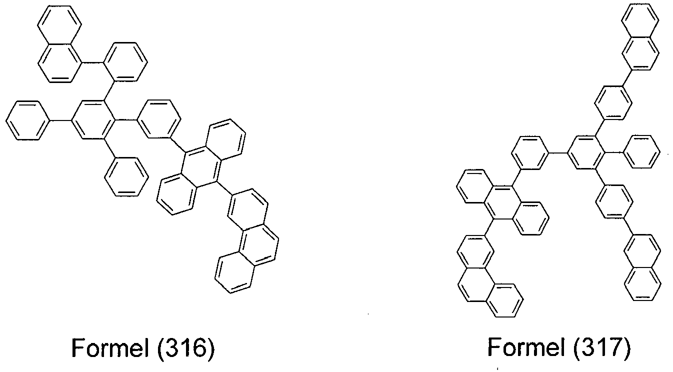

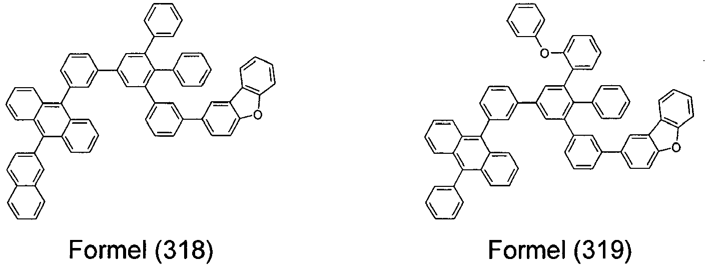

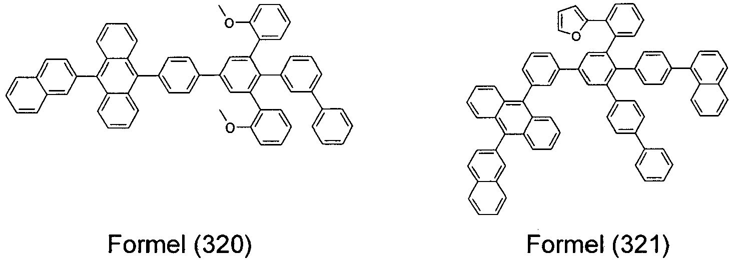

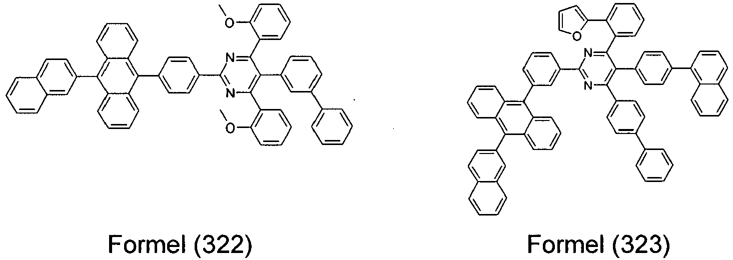

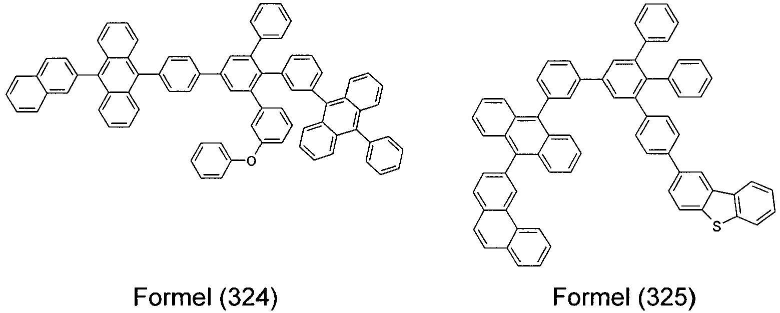

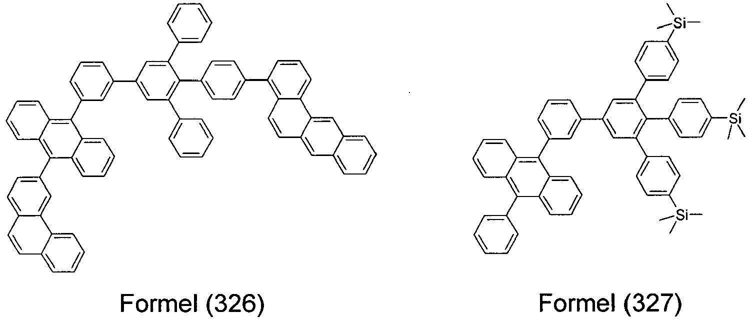

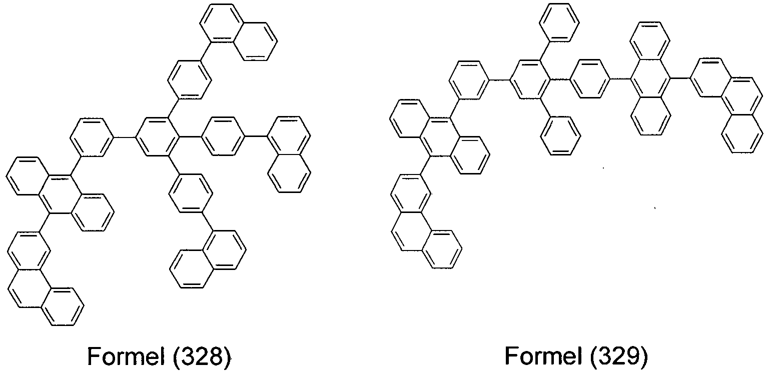

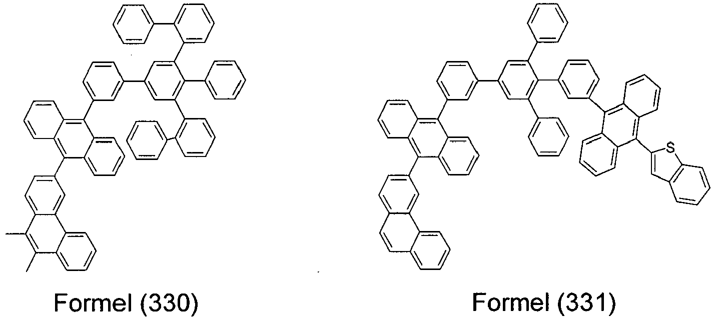

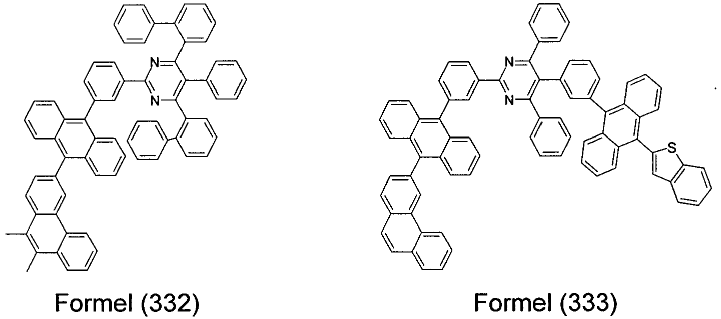

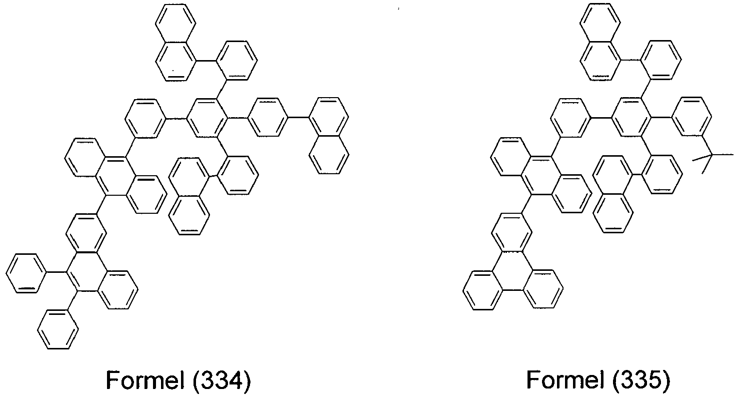

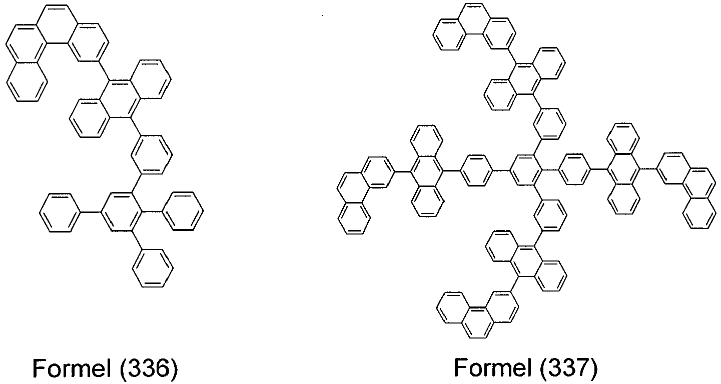

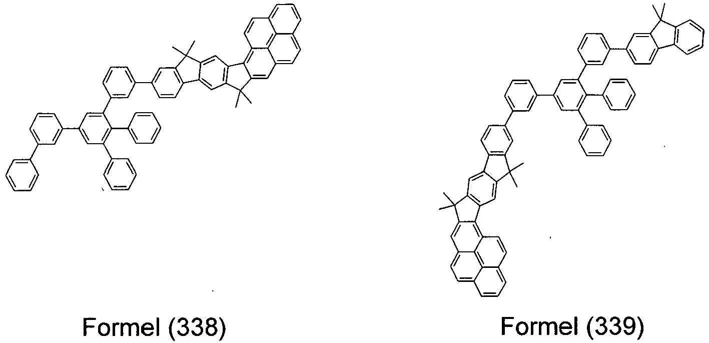

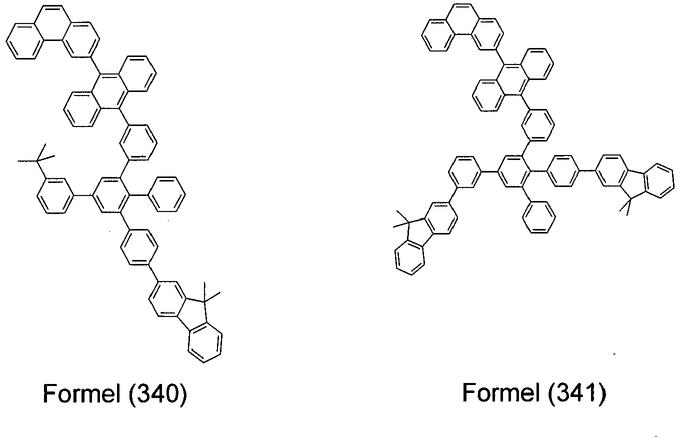

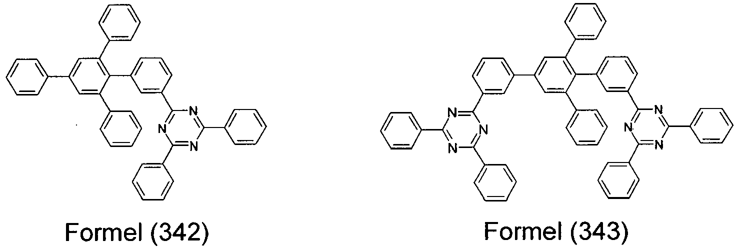

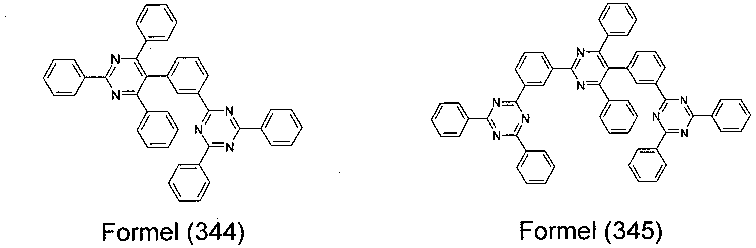

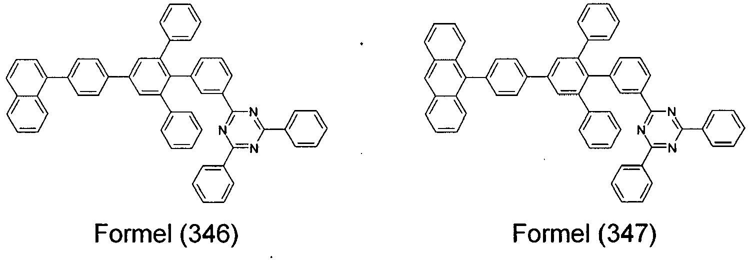

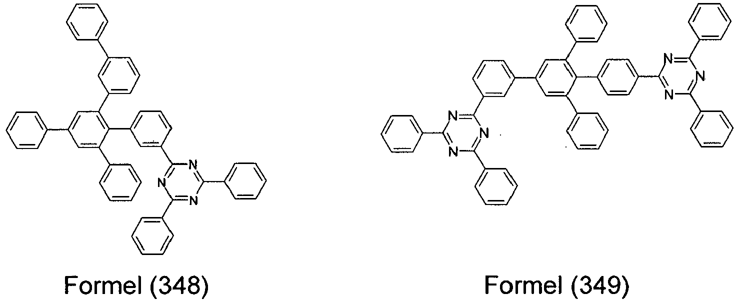

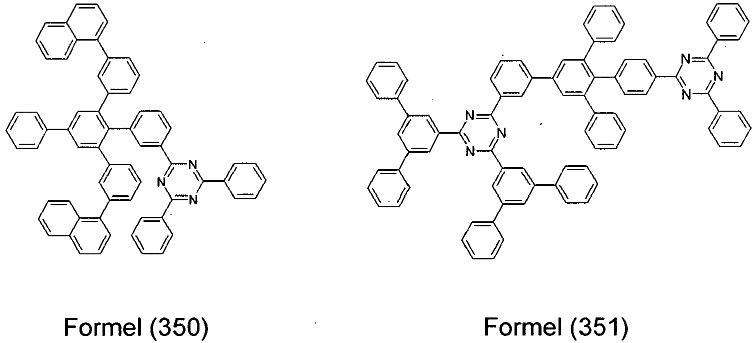

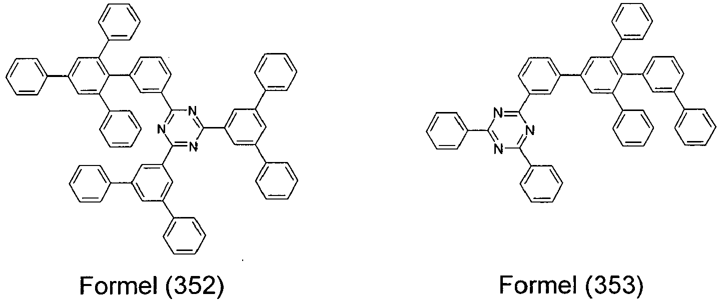

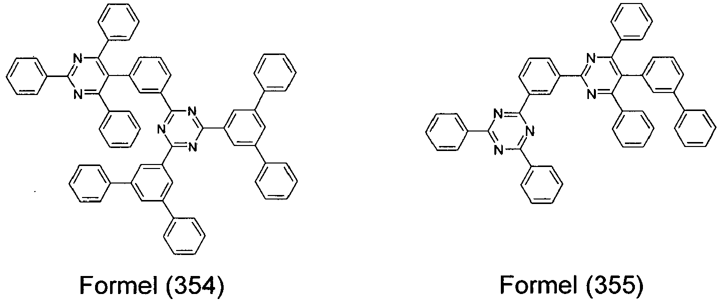

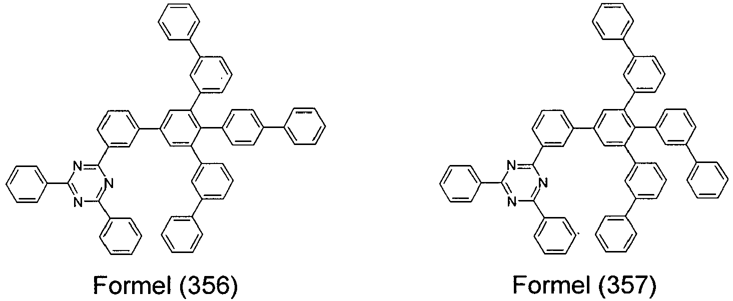

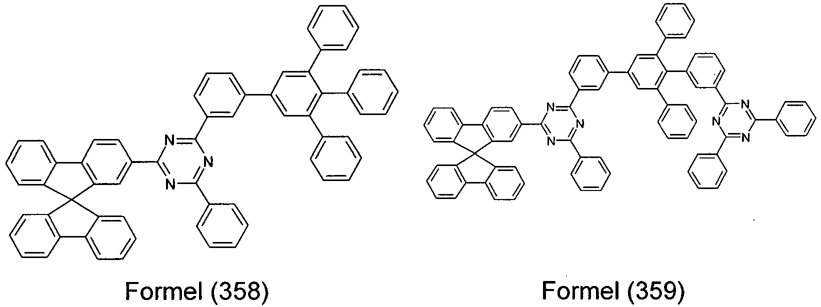

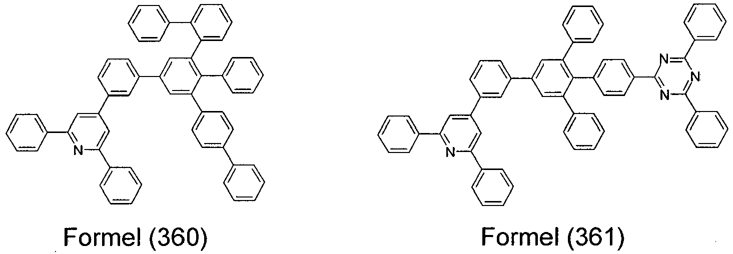

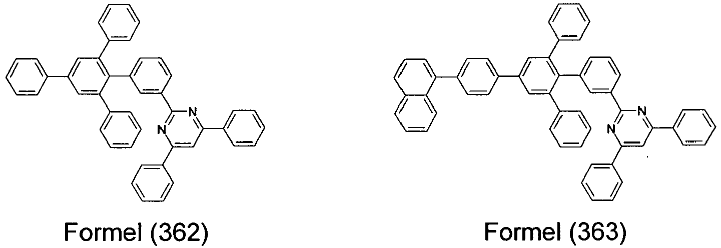

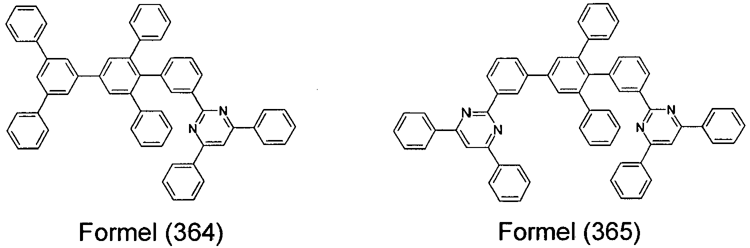

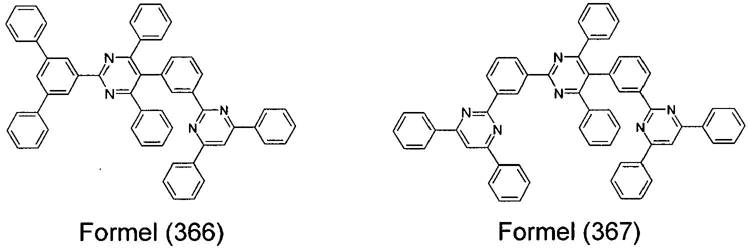

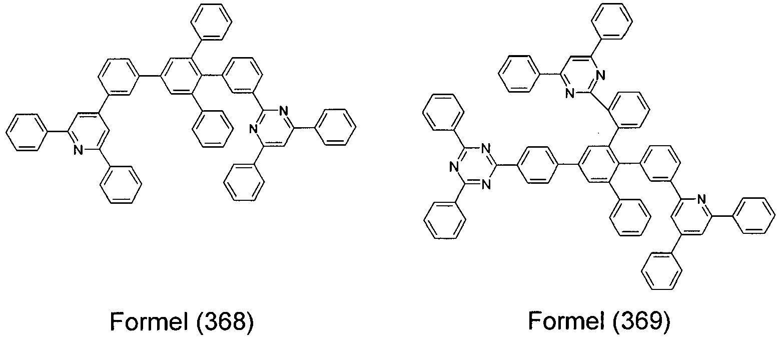

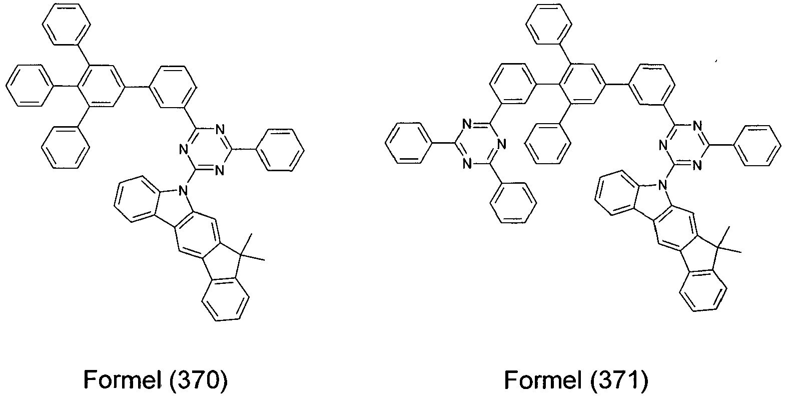

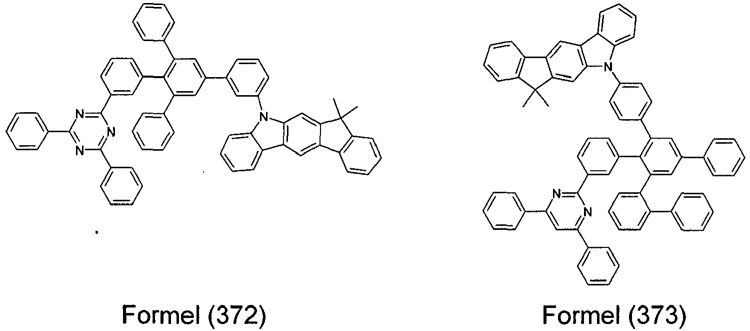

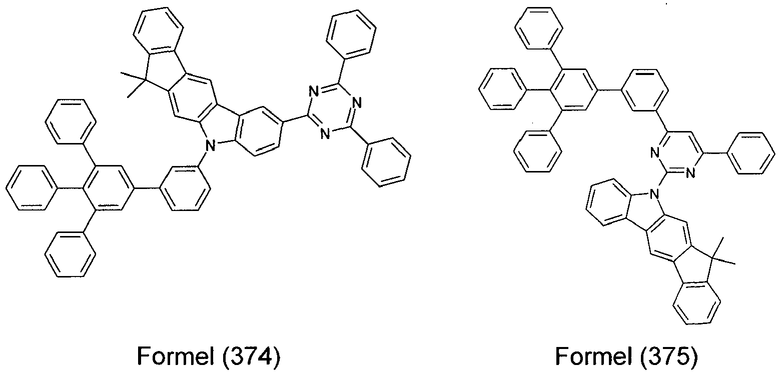

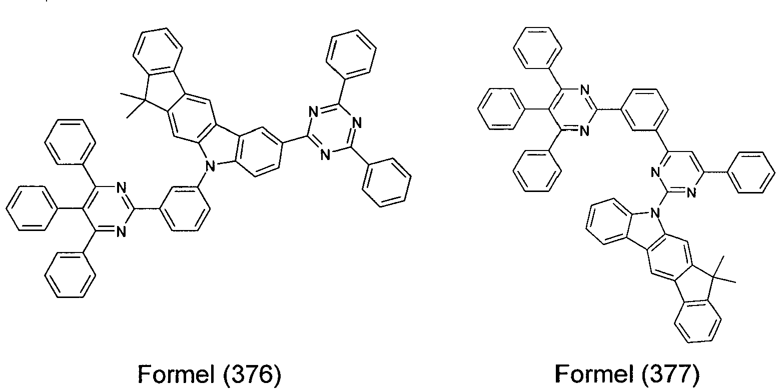

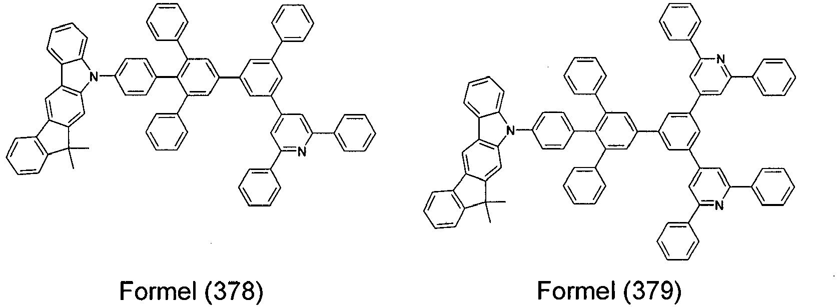

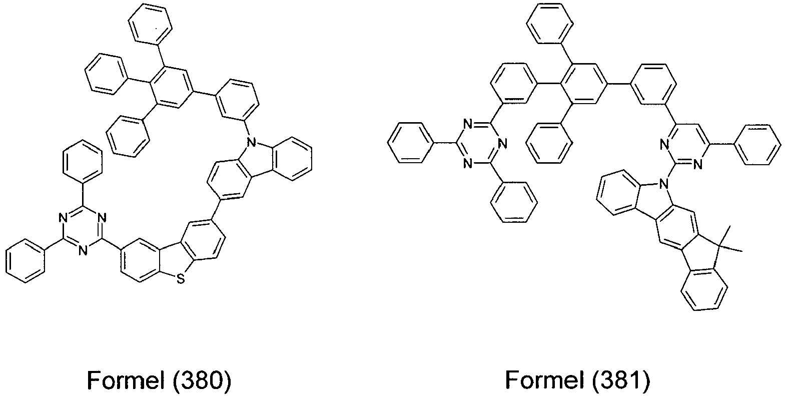

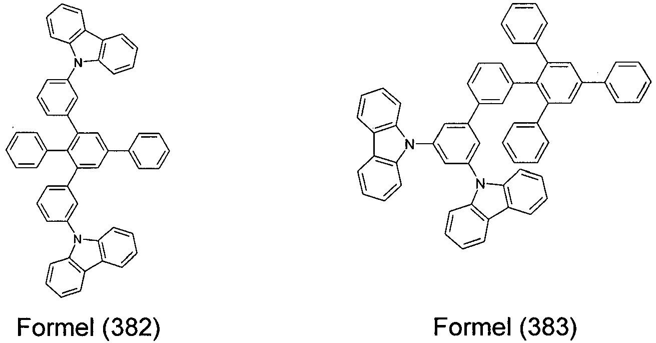

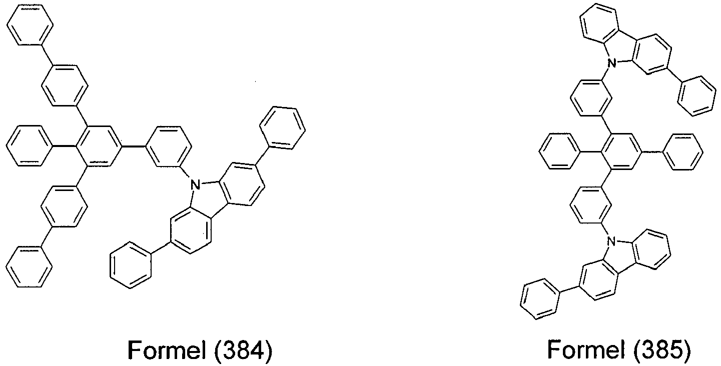

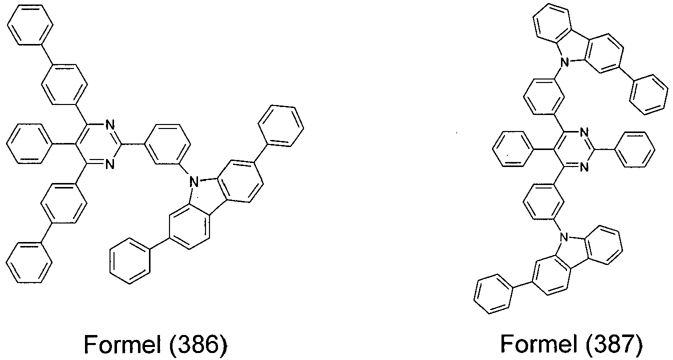

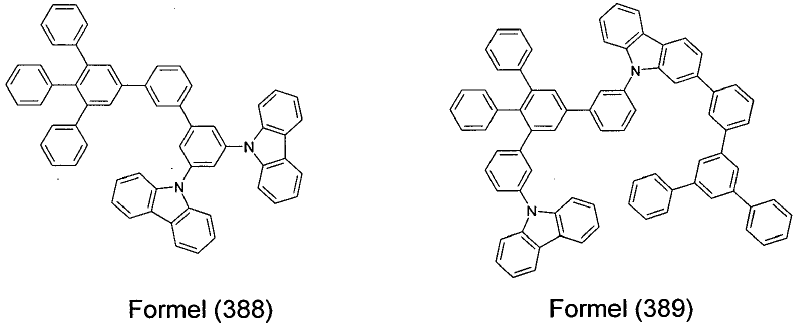

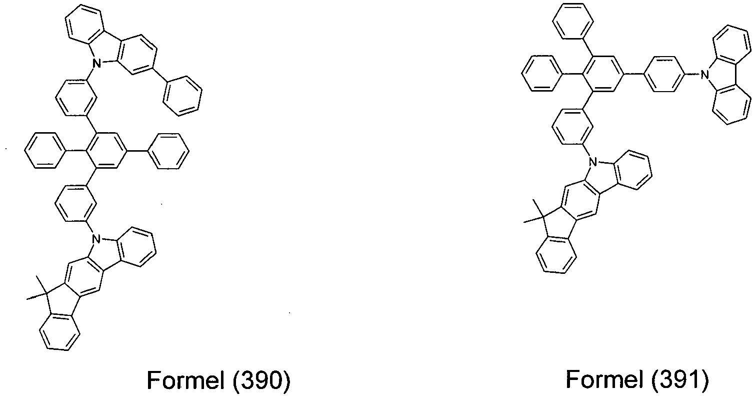

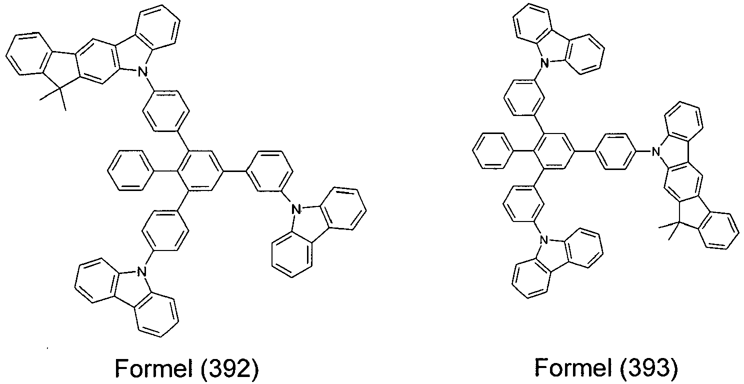

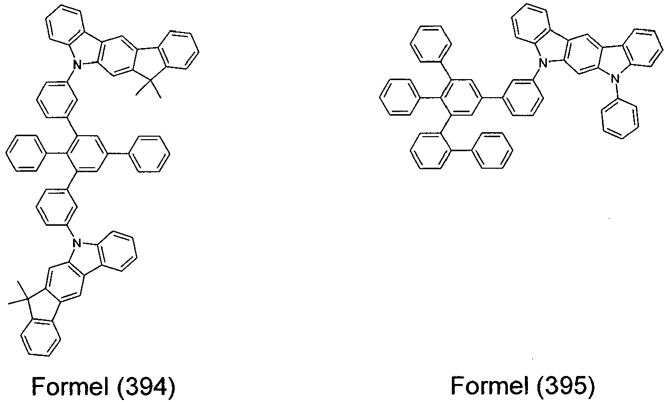

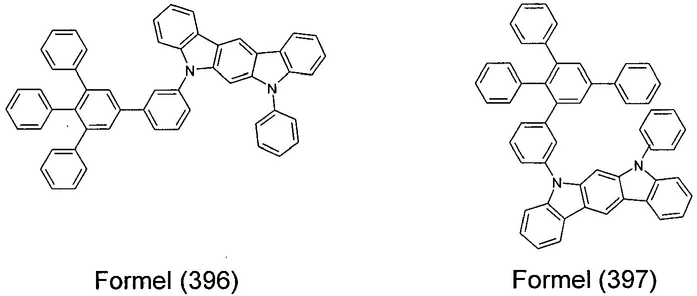

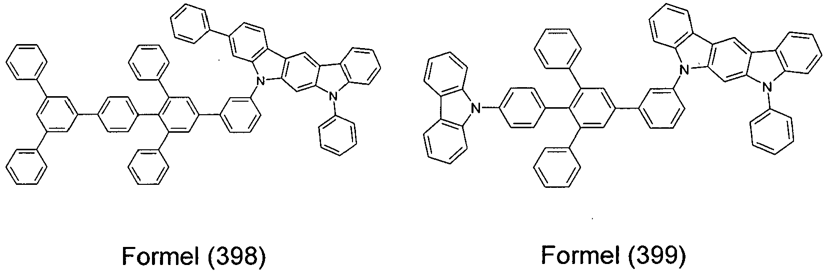

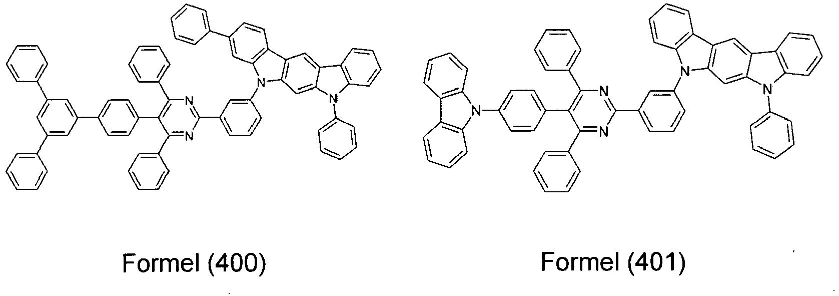

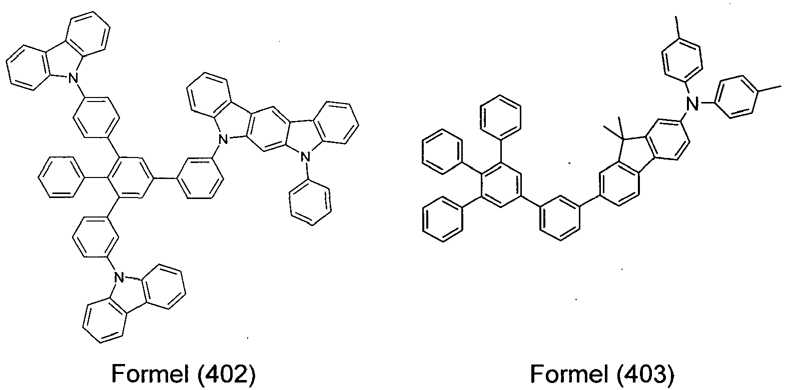

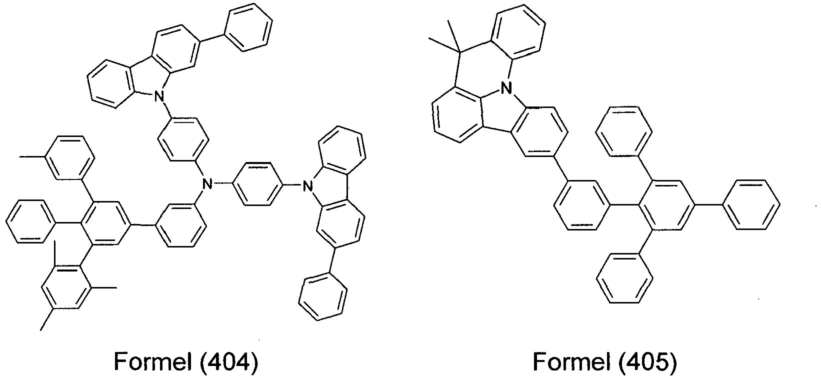

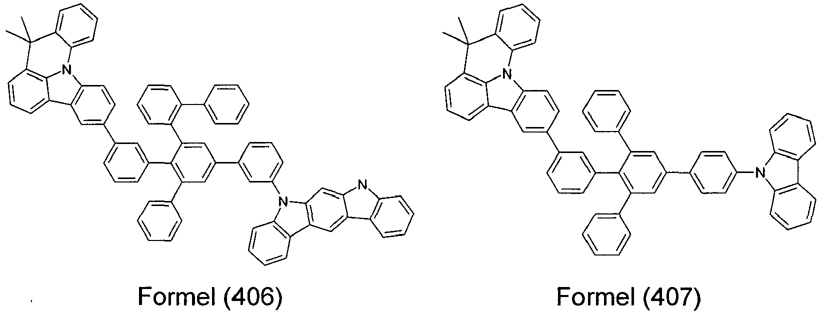

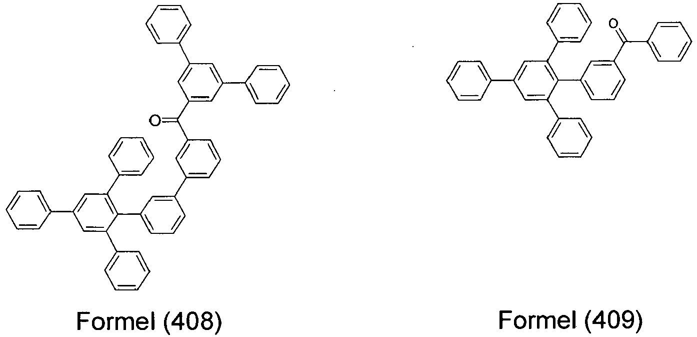

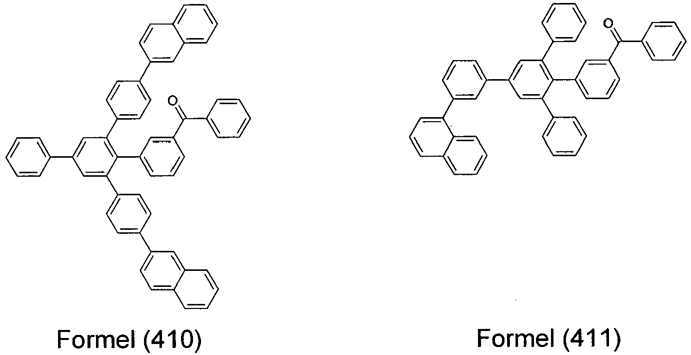

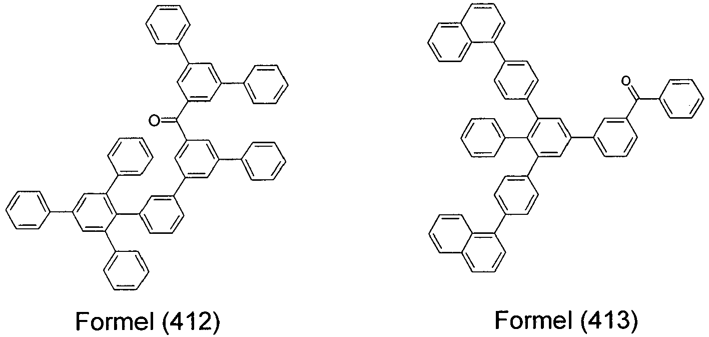

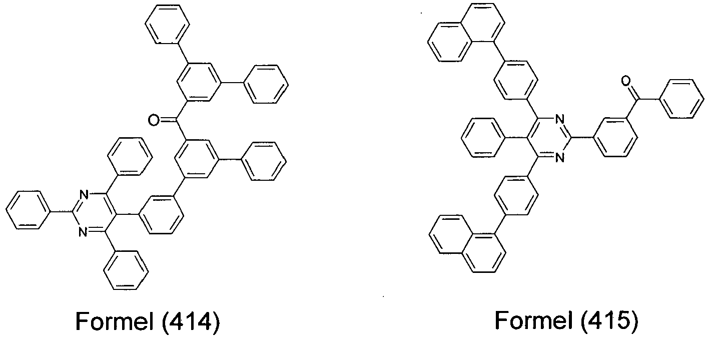

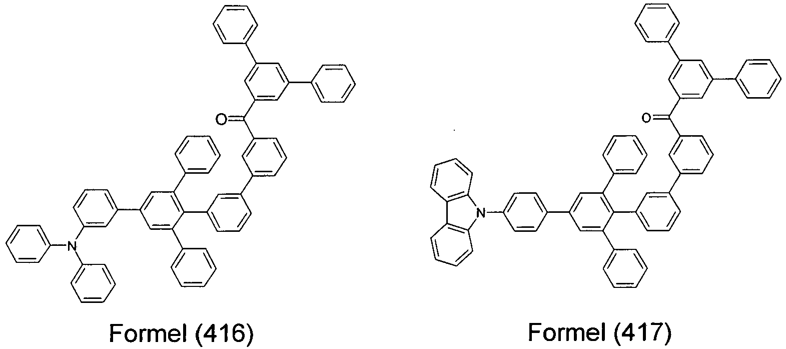

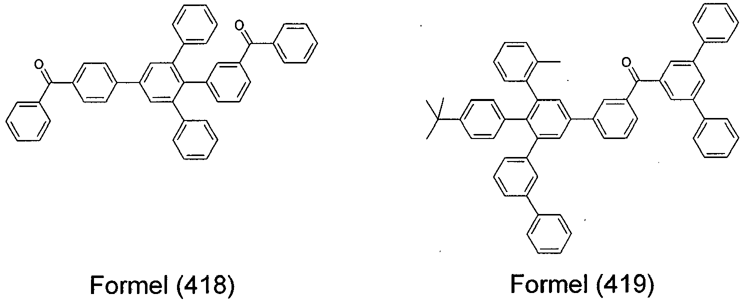

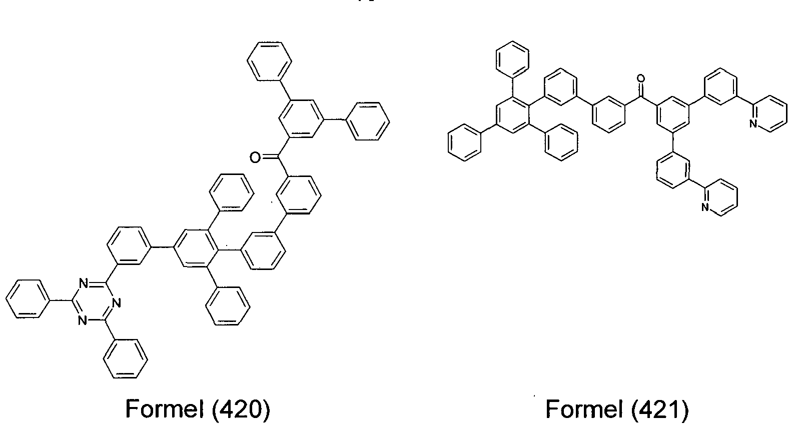

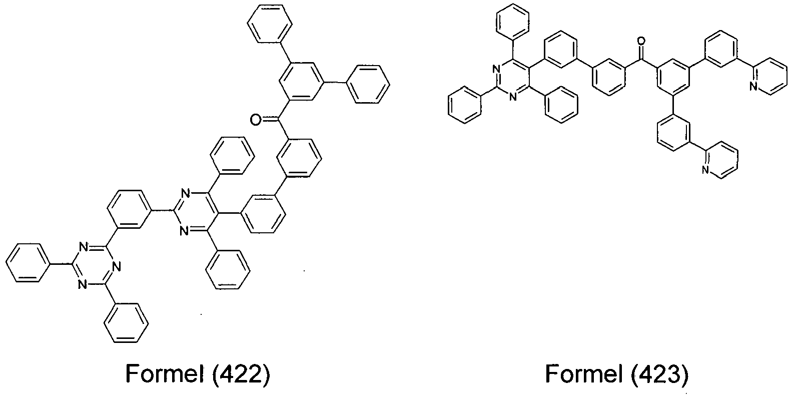

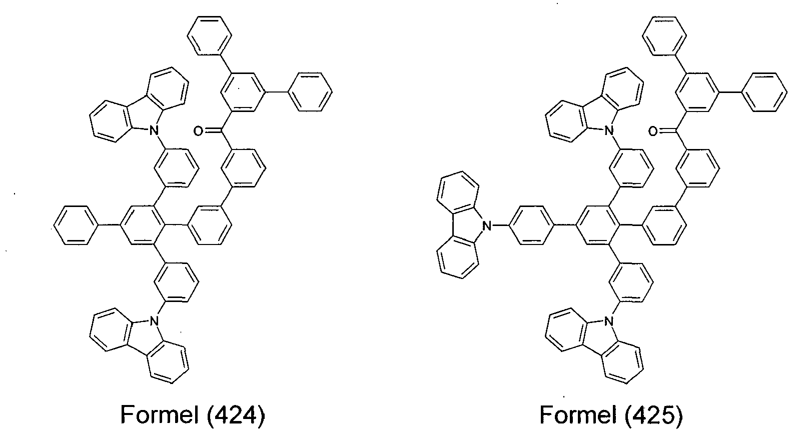

Die Verbindungen gemäß den Formeln (27) oder (28) können in einer elektronischen Vorrichtung verwendet werden. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser), "organic plasmon emitting devices" (D. M. Koller et al., Nature Photonics 2008, 1-4) und Elektrophotographie-Vorrichtungen, bevorzugt organischen Elektrolumineszenzvorrichtungen (OLEDs), besonders bevorzugt phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Ein möglicher Schichtaufbau ist bspw. der folgende: Kathode/ EML/Zwischeschicht/Pufferschicht/Anode, wobei EML die emittierende Schicht repräsentiert. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013). Weiterhin kann auf eine oder beide der Elektroden eine optische Auskopplungsschicht aufgebracht sein.

Die Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine der Verbindungen gemäß Formel (27) oder Formel (28) als Host- oder Matrixmaterial für fluoreszierende oder phosphoreszierende Emitter, insbesondere für phosphoreszierende Emitter, und/oder in einer Lochblockierschicht und/oder in einer Elektronentransportschicht und/oder in einer elektronenblockierenden bzw. exzitonenblockierenden Schicht und/oder in einer Lochtransportschicht und/oder in einer optischen Auskopplungsschicht. Dabei gelten die oben aufgeführten bevorzugten Ausführungsformen auch für die Verwendung der Materialien in organischen elektronischen Vorrichtungen.

In einer bevorzugten Ausführungsform der Erfindung wird die Verbindung gemäß Formel (27) oder Formel (28) als Host- oder Matrixmaterial für eine fluoreszierende oder phosphoreszierende Verbindung in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Host- oder Matrixmaterial enthält.

Wenn die Verbindung gemäß Formel (27) oder Formel (28) als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, ganz bevorzugt aus einem angeregten Triplett- und/oder Quintettzustand und ganz besonders bevorzugt aus einem Triplettzustand. Im Sinne dieser Anmeldung sollen alle lumineszierenden Komplexe mit Metallen der zweiten und dritten Übergangsmetallreihe, insbesondere alle Iridium- und Platinkomplexe, sowie alle lumineszierenden Kupferkomplexe als phosphoreszierende Verbindungen angesehen werden.

Eine weitere bevorzugte Ausführungsform der vorliegenden Erfindung ist der Einsatz der Verbindung gemäß Formel (27) oder Formel (28) als Matrixmaterial für einen phosphoreszierenden Emitter in Kombination mit einem weiteren Matrixmaterial. Besonders geeignete Matrixmaterialien, welche in Kombination mit den erfindungsgemäßen Verbindungen gemäß Formel (27) oder Formel (28) eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß der nicht offen gelegten Anmeldung DE 102008056688.8, Diazaphosphol-Derivate, z. B. gemäß der nicht offen gelegten Anmeldung DE 102009022858.6, oder Indenocarbazolderivate, z. B. gemäß den nicht offen gelegten Anmeldungen DE 102009023155.2 und DE 102009031021.5.

Weiterhin bevorzugt im Sinne der vorliegenden Erfindung sind Mischungen bestehend aus mehr als zwei Matrixmaterialien, wobei wenigstens eines der Matrixmaterialien eine der erfindungsgemäßen Verbindungen ist.

Weitere Matrixmaterialien, die mit den erfindungsgemäßen Verbindungen in Kombination eingesetzt werden können sind grundsätzlich alle Matrixmaterialien, wobei bevorzugte Matrixmaterialien die oben genannten sind.

Schließlich sind Mischungen enthaltend zwei oder mehr der erfindungsgemäßen Verbindungen als Matrixmaterial ganz besonders bevorzugt.

Als phosphoreszierende Verbindungen (Triplettemitter) eignen sich insbesondere Verbindungen, die bei geeigneter Anregung Licht bzw. Strahlung, bspw. im sichtbaren Bereich und/oder ultravioletten Bereich und/oder im infraroten Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Die erfindungsgemäßen Matrixmaterialien bzw. die oben beschriebenen Mischungen enthaltend ein oder mehrere der erfindungsgemäßen Matrixmaterialien können dabei als Matrixmarial für einzelne Emitter oder für Mischungen von Emittern eingesetzt werden.

Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere fluoreszierende Verbindungen verwenden.

Phosphoreszierende Metallkomplexe enthalten bevorzugt Ir, Ru, Pd, Pt, Os oder Re. Bevorzugte Liganden für phosphoreszierende Metallkomplexe sind 2-Phenylpyridin-Derivate, 7,8-Benzochinolin-Derivate, 2-(2-Thienyl)-pyridin-Derivate, 2-(1-Naphthyl)pyridin-Derivate oder 2-Phenylchinolin-Derivate. Alle diese Verbindungen können substituiert sein, z.B. für blau mit Fluor-, Cyano- und/oder Trifluormethylsubstituenten. Auxiliäre Liganden sind bevorzugt Acetylacetonat oder Picolinsäure.

Insbesondere sind Komplexe von Pt oder Pd mit tetradentaten Liganden, (US 2007/0087219), Pt-Porphyrinkomplexe mit vergrößertem Ringsystem (US 2009/0061681 A1) und Ir-Komplexe geeignet, z.B. 2,3,7,8,12,13,17, 18-Octaethyl-21H, 23H-porphyrin-Pt(II), Tetraphenyl-Pt(II)-tetrabenzo-porphyrin (US 2009/0061681), *Cis*-Bis(2-phenylpyridinato-N,C^{2'})Pt(II), *Cis-*Bis(2-(2'-thienyl)pyridinato-N,C^{3'})Pt(II), Cis-Bis-(2-(2'-thienyl)chinolinato-N,C^{5'})Pt(II), (2-(4,6-Difluorophenyl)pyridinato-N,C^{2'})Pt(II)(acetylacetonat), oder Tris(2-phenylpyridinato-N,C^{2'})Ir(III) (= Ir(ppy)₃, grün), Bis(2-phenyl-pyridinato-N,C²)Ir(III)(acetylacetonat) (= Ir(ppy)₂acetylacetonat, grün, US 2001/0053462 A1, Baldo, Thompson et.al. Nature 403, (2000), 750-753), Bis(1-phenylisochinolinato-N,C^{2'})(2-phenylpyridinato-N,C^{2'})-Iridium(III), Bis(2-phenylpyridinato-N,C^{2'})(1-phenylisochinolinato-N,C^{2'})-Iridium(III), Bis(2-(2'-benzothienyl)pyridinato-N,C^{3'})Iridium(III)(acetylacetonat), Bis(2-(4',6'-difluorophenyl)pyridinato-N,C^{2'})Iridium(III)(piccolinat) (Flrpic, blau), Bis(2-(4',6'-difluorophenyl)pyridinato-N,C^{2'})Ir(III)(tetrakis(1-pyrazolyl)borat), Tris(2-(biphenyl-3-yl)-4-tertbutylpyridin)iridium(III), (ppz)₂Ir(5phdpym) (US 2009/0061681 A1), (45ooppz)₂Ir(5phdpym) (US 2009/0061681 A1), Derivate von 2-Phenylpyridin-Ir-Komplexen, wie z.B. PQIr (= Iridium(III)bis(2-phenyl-quinolyl-N,C^{2'})acetylacetonat), Tris(2-phenylisochinolinato-N,C)Ir(III) (rot), Bis(2-(2'-benzo[4,5-a]thienyl)-pyridinato-N,C³)Ir(acetylacetonat) ([Btp₂Ir(acac)], rot, Adachi et al. Appl. Phys. Lett. 78 (2001), 1622-1624).

Ebenfalls geeignet sind Komplexe von trivalenten Lanthaniden wie z.B: Tb³⁺ und Eu³⁺ (J.Kido et al. Appl.Phys.Lett. 65 (1994), 2124, Kido et al. Chem. Lett.657, 1990, US 2007-0252517 A1) oder phosphoreszente Komplexe von Pt(II), Ir(I), Rh(I) mit Maleonitrildithiolat (Johnson et al., JACS 105, 1983, 1795), Re(I)-Tricarbonyl-diimin-Komplexe (Wrighton, JACS 96, 1974, 998 u.a.), Os(II)-Komplexe mit Cyanoliganden und Bipyridyl- oder Phenanthrolin-Liganden (Ma et al., Synth. Metals 94, 1998, 245).

Weitere phosphoreszierende Emitter mit tridentaten Liganden werden beschrieben in US 6824895 und US 10/729238. Rot emittierende phosphoreszente Komplexe findet man in US 6835469 und US 6830828.

Wenn die erfindungsgemäße Verbindung gemäß Formel (27) oder Formel (28) als Hostmaterial für eine emittierende Verbindung in einer emittierenden Schicht eingesetzt wird, wird sie bevorzugt in Kombination mit einem oder mehreren fluoreszierenden Materialien (Singulettemitter) eingesetzt. Unter Fluoreszenz im Sinne dieser Erfindung wird die Lumineszenz aus einem angeregten Zustand mit niedriger Spinmultiplizität verstanden, also aus einem Spinzustand S = 1.

Eine weitere bevorzugte Ausführungsform der vorliegenden Erfindung ist der Einsatz der erfindungsgemäßen Verbindungen gemäß Formel (27) oder Formel (28) als Hostmaterial für einen fluoreszierenden Emitter in Kombination mit einem weiteren Hostmaterial. Besonders geeignete Hostmaterialien, welche in Kombination mit den Verbindungen gemäß Formel (27) oder Formel (28) eingesetzt werden können, sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen wie z.B. Anthracen, Benzanthracen, Benzphenanthren (DE 102009005746.3, WO 2009/069566), Phenanthren, Tetracen, Coronen, Chrysen, Fluoren, Spirofluoren, Perylen, Phthaloperylen, Naphthaloperylen, Decacyclen, Rubren, der Oligoarylenvinylene (z. B. DPVBi = 4,4'-Bis(2,2-diphenylethenyl)-1,1'-biphenyl) oder Spiro-DPVBi gemäß EP 676461), der polypodalen Metall¬¬komplexe (z. B. gemäß WO 2004/081017), insbesondere Metallkomplexe von 8-Hydroxychinolin, z.B. Alq₃ (= Aluminium(III)tris(8-hydroxychinolin)) oder Bis(2-methyl-8-chinolinolato)-4-(phenylphenolinolato)aluminium, auch mit Imidazol-Chelat (US 2007/0092753 A1) sowie der Chinolin-Metallkomplexe, Aminochinolin-Metallkomplexe, Benzochinolin-Metallkomplexe, der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäure¬derivate (z. B. gemäß WO 2006/117052) oder der Benzanthracene (z. B. gemäß DE 102007024850, WO 2008/145239).

Besonders bevorzugte Host¬materialien sind ausgewählt aus den Klassen der Oligoarylene, enthaltend Naphthalin, Anthracen, Benzanthracen und/oder Pyren oder Atropisomere dieser Verbindungen, der Ketone, der Phosphinoxide und der Sulfoxide. Ganz Bbesonders bevorzugte Host-materialien sind ausgewählt aus den Klassen der Oligoarylene, enthaltend Anthracen, Benzanthracen und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Weiterhin bevorzugt im Sinne der vorliegenden Erfindung sind Mischungen bestehend aus mehr als zwei Hostmaterialien, wobei wenigstens eines der Hostmaterialien eine der erfindungsgemäßen Verbindungen ist. Weitere Hostmaterialien, die mit den erfindungsgemäßen Verbindungen in Kombination eingesetzt werden können sind grundsätzlich alle Hostmaterialien, wobei bevorzugte Hostmaterialien die oben genannten sind.

Schließlich sind Mischungen enthaltend zwei oder mehr der erfindungsgemäßen Verbindungen als Hostmaterial ganz besonders bevorzugt.

Als fluoreszierende Verbindungen (Singulettemitter) eignen sich insbesondere Verbindungen, die bei geeigneter Anregung Licht bzw. Strahlung, bspw. im sichtbaren Bereich und/oder ultravioletten Bereich und/oder im infraroten Bereich emittieren.

Bevorzugte Dotanden (Emitter) sind ausgewählt aus der Klasse der Monostyrylamine, der Distyrylamine, der Tristyrylamine, der Tetrastyrylamine, der Styrylphosphine, der Styrylether und der Arylamine.

Unter einem Monostyrylamin wird eine Verbindung verstanden, die eine substituierte oder unsubstituierte Styrylgruppe und mindestens ein, bevorzugt aromatisches, Amin enthält. Unter einem Distyrylamin wird eine Verbindung verstanden, die zwei substituierte oder unsubstituierte Styrylgruppen und mindestens ein, bevorzugt aromatisches, Amin enthält. Unter einem Tristyrylamin wird eine Verbindung verstanden, die drei substituierte oder unsubstituierte Styrylgruppen und mindestens ein, bevorzugt aromatisches, Amin enthält. Unter einem Tetrastyrylamin wird eine Verbindung verstanden, die vier substituierte oder unsubstituierte Styrylgruppen und mindestens ein, bevorzugt aromatisches, Amin enthält. Die Styrylgruppen sind besonders bevorzugt Stilbene, die auch noch weiter substituiert sein können. Entsprechende Phosphine und Ether sind in Analogie zu den Aminen definiert. Unter einem Arylamin bzw. einem aromatischen Amin im Sinne dieser Erfindung wird eine Verbindung verstanden, die drei substituierte oder unsubstituierte aromatische oder hetero¬aromatische Ringsysteme direkt an den Stickstoff gebunden enthält. Bevorzugt ist mindestens eines dieser aromatischen oder heteroaromatischen Ringsysteme ein kondensiertes Ringsystem, bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9 Position. Unter einem aromatischen Anthracendiamin wird eine Verbindung verstanden, in der zwei Diarylaminogruppen direkt an eine Anthracengruppe gebunden sind, vorzugsweise in 2,6- oder 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind.

Weitere bevorzugte Dotanden sind gewählt aus Indenofluorenaminen bzw. -diaminen, beispielsweise gemäß WO 2006/122630, Benzoindenofluorenaminen bzw. -diaminen, beispiels¬weise gemäß WO 08/006449, und Dibenzoindenofluorenaminen bzw. -diaminen, beispielsweise gemäß WO 07/140847.

Beispiele für Dotanden aus der Klasse der Styrylamine sind substituierte oder unsubstituierte Tristilbenamine oder die Dotanden, die in WO 2006/000388, WO 2006/058737, WO 2006/000389, WO 2007/065549 und WO 2007/115610 beschrieben sind. Distyrylbenzol-und Distyrylbiphenyl-Derivate sind beschrieben in US 5121029. Weitere Styrylamine sind in US 2007/0122656 zu finden.

Weitere bevorzugte Dotanden sind gewählt aus Derivaten von Naphthalin, Anthracen, Tetracen, Benzanthracen, Benzphenanthren (DE 102009 005746.3), Fluoren, Fluoranthen, Periflanthen, Indenoperylen, Phenanthren, Perylen (US 2007/0252517), Pyren, Chrysen, Decacyclen, Coronen, Tetraphenylcyclopentadien, Pentaphenylcyclopentadien, Fluoren, Spirofluoren, Rubren, Cumarin (US 4769292, US 6020078, US 2007/0252517), Pyran, Oxazoln, Benzoxazol, Benzothiazol, Benzimidazol, Pyrazin, Zimtsäureestern, Diketopyrrolopyrrol, Acridon und Chinacridon (US 2007/0252517).

Von den Anthracenverbindungen sind besonders bevorzugt in 9,10-Position substituierte Anthracene wie z. B. 9,10-Diphenylanthracen und 9,10-Bis(phenylethynyl)anthracen. Auch 1,4-Bis(9'-ethynylanthracenyl)-benzol ist ein bevorzugter Dotand.

Ebenfalls bevorzugt sind Derivate von Rubren, Cumarin, Rhodamin, Chinacridon wie z.B. DMQA (= N,N'-dimethylchinacridon), Dicyanomethylenpyran wie z.B. DCM (= 4-(dicyanoethylen)-6-(4-dimethylaminostyryl-2-methyl)-4H-pyran), Thiopyran, Polymethin, Pyrylium- und Thiapyryliumsalzen, Periflanthen und Indenoperylen.

Blaue Fluoreszenzemitter sind bevorzugt Polyaromaten wie z.B. 9,10-Di(2-naphthylanthracen) und andere Anthracen-Derivate, Derivate von Tetracen, Xanthen, Perylen wie z.B. 2,5,8,11-Tetra-t-butyl-perylen, Phenylen, z.B. 4,4'-(Bis(9-ethyl-3-carbazovinylen)-1,1'-biphenyl, Fluoren, Fluoranthen, Arylpyrene (US 11/097352), Arylenvinylene (US 5121029, US 5130603), Derivate von Rubren, Cumarin, Rhodamin, Chinacridon wie z.B. DMQA, Dicyanomethylenpyran wie z.B. DCM, Thiopyrane, Polymethin, Pyrylium- und Thiapyryliumsalzen, Periflanthen, Indenoperylen, Bis(azinyl)imin-Bor-Verbindungen (US 2007/0092753 A1), Bis(azinyl)-methenverbindungen und Carbostyryl-Verbindungen.

Weitere bevorzugte blaue Fluoreszenzemitter sind in C.H.Chen et al.: "Recent developments in organic electroluminescent materials" Macromol. Symp. 125, (1997) 1-48 und "Recent progress of molecular organic electroluminescent materials and devices" Mat. Sci. and Eng. R, 39 (2002), 143-222 beschrieben.

Weitere bevorzugte blau fluoreszierende Emitter sind die in der Anmeldung DE 102008035413 offenbarten Kohlenwasserstoffe.

Die erfindungsgemäßen Hostmaterialien bzw. die oben beschriebenen Mischungen enthaltend ein oder mehrere der erfindungsgemäßen Hostmaterialien können dabei als Hostmaterial für einzelne Emitter oder für Mischungen von Emittern eingesetzt werden.

Die Mischung aus der erfindungsgemäßen Verbindung(en) und der emittierenden (fluoreszierende und/oder phosphoreszierende) Verbindung enthält zwischen 99 und 1 Gew.-% , vorzugsweise zwischen 98 und 10 Gew.-%, besonders bevorzugt zwischen 97 und 60 Gew.-%, insbesondere zwischen 95 und 80 Gew.-% der Verbindung gemäß Formel (27) oder Formel (28) bezogen auf die Gesamtmischung aus Emitter und Matrix- bzw. Hostmaterial. Entsprechend enthält die Mischung zwischen 1 und 99 Gew.-%, vorzugsweise zwischen 2 und 90 Gew.-%, besonders bevorzugt zwischen 3 und 40 Gew.-%, insbesondere zwischen 5 und 20 Gew.-% des Emitters bezogen auf die Gesamtmischung aus Emitter und Matrix- bzw. Hostmaterial.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In einer weiteren bevorzugten Ausführungsform der Erfindung wird die Verbindung gemäß Formel (27) oder Formel (28) als Elektronentransportmaterial in einer Elektronentransport- oder Elektroneninjektionsschicht eingesetzt. Dabei kann die emittierende Schicht fluoreszierend oder phosphoreszierend sein. Wenn die Verbindung als Elektronentransportmaterial eingesetzt wird, kann es bevorzugt sein, wenn sie dotiert ist, beispielsweise mit Alkalimetallkomplexen, wie z. B. Liq (Lithiumhydroxychinolinat), oder mit Alkalimetallsalzen, wie z. B. LiF.

In nochmals einer weiteren bevorzugten Ausführungsform der Erfindung werden die erfindungsgemäßen Verbindungen der Formel (27) oder Formel (28) in einer Lochblockierschicht eingesetzt. Unter einer Lochblockierschicht wird eine Schicht verstanden, die auf Kathodenseite direkt an eine emittierende Schicht angrenzt.

In nochmals einer weiteren Ausführungsform der Erfindung werden die Verbindungen der Formel (27) oder Formel (28) in einer Lochtransportschicht bzw. in einer Elektronenblockierschicht bzw. Exzitonenblockierschicht eingesetzt.

Es ist weiterhin möglich, die Verbindungen gemäß Formel (27) oder Formel (28) sowohl in einer Lochblockierschicht bzw. Elektronentransportschicht als auch als Matrix in einer emittierenden Schicht oder sowohl in einer Lochtransportschicht bzw. Exzitonenblockierschicht als auch als Matrix in einer emittierenden Schicht zu verwenden.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (27) oder Formel (28) einsetzen.

Die vorliegende Erfindung bezieht sich daher auch auf Zusammensetzungen enthaltend wenigstens eine der erfindungsgemäßen Verbindungen sowie ein weiteres organisch funktionelles Material ausgewählt aus der Gruppe der Emitter, Host Materialien, Matrix Materialien, Elektronentransportmaterialien (ETM), Elektroneninjektionsmaterialien (EIM), Lochtransportmaterialien (HTM), Lochinjektionsmaterialien (HIM), Elektronenblockiermaterialien (EBM), Lochblockiermaterialien (HBM), Exzitonenblockiermaterialien (ExBM), besonders bevorzugt Emitter und ganz besonders bevorzugt fluoreszierende und/oder phosphoreszierende Emitter.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden. Diese Verfahren eignen sich insbesondere auch für Oligomere, Dendrimere und Polymere.

Eine weitere Ausführungsform der vorliegenden Erfindung bezieht sich auf Formulierungen enthaltend eine oder mehrere der erfindungsgemäßen Verbindungen sowie ein oder mehrere Lösungsmittel. Die Formulierung eignet sich hervorragend zum Erzeugen von Schichten aus Lösung.

Geeignete und bevorzugte Lösungsmittel sind beispielsweise Toluol, Anisol, Xylole, Methylbenzoat, Dimethylanisole, Trimethylbenzole, Tetralin, Veratrole, Tetrahydrofuran, Chlorbenzol oder Dichlorbenzole sowie Gemische derselben.

Ebenso sind Hybridverfahren möglich, bei denen beispielsweise eine oder mehrere Schichten aus Lösung aufgebracht werden und eine oder mehrere andere Schichten im Vakuum aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäße organische Elektrolumineszenzvorrichtung kann beispielsweise in Displays oder für Beleuchtungszwecke verwendet werden, aber auch für medizinische oder kosmetische Anwendungen.

Ein weiterer Gegenstand der Erfindung ist daher die Verwendung der erfindungsgemäßen Verbindungen in einer elektronischen Vorrichtung.

Die erfindungsgemäßen Verbindungen eignen sich zum Einsatz in lichtemittierenden Vorrichtungen. Somit sind diese Verbindungen sehr vielseitig einsetzbar. Einige der Hauptanwendungsgebiete sind dabei Display- oder Beleuchtungs-Technologien. Weiterhin ist es besonders vorteilhaft, die Verbindungen sowie Vorrichtungen enthaltend diese Verbindungen im Bereich der Phototherapie einzusetzen.

Ein weiterer Gegenstand der vorliegenden Erfindung bezieht sich daher auf die Verwendung der erfindungsgemäßen Verbindungen und Vorrichtungen enthaltend die Verbindungen zur Behandlung, Prophylaxe und Diagnose von Erkrankungen. Noch ein weiterer Gegenstand der vorliegenden Erfindung bezieht sich auf die Verwendung, der erfindungsgemäßen Verbindungen und Vorrichtungen enthaltend die Verbindungen zur Behandlung und Prophylaxe kosmetischen Umstände.

Ein weiterer Gegenstand der vorliegenden Erfindung bezieht sich auf die erfindungsgemäßen Verbindungen zu Herstellung von Vorrichtungen zur Therapie, Prophylaxe und/oder Diagnose therapeutischer Erkrankungen.

Dabei sind viele Erkrankungen mit kosmetischen Aspekten assoziiert. So leidet ein Patient mit schwerer Akne in der Gesichtspartie nicht nur an den medizinischen Ursachen und Folgen der Erkrankung, sondern auch an den kosmetischen Begleitumständen.

Phototherapie oder Lichttherapie findet in vielen medizinischen und/oder kosmetischen Bereichen Anwendung. Die erfindungsgemäßen Verbindungen sowie die Vorrichtungen enthaltend diese Verbindungen können daher zur Therapie und/oder Prophylaxe und/oder Diagnose von allen Erkrankungen und/oder in kosmetischen Anwendungen eingesetzt werden, für die der Fachmann die Anwendung von Phototherapie in Betracht zieht. Der Begriff Phototherapie beinhaltet dabei neben der Bestrahlung auch die photodynamischen Therapie (PDT) sowie das Desinfizieren und Sterilisieren im Allgemeinen. Behandelt werden können mittels Phototherapie oder Lichttherapie nicht nur Menschen oder Tiere, sondern auch jegliche andere Art lebender oder unbelebter Materie. Hierzu gehören, bspw., Pilze, Bakterien, Mikroben, Viren, Eukaryonten, Prokaryonten, Nahrungsmittel, Getränke, Wasser und Trinkwasser.

Der Begriff Phototherapie beinhaltet auch jede Art der Kombination von Lichttherapie und anderen Therapiearten, wie bspw. die Behandlung mit Wirkstoffen. Viele Lichttherapien haben zum Ziel, äußere Partien eines Objektes zu bestrahlen oder zu behandeln, so wie die Haut von Menschen und Tieren, Wunden, Schleimhäute, Auge, Haare, Nägel, das Nagelbett, Zahnfleisch und die Zunge. Die erfindungsgemäße Behandlung oder Bestrahlung kann daneben auch innerhalb eines Objektes durchgeführt werden, um bspw. innere Organe (Herz, Lunge etc.) oder Blutgefäße oder die Brust zu behandeln.

Die erfindungsgemäßen therapeutischen und/oder kosmetischen Anwendungsgebiete sind bevorzugt ausgewählt aus der Gruppe der Hauterkrankungen und Haut-assoziierten Erkrankungen oder Veränderungen bzw. Umstände wie bspw. Psoriasis, Hautalterung, Hautfaltenbildung, Hautverjüngung, vergrößerte Hautporen, Cellulite, ölige/fettige Haut, Follikulitis, aktinische Keratose, precancerose aktinische Keratose, Haut Läsionen, sonnengeschädigte und sonnengestresste Haut, Krähenfüße, Haut Ulkus, Akne, Akne rosacea, Narben durch Akne, Akne Bakterien, Photomodulierung fettiger/öliger Talgdrüsen sowie deren umgebende Gewebe, Ikterus, Neugeborenenikterus, Vitiligo, Hautkrebs, Hauttumore, Crigler Naijar, Dermatitis, atopische Dermatitis, diabetische Hautgeschwüre sowie Desensibilisierung der Haut.

Besonders bevorzugt im Sinne der Erfindung sind die Behandlung und/oder Prophylaxe von Psoriasis, Akne, Cellulite, Hautfaltenbildung, Hautalterung, Ikterus und Vitiligo.

Weitere erfindungsgemäße Anwendungsgebiete für die Zusammensetzungen und/oder Vorrichtungen enthaltend die erfindungsgemäßen Zusammensetzungen sind ausgewählt aus der Gruppe der Entzündungserkrankungen, rheumatoide Arthritis, Schmerztherapie, Behandlung von Wunden, neurologische Erkrankungen und Umstände, Ödeme, Paget's Erkrankung, primäre und metastasierende Tumoren, Bindegewebserkrankungen bzw. -veränderungen, Veränderungen des Kollagens, Fibroblasten und von Fibroblasten stammende Zellspiegel in Geweben von Säugetieren, Bestrahlung der Retina, neovasculare und hypertrophe Erkrankungen, allergische Reaktionen, Bestrahlung der Atemwege, Schwitzen, okulare neovaskulare Erkrankungen, virale Infektionen besonders Infektionen durch Herpes Simplex oder HPV (Humane Papillomviren) zur Behandlung von Warzen und Genitalwarzen.

Besonders bevorzugt im Sinne der Erfindung sind die Behandlung und/oder Prophylaxe von rheumatoider Arthritis, viraler Infektionen, und Schmerzen.

Weitere erfindungsgemäße Anwendungsgebiete für die Verbindungen und/oder Vorrichtungen enthaltend die erfindungsgemäßen Verbindungen sind ausgewählt aus der Winterdepression, Schlafkrankheit, Bestrahlung zur Verbesserung der Stimmung, Linderung von Schmerzen besonders Muskelschmerzen durch bspw. Verspannungen oder Gelenkschmerzen, Beseitigung der Steifheit von Gelenken und das Aufhellen der Zähne (Bleaching).

Weitere erfindungsgemäße Anwendungsgebiete für die Verbindungen und/oder Vorrichtungen enthaltend die erfindungsgemäßen Verbindungen sind ausgewählt aus der Gruppe der Desinfektionen. Mit den erfindungsgemäßen Verbindungen und/oder mit den erfindungsgemäßen Vorrichtungen können jegliche Art von Objekten (unbelebte Materie) oder Subjekten (lebende Materie wie bspw. Mensch und Tier) zum Zweck der Desinfektion, Sterilisation oder Konservierung behandelt werden. Hierzu zählt, zum Beispiel, die Desinfektion von Wunden, die Reduktion von Bakterien, das Desinfizieren chirurgischer Instrumente oder anderer Gegenstände, das Desinfizieren oder Konservieren von Nahrungs- und Lebensmitteln, von Flüssigkeiten, insbesondere Wasser, Trinkwasser und andere Getränke, das Desinfizieren von Schleimhäuten und Zahnfleisch und Zähnen. Unter Desinfektion wird hierbei die Reduktion lebender mikrobiologischer Verursacher unerwünschter Effekte, wie Bakterien und Keime, verstanden.

Zu dem Zweck der oben genannten Phototherapie emittieren Vorrichtungen enthaltend die erfindungsgemäßen Verbindungen bevorzugt Licht der Wellenlänge zwischen 250 and 1250 nm, besonders bevorzugt zwischen 300 and 1000 nm und insbesondere bevorzugt zwischen 400 and 850 nm.

In einer besonders bevorzugten Ausführungsform der vorliegenden Erfindung werden die erfindungsgemäßen Verbindungen in einer organischen lichtemittierenden Diode (OLED) oder einer organischen lichtemittierenden elektrochemischen Zelle (OLEC) zum Zwecke der Phototherapie eingesetzt. Sowohl die OLED als auch die OLEC können dabei einen planaren oder Fiber- bzw. Faser-artigen Aufbau mit beliebigem Querschnitt (z.B. rund, oval, polygonal, quadratisch) mit einem ein-oder mehrschichtigen Aufbau aufweisen. Diese OLECs und/oder OLEDs können in andere Vorrichtungen eingebaut werden, die weitere mechanische, adhäsive und/oder elektronische Bausteine (z.B. Batterie und/oder Steuerungseinheit zur Einstellung der Bestrahlungszeiten, -intensitäten und -wellenlängen) enthalten. Diese Vorrichtungen enthaltend die erfindungsgemäßen OLECs und/order OLEDs sind vorzugsweise ausgewählt aus der Gruppe enthaltend Pflaster, Pads, Tapes, Bandagen, Manschetten, Decken, Hauben, Schlafsäcken,Textilien und Stents.

Die Verwendung von den genannten Vorrichtungen zu dem genannten therapeutischen und/oder kosmetischen Zweck ist besonders vorteilhaft gegenüber dem Stand der Technik, da mit Hilfe der erfindungsgemäßen Vorrichtungen unter Verwendung der OLEDs und/oder OLECs homogene Bestrahlungen geringerer Bestrahlungsintensitäten an nahezu jedem Ort und zu jeder Tageszeit möglich sind. Die Bestrahlungen können stationär, ambulant und/oder selbst, d.h., ohne Einleitung durch medizinisches oder kosmetisches Fachpersonal durchgeführt werden. So können, bspw., Pflaster unter der Kleidung getragen werden, so dass eine Bestrahlung auch während der Arbeitszeit, in der Freizeit oder während des Schlafes möglich ist. Auf aufwendige stationäre/ambulante Behandlungen mit kann in vielen Fällen verzichtet bzw. deren Häufigkeit reduziert werden. Die erfindungsgemäßen Vorrichtungen können zum Widergebrauch gedacht sein oder Wegwerfartikel darstellen, die nach ein-, zwei oder dreimaligem Gebrauch entsorgt werden können.

Weitere Vorteile gegenüber dem Stand der Technik sind bspw. eine geringere Wärmeentwicklung und emotionale Aspekte. So werden Neugeborene, die aufgrund einer Gelbsucht (Ikterus) therapiert werden müssen typischerweise mit verbundenen Augen in einem Brutkasten, ohne körperlichen Kontakt zur den Eltern bestrahlt, was eine emotionale Stresssituation für Eltern und Neugeborene darstellt. Mit Hilfe einer erfindungsgemäßen Decke enthaltend die erfindungsgemäßen OLEDs und/oder OLECs kann der emotionale Stress signifikant vermindert werden. Zudem ist eine bessere Temperierung des Kindes durch eine verringerte Wärmeproduktion der erfindungsgemäßen Vorrichtungen gegenüber herkömmlicher Bestrahlungsgeräte möglich.

Die erfindungsgemäßen Verbindungen und die erfindungsgemäßen elektronischen Vorrichtungen, insbesondere organischen Elektrolumineszenzvorrichtungen zeichnen sich durch folgende überraschende Vorteile gegenüber dem Stand der Technik aus:
1. Die erfindungsgemäßen Verbindungen bzw. Verbindungen gemäß Formel (27) oder Formel (28), eingesetzt als Host- bzw. Matrixmaterial für fluoreszierende oder phosphoreszierende Emitter führen zu sehr hohen Effizienzen sowie zu langen Lebensdauern. Dies gilt insbesondere, wenn die Verbindungen als Matrixmaterial für einen phosphoreszierenden Emitter eingesetzt werden.
2. Die erfindungsgemäßen Verbindungen bzw. Verbindungen gemäß Formel (27) oder Formel (28) eignen sich nicht nur als Matrix für grün und rot phosphoreszierende Verbindungen, sondern auch für blau phosphoreszierende Verbindungen.
3. Auch bei Verwendung als Elektronentransportmaterial zeigen die erfindungsgemäßen Verbindungen bzw. Verbindungen gemäß Formel (27) oder Formel (28) gute Eigenschaften.
4. Im Gegensatz zu vielen Verbindungen gemäß dem Stand der Technik, die der teilweisen oder vollständigen pyrolytischen Zersetzung bei Sublimation unterliegen, weisen die erfindungsgemäßen Verbindungen eine hohe thermische Stabilität auf.
5. Die erfindungsgemäßen Verbindungen, eingesetzt in organischen Elektrolumineszenzvorrichtungen, führen zu hohen Effizienzen und zu steilen Strom-Spannungs-Kurven mit niedrigen Einsatzspannungen.

Diese oben genannten Vorteile gehen nicht mit einer Verschlechterung der weiteren elektronischen Eigenschaften einher.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

### Beispiele

Die nachfolgenden Synthesen werden, sofern nicht anders angegeben, unter einer Schutzgasatmosphäre in getrockneten Lösungsmitteln durchgeführt. Die Edukte **(I),** (**III**), (**VI**), **(VII)** und **(XIII)** können kommerziell bezogen werden (Sigma-Aldrich, Alessa Syntec, VWR, Carbone Scientific Co., Ltd).

### Beispiel 1

### Herstellung der Verbindungen (II), (IV), (V), (VIII), (IX) und (X)

### Syntheseprozedur für die Darstellung von Verbindung (X):

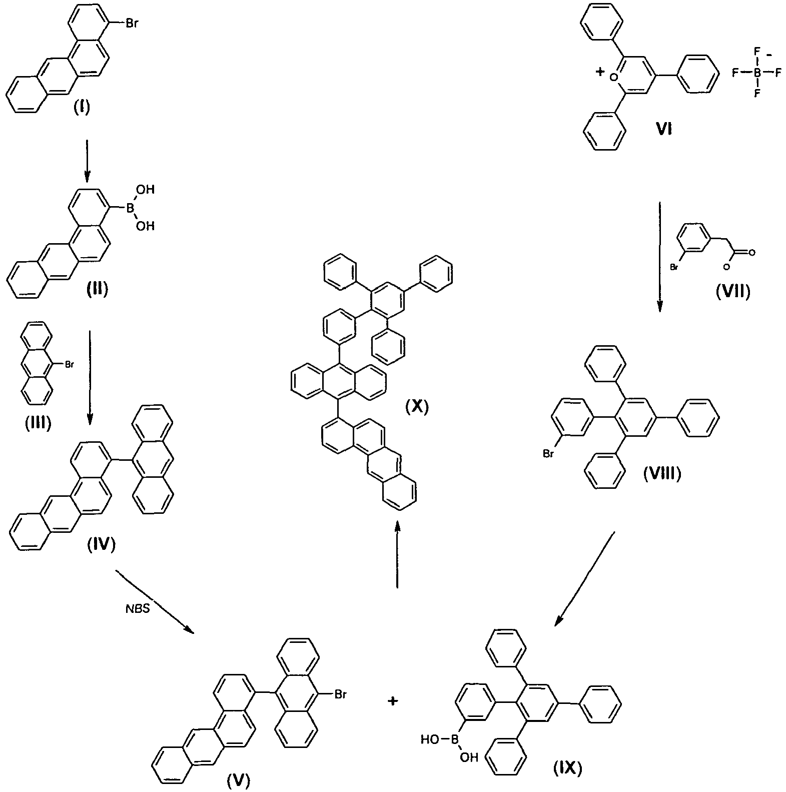

### a) Synthese von Verbindung (II)

Eine Suspension von 30.7 g (100 mmol) 4-Brombenz[a]anthracen **(I)** in 1000 ml THF wird bei -78°C unter gutem Rühren tropfenweise mit 52 ml (130 mmol) *n*-Butyllithium (2.5 M in *n*-Hexan) versetzt und 2h nachgerührt. Die rote Lösung wird unter gutem Rühren auf einmal mit 16.7 ml (150 mmol) Trimethylborat versetzt, 30 min. bei -78°C nachgerührt, dann während 3h auf Raumtemperatur erwärmt, mit 300 ml Wasser versetzt und 30 min. gerührt. Die organische Phase wird abgetrennt und im Vakuum zur Trockene eingeengt. Der Feststoff wird in 100 ml *n*-Hexan aufgenommen, abgesaugt, einmal mit 100 ml *n*-Hexan gewaschen und im Vakuum getrocknet. Ausbeute: 23.7 g (87.0 mmol), 87.0%, Reinheit ca. 90.0%-ig (NMR) an Boronsäure, mit wechselnden Mengen an Boronsäureanhydrid und Borinsäure. Die Boronsäure kann ohne weitere Reinigung in dieser Form verwendet werden.

### b) Synthese von Verbindung (IV)

25.0 g (97.2 mmol) 9-Bromanthracen (**III**), 27.0 g (99.2 mmol) Benz[a]anthracen-4-boronsäure (**II**) und 44.5 g (210 mmol) Trikaliumphosphat werden in 500 mL Toluol, 600 mL Wasser und 100 ml Dioxan suspendiert. Zu dieser Suspension werden 1.83 g (6.01 mmol) Trio-tolylphosphin und dann 225 mg (1.00 mmol) Palladium(II)acetat gegeben und anschließend 16h unter Rückfluss erhitzt. Nach Erkalten wird die organische Phase abgetrennt, dreimal mit 500 mL Wasser gewaschen, mit Natriumsulfat getrocknet und anschließend zur Trockene eingeengt. Der Feststoff wird aus 300 ml Toluol umkristallisiert und abschließend bei vermindertem Druck getrocknet. Die Ausbeute beträgt 26.2 g (64.8 mmol), entsprechend 64.8% der Theorie.

### c) Synthese von Verbindung (V)

Zu einer auf 0°C gekühlten Suspension von 26.0 g (64.3 mmol) der Verbindung **(IV)** in 600 ml Chloroform werden 1.30 g (8.02 mmol) Eisen(III)chlorid und dann 13.3 g (74.7 mmol) N-Bromsuccinimid gegeben und 4h bei 0°C gerührt. Nach Erwärmen auf Raumtemperatur werden 400 ml Wasser zugegeben, die organische Phase abgetrennt, dreimal mit 300 mL Wasser gewaschen, mit Natriumsulfat getrocknet und anschließend zur Trockene eingeengt. Der erhaltene orangefarbene Feststoff wird aus Toluol umkristallisiert und abschließend bei vermindertem Druck getrocknet. Die Ausbeute beträgt 23.7 g (49.0 mmol), entsprechend 76.6% der Theorie.

### d) Synthese von Verbindung (VIII)

8.60 g (159 mmol) Natriummethanolat werden mit 33.0 g (153 mmol) 3-Bromphenylessigsäure **(VII)** in 86 ml Methanol 30 min gerührt und anschließend zur Trockene eingeengt. Das entstandene 3-Brombenzoat wird mit 30.4 g (76.7 mmol) 2,4,6-Triphenylpyryliumtetrafluoroborat (**VI**) in 165 ml Essigsäureanhydrid 4h refluxiert. Nach Erkalten wird das Essigsäureanhydrid per Destillation entfernt, der Rückstand wird in 400 ml Dichlormethan aufgenommen und zweimal mit je 200 ml Wasser gewaschen, mit Natriumsulfat getrocknet und anschließend zur Trockene eingeengt. Die dunkelgrüne Flüssigkeit wird mit einem Heptan/Ethylacetat-Gemisch (9:1) über Kieselgel filtriert, entsprechende Fraktionen werden zur Trockene eingeengt. Der erhaltene orangefarbene Feststoff wird aus Ethanol umkristallisiert und abschließend bei vermindertem Druck getrocknet. Die Ausbeute beträgt 18.1 g (39.2 mmol), entsprechend 51.1 % der Theorie.

### e) Synthese von Verbindung (IX)

Eine Suspension von 10.0 g (21.7 mmol) der Verbindung **(VIII)** in 500 ml THF wird bei -78°C unter gutem Rühren tropfenweise mit 8.80 ml (22.0 mmol) *n*-Butyllithium (2.5 M in *n*-Hexan) versetzt und 1 h nachgerührt. Die Lösung wird unter gutem Rühren auf einmal mit 5.10 ml (22.2 mmol) Triisopropylborat versetzt, 2h bei -78°C nachgerührt, dann während 1 h auf Raumtemperatur erwärmt. Nach dem Abkühlen auf 0°C werden 200 ml 0.2 N Salzsäure zugetropft und 300 ml Ethylacetat zugegeben. Die wässrige Phase wird abgetrennt, zweimal mit 200 ml Ethylacetat extrahiert, die vereinigten organischen Phasen werden einmal mit 200 ml Wasser, einmal mit 200 ml gesättigter Natriumhydrogencarbonat-Lösung und einmal mit 200 ml gesättigter Natriumchlorid-Lösung gewaschen, mit Natriumsulfat getrocknet und anschließend zur Trockene eingeengt. Der erhaltene farblose Feststoff wird heiß in einem Heptan/Toluol-Gemisch gerührt, abgesaugt und abschließend bei vermindertem Druck getrocknet. Die Ausbeute beträgt 7.08 g (16.6 mmol), entsprechend 76.2% der Theorie.

### f) Synthese von Verbindung (X)

900 mg (1.86 mmol) der Verbindung **(V),** 880 mg (2.06 mmol) der Verbindung (**IX**) und 3.50 ml einer 2.0 M Natriumcarbonat-Lösung (7.00 mmol) werden in 15 ml Toluol und 15 ml Ethanol suspendiert. Zu dieser Suspension werden 30.0 mg (26.0 µmol) Tetrakis(triphenyl-phosphin)-palladium(0) gegeben und anschließend 16h unter Rückfluss erhitzt. Nach Erkalten wird die organische Phase abgetrennt, dreimal mit 20 ml Wasser und einmal mit 20 ml gesättigter Natriumchlorid-Lösung gewaschen, mit Natriumsulfat getrocknet und anschließend zur Trockene eingeengt. Der Rückstand wird mit einem Heptan/Ethylacetat-Gemisch (9:1) über Kieselgel filtriert, entsprechende Fraktionen zur Trockene eingeengt. Der erhaltene Feststoff wird zweimal aus einem Toluol/Ethanol-Gemisch umkristallisiert und abschließend bei vermindertem Druck getrocknet. Die Ausbeute beträgt 1.18 g (1.50 mmol), entsprechend 78.7 % der Theorie.

### Beispiel 2

### Herstellung der Verbindungen (XI), (XII) und (XIV) bis (XVIII)

### Syntheseprozedur für die Darstellung von Verbindung (XVIII):

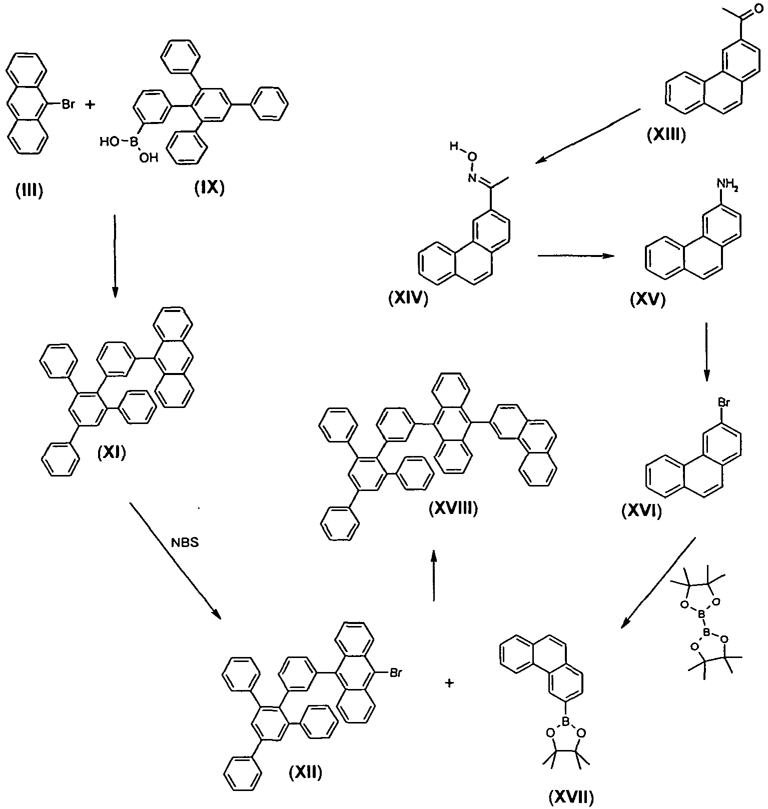

### a) Synthese von Verbindung (XI)

2.70 g (10.5 mmol) 9-Bromanthracen (**III**), 5.00 g (11.7 mmol) der Verbindung (**IX**) und 3.90 g (36.8 mmol) Natriumcarbonat werden in 60 ml Toluol, 60 ml Ethanol und 14 ml Wasser suspendiert. Zu dieser Suspension werden 130 mg (0.112 mmol) Tetrakis(triphenylphosphin)-palladium(0) hinzugegeben und anschließend wird 16h unter Rückfluss erhitzt. Nach Erkalten wird die organische Phase abgetrennt, dreimal mit 50 ml Wasser gewaschen, mit Natriumsulfat getrocknet und anschließend zur Trockene eingeengt. Der Feststoff wird aus Toluol umkristallisiert und abschließend bei vermindertem Druck getrocknet. Die Ausbeute beträgt 5.19 g (9.29 mmol), entsprechend 88.5% der Theorie.

### b) Synthese von Verbindung (XII)

Zu einer auf 0 °C gekühlten Suspension von 5.00 g (8.95 mmol) der Verbindung (**XI**) in 90 ml Chloroform werden 150 mg (0.925 mmol) Eisen(III)chlorid und dann 1.90 g (10.7 mmol) N-Bromsuccinimid gegeben und 2h bei 0°C gerührt. Nach Erwärmen auf Raumtemperatur werden 100 ml Wasser zugegeben, die organische Phase abgetrennt, dreimal mit 50 ml Wasser gewaschen, mit Natriumsulfat getrocknet und anschließend zur Trockene eingeengt. Der erhaltene Feststoff wird aus einem Heptan-Ethylacetat-Gemisch umkristallisiert und abschließend bei vermindertem Druck getrocknet. Die Ausbeute beträgt 4.30 g (6.74 mmol), entsprechend 75.4% der Theorie.

### c) Synthese von Verbindung (XIV)

200 g (908 mmol) 3-Acetylphenanthren (**XIII**), 167 g (2.36 mol) Hydroxylammoniumchlorid und 257 ml (3.18 mmol) Pyridin werden in 800 ml Ethanol suspendiert und 2h bei 100°C Badtemperatur refluxiert. Nach Erkalten werden 700 ml Ethylacetat und 700 ml Wasser zugegeben, die organische Phase abgetrennt, dreimal mit 500 ml Wasser gewaschen, mit Natriumsulfat getrocknet und anschließend zur Trockene eingeengt. Der Rückstand wird in 500 ml Ethanol ausgerührt und abschließend bei vermindertem Druck getrocknet. Die Ausbeute beträgt 160 g (682 mmol), entsprechend 75.1% der Theorie.

### d) Synthese von Verbindung (XV)

1.50 kg (15.3 mol) Polyphosphorsäure werden auf 100°C erhitzt, 160 g (680 mmol) der Verbindung **(XIV)** werden portionsweise innerhalb einer Stunde zugegeben und weitere 20 min bei 100°C gerührt. Nach Erkalten werden vorsichtig 1000 ml Eiswasser zugegeben und 30 min bei Raumtemperatur gerührt. Der entstandene Feststoff wird abgesaugt, mit Wasser nachgewaschen und bei vermindertem Druck getrocknet. Der Feststoff wird mit 200 ml 37%-iger Salzsäure und 2.50 I Methanol versetzt und 16h refluxiert. Der Großteil des Methanols wird unter vermindertem Druck entfernt, der hellgrüne Feststoff wird abgesaugt. Dieser wird in 500 ml Wasser suspendiert, mit 300 ml 30%-iger NaOH-Lösung neutralisiert und dreimal mit je 300 ml Ethylacetat extrahiert. Die vereinigten organischen Phasen werden zweimal mit 300 ml Wasser gewaschen, mit Natriumsulfat getrocknet und anschließend zur Trockene eingeengt. Der Rückstand wird mit einem Heptan/Ethylacetat-Gemisch (3:1) über Kieselgel filtriert, entsprechende Fraktionen eingeengt und abschließend bei vermindertem Druck getrocknet. Die Ausbeute beträgt 82.0 g (423 mmol), entsprechend 62.3% der Theorie.

### e) Synthese von Verbindung (XVI)

82.0 g (423 mmol) Phenanthren-3-ylamin **(XV)** und 95.0 g (425 mmol) Kupfer(II)bromid werden in 1.70 I Acetonitril suspendiert und auf 0°C gekühlt. 100 ml (1.33 mol) tert-Butylnitrit werden innerhalb von 45 min so zugetropft, dass eine Innentemperatur von 5 °C nicht überschritten wurde und 1 h bei 0°C nachgerührt. Der Ansatz wird auf 1.50 kg Eis gegeben, 30 min nachgerührt, der dunkelbraune Feststoff wird abgesaugt und verworfen. Die Mutterlauge wird mit 1000 ml Ethylacetat versetzt, die organische Phase abgetrennt, zweimal mit je 500 ml 2 N HCl und zweimal mit je 500 ml Wasser gewaschen, mit Natriumsulfat getrocknet und anschließend zur Trockene eingeengt. Der Rückstand wird mit einem Heptan/Ethylacetat-Gemisch (10:1) über Kieselgel filtriert, entsprechende Fraktionen eingeengt und abschließend bei vermindertem Druck getrocknet. Die Ausbeute beträgt 35.9 g (140 mmol), entsprechend 32.9% der Theorie.

### f) Synthese von Verbindung (XVII)

35.6 g (138 mmol) 3-Bromphenanthren **(XVI),** 42.0 g (165 mmol) Bispinakolatodiboron und 46.0 g (469 mmol) Kaliumacetat werden in 500 ml Dimethylsulfoxid suspendiert. Zu dieser Suspension werden 3.50 g (4.29 mmol) 1,1-Bis(diphenylphosphino)ferrocen-dichloro-palladium(II)*DCM gegeben, und die Reaktionsmischung wird 6h bei 80°C gerührt. Nach Erkalten werden 1000 ml Ethylacetat und 1000 ml Wasser hinzugegeben, die organische Phase wird abgetrennt, dreimal mit je 300 ml Wasser gewaschen, mit Natriumsulfat getrocknet und anschließend zur Trockene eingeengt. Das Rohprodukt wird mit einem Heptan/Ethylacetat-Gemisch (10:1) über Kieselgel gesäult, entsprechende Fraktionen eingeengt und abschließend bei vermindertem Druck getrocknet. Die Ausbeute beträgt 38.5 g (127 mmol), entsprechend 92.0% der Theorie.

### g) Synthese von Verbindung (XVIII)

4.20 g (6.59 mmol) der Verbindung (**XII**), 2.20 g (7.23 mmol) der Verbindung **(XVII)** und 2.50 g (23.6 mmol) Natriumcarbonat werden in 40 ml Toluol, 40 ml Ethanol und 10 ml Wasser suspendiert. Zu dieser Suspension werden 100 mg (87.0 µmol) Tetrakis(triphenylphosphin)-palladium(0) gegeben und anschließend 16h unter Rückfluss erhitzt. Nach Erkalten wird die organische Phase abgetrennt, dreimal mit 50 ml Wasser und einmal mit 50 ml gesättigter Natriumchlorid-Lösung gewaschen, mit Natriumsulfat getrocknet und anschließend zur Trockene eingeengt. Der Rückstand wird aus einem Heptan-Toluol-Gemisch umkristallisiert und mit Toluol heiß extrahiert. Die Ausbeute beträgt 4.08 g (5.55 mmol), entsprechend 84.3% der Theorie.

### Beispiel 3 (Vergleichsbeispiel):

### Herstellung der Verbindung (XX)

### Syntheseprozedur für die Darstellung von Verbindung (XX)

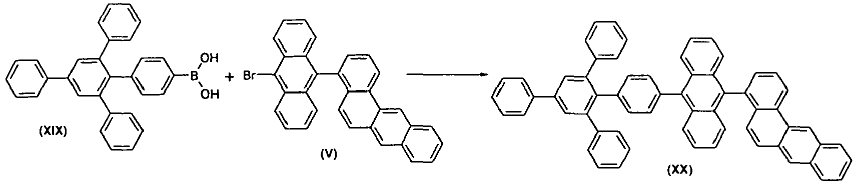

Die Synthese der Verbindung **(XX)** wird analog zu der von Verbindung **(X)** durchgeführt, wobei die Verbindung (**IX**) durch 880 mg (2.06 mmol) der Verbindung (**XIX**) ersetzt wird. Die Ausbeute beträgt 1.10 g (1.40 mmol), entsprechend 75.3% der Theorie.

### Beispiel 4 (Vergleichsbeispiel):

### Herstellung der Verbindung (XXIV)

### Syntheseprozedur für die Darstellung von Verbindung (XXIV)

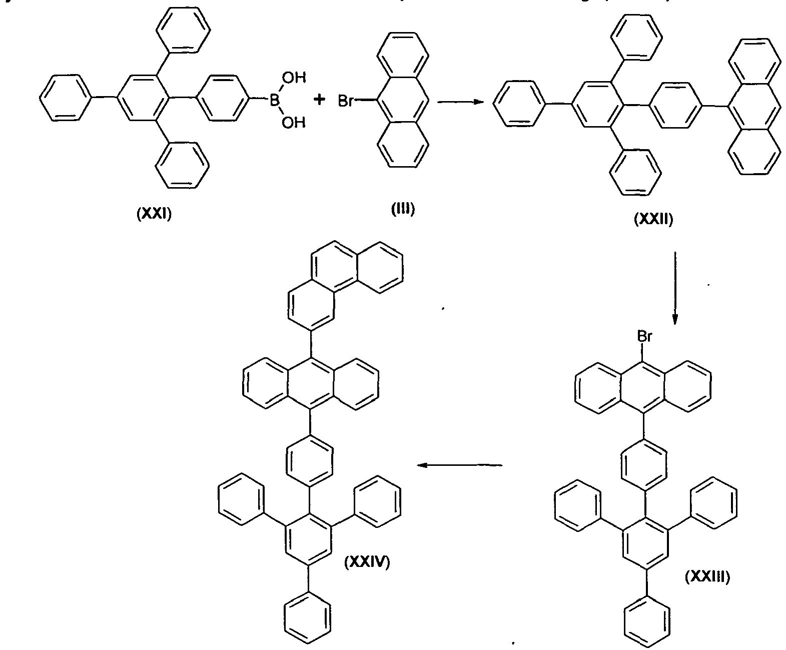

### a) Synthese von Verbindung (XXII)

Die Synthese der Verbindung **(XXII)** wird analog zu der von Verbindung (**XI**) durchgeführt, wobei die Verbindung (**IX**) durch 5.00 g (11.7 mmol) der Verbindung (**XXI**) ersetzt wird. Die Ausbeute beträgt 5.01 g (8.96 mmol), entsprechend 85.4% der Theorie.

### b) Synthese von Verbindung (XXIII)

Die Synthese der Verbindung **(XXIII)** wird analog zu der von Verbindung (**XII**) durchgeführt, wobei die Verbindung (**XI**) durch 5.00 g (8.95 mmol) der Verbindung (**XXII**) ersetzt wird. Die Ausbeute beträgt 4.46 g (6.99 mmol), entsprechend 78.1 % der Theorie.

### c) Synthese von Verbindung (XXIV)

Die Synthese der Verbindung (**XXIV**) wird analog zu der von Verbindung **(XVIII)** durchgeführt, wobei die Verbindung **(XII)** durch 4.20 g (6.59 mmol) der Verbindung (**XXIII**) ersetzt wird. Die Ausbeute beträgt 4.03 g (5.48 mmol), entsprechend 83.2% der Theorie.

### Beispiel 5:

### Herstellung und Charakterisierung fluoreszierender organischer Elektrolumineszenzvorrichtungen, enthaltend die erfindungsgemäßen Verbindungen

Die Strukturen von SEB-1 (synthetisiert nach WO 08/006449) und ETM-1 (WO 2005/053055) sind der Übersichtlichkeit halber im Folgenden abgebildet.

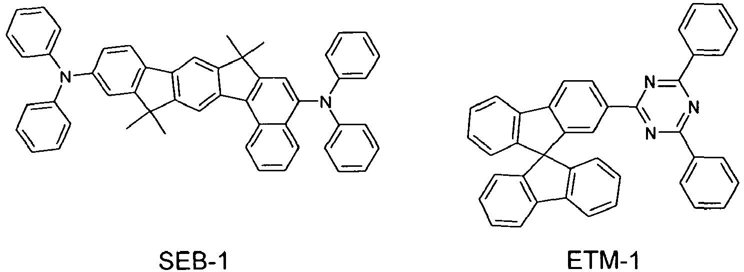

Die erfindungsgemäßen Materialien können aus Lösung verwendet werden und führen dort zu einfachen Vorrichtungen mit dennoch sehr guten Eigenschaften. Zur Herstellung der beschriebenen Vorrichtungen werden Techniken verwendet, die dem Fachmann auf dem Gebiet sehr gut bekannt sind. Die Herstellung solcher Bauteile lehnt sich an die Herstellung polymerer Leuchtdioden (PLEDs) an, die in der Literatur bereits vielfach beschrieben ist (z. B. in der WO 2004/037887). Im vorliegenden Fall werden die erfindungsgemäßen Verbindungen **(X)** und **(XVIII)** bzw. die ebenfalls löslichen Vergleichsverbindungen **(XX)** und (**XXIV**) in Toluol gelöst. Der Emitteranteil liegt bei 5 Gew.%. Der typische Feststoffgehalt solcher Lösungen liegt bei 15 g/l.

Der Aufbau der Vorrichtung ist wie folgt: Katode (Al 100 nm)/ETL (20 nm)/EML (50 nm)/ Zwischenschicht (20 nm)/Pufferschicht (PEDOT:PSS, 20 nm)/Anode, wobei ETL die Elektronentransportschicht ist, die 20 Gew.-% der Verbindung ETM-1 und 80 Gew.-% Liq enthält und EML die emittierende Schicht darstellt. Die emittierende Schicht enthält dabei 95 Gew.-% des erfindungsgemäßen Hostmaterials und 5 Gew.-% des Emitters SEB-1. Strukturierte ITO-Substrate (Technoprint) und das Material für die sogenannte Pufferschicht (PEDOT:PSS) (als wässrige Dispersion Clevios Baytron P von H.C. Starck) sind käuflich erhältlich. Die verwendete Zwischenschicht (Interlayer) dient der Lochinjektion; in diesem Fall wird HIL-012 von Merck KGaA verwendet. Die Emissionsschicht wird in einer Inertgasatmosphäre, im vorliegenden Fall Argon, aufgeschleudert und 10 min bei 120°C ausgeheizt. Zuletzt wird eine Kathode aus Aluminium im Vakuum aufgedampft. Zwischen der emittierenden Schicht und der Kathode können auch eine Lochblockierschicht und/oder eine Elektronentransportschicht per Bedampfung aufgebracht werden, auch kann der Interlayer durch eine oder mehrere Schichten ersetzt werden, die lediglich die Bedingung erfüllen müssen, durch den nachgelagerten Prozessierungsschritt der Abscheidung der emittierenden Schicht aus Lösung nicht wieder abgelöst zu werden.

Die Devices werden standardmäßig mittels Methoden charakterisiert, die dem Fachmann gut bekannt sind. Die genannten OLED-Beispiele sind noch nicht optimiert. Tabelle 1 fasst die erhaltenen Daten zusammen. Bei den prozessierten Devices zeigt sich, dass die erfindungsgemäßen Materialien den zuvor zur Verfügung stehenden in Effizienz und/oder Lebensdauer überlegen sind.

Tabelle 1: Ergebnisse mit aus Lösung prozessierten erfindungsgemäßen Materialien in der beschriebenen Devicekonfiguration (Ba/Al)/EMUZwischenschicht/Pufferschicht/ITO.

| Bsp. | EML 50 nm | Max. Eff. [cd/A] | Spannung [V] bei 1000 cd/m² | CIE (x, y) | Lebensdauer [h], Anfangshelligkeit 1000 cd/m² |
|---|---|---|---|---|---|
| 3 Verql. | **(XX)** : SEB-1 | 4.5 | 4.9 | 0.14, 0.18 | 300 |
| 1 | **(X)** : SEB-1 | 5.9 | 4.8 | 0.14, 0.18 | 500 |
| 4 Verql. | **(XXIV)** : SEB-1 | 4.4 | 4.8 | 0.14, 0.18 | 350 |
| 2 | **(XVIII)** : SEB-1 | 6.1 | 4.8 | 0.14, 0.18 | 600 |

### Beispiel 6

### Herstellung der Verbindung (XXVI)

### Syntheseprozedur für die Darstellung von Verbindung (XXVI)

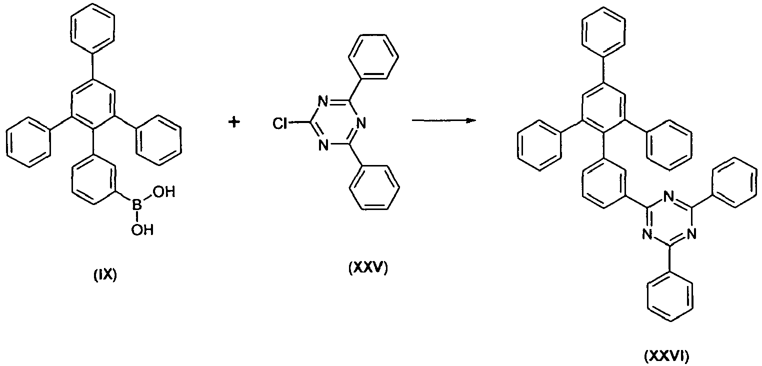

Zu einer Lösung von 33.0 g (310 mol) Natriumcarbonat in dest. Wasser (3 ml) werden 10.0 g (20 mmol) 4,4,5,5-Tetramethyl-2-(3-(2,4,6-triphenyl)phenyl)phenyl-1,3,2-dioxaborolan und 500 ml Toluol hinzugegeben. Zur Reaktionsmischung wird ein Tropfen Aliquat, 5.0 g Verbindung **(XXV)** (19 mmol) und anschließdend 0.2 g Tetrakis(triphenylphosphin)palladium (0.2 mmol). Die Reaktionsmischung wird für zwei Tage bei 90°C gerührt. Nach Zugabe von 150 ml Dichlormethan wird die organische Phase mit einer Natriumchlorid-Lösung (ges.) gewaschen, über Magnesiumsulfat getrocknet und anschließend zur Trockene eingeengt. Der Rückstand wird durch säulenchromatographie über Kieselgel (CH:DCM = 4:1) gereinigt. Anschließend wird das Produkt in Dichlormethan gelöst und aus Methanol ausgefällt. Der farblose Feststoff wird in siedendem Toluol gelöst und durch Zugabe von n-Pentan erneut ausgefällt.. Die Ausbeute beträgt 5.0 g (8 mmol), entsprechend 41.0% der Theorie.

### Beispiel 7 (Vergleichsbeispiel)

### Herstellung der Verbindungen (XXVII) bis (XXIX)

### Syntheseprozedur für die Darstellung von Verbindung (XXIX)

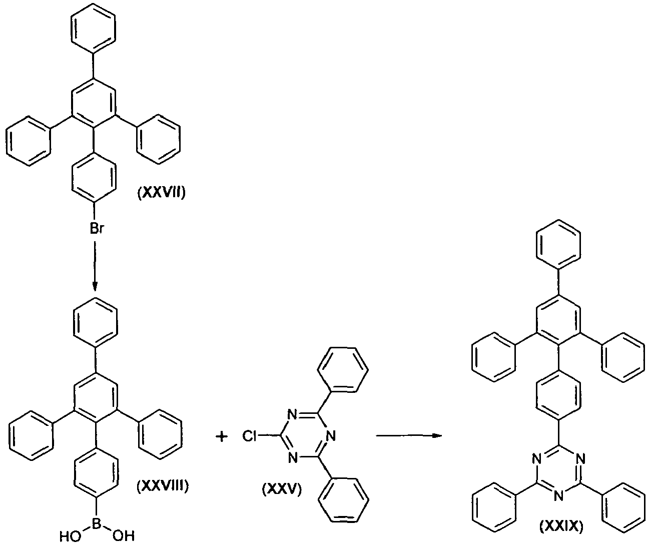

### a) Synthese von Verbindung (XXVII)

Die Synthese der Verbindung **(XXVII)** wird analog zu der von Verbindung (**VIII**) durchgeführt, wobei der 3-Bromphenylessigsäure durch 42.2 g (195 mmol) 4-Bromphenylessigsäure ersetzt wird. Die Ausbeute beträgt 23.2 g (50.2 mmol), entsprechend 50.9% der Theorie.

### b) Synthese von Verbindung (XXVIII)

Die Synthese der Verbindung (**XXVIII**) wird analog zu der von Verbindung (**IX**) durchgeführt, wobei die Verbindung (**VIII**) durch 20.0 g (43.4 mmol) durch Verbindung (**XXVII**) ersetzt wird. Die Ausbeute beträgt 14.20 g (33.2 mmol), entsprechend 76.2% der Theorie.

### c) Synthese von Verbindung (XXIX)

Die Synthese der Verbindung (**XXIX**) wird analog zu der von Verbindung (**XXVI**) durchgeführt, wobei die Verbindung (**IX**) durch 12.0 g (45.5 mmol) Verbindung (**XXVIII**) ersetzt wird. Die Ausbeute beträgt 12.1 g (19.1 mmol), entsprechend 42.0% der Theorie.

### Beispiel 8

### Herstellung und Charakterisierung phosphoreszierender organischer Elektrolumineszenzvorrichtungen, enthaltend die erfindungsgemäßen Verbindungen

Die Strukturen des Emitters TEG-1 (synthetisiert nach WO 2004/085449) und des Co-Hosts C1 (nach WO 2009/124627) sind der Übersichtlichkeit halber im Folgenden abgebildet.

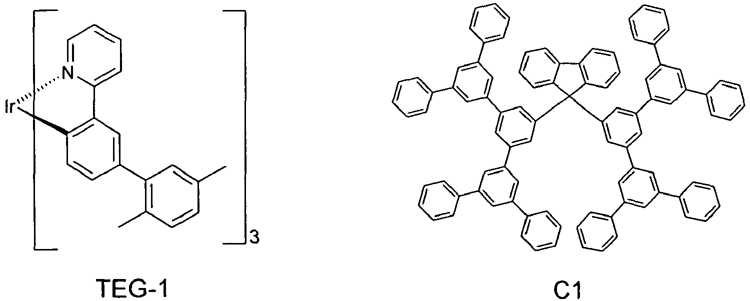

Die erfindungsgemäßen Materialien können aus Lösung verwendet werden und führen dort zu einfachen Vorrichtungen mit dennoch sehr guten Eigenschaften. Zur Herstellung der beschriebenen Vorrichtungen werden Techniken verwendet, die dem Fachmann auf dem Gebiet sehr gut bekannt sind. Die Herstellung solcher Bauteile lehnt sich an die Herstellung polymerer Leuchtdioden (PLEDs) an, die in der Literatur bereits vielfach beschrieben ist (z. B. in der WO 2004/037887). Im vorliegenden Fall werden die erfindungsgemäßen Verbindungen (bspw. die Verbindung der Formel (**XXVI**)) bzw. die ebenfalls lösliche Vergleichsverbindungen (**XXIX**) in Toluol gelöst. Der typische Feststoffgehalt solcher Lösungen liegt zwischen 16 und 25 g/l, wenn, wie hier, die für eine Device typische Schichtdicke von 80 nm mittels Spincoating erzielt werden soll. Der Aufbau der Vorrichtung ist wie folgt: Katode (Ba/Al:3 nm/150 nm)/EML (80 nm)/ Zwischenschicht (20 nm)/Pufferschicht (PEDOT:PSS, 80 nm)/Anode, wobei EML die emittierende Schicht darstellt, die in dem hier vorliegenden Fall entweder 83 Gew.-% des Matrixmaterials (**XXVI** oder **XXIX**) oder 41.5 Gew.-% des Matrixmaterials (**XXVI** oder **XXIX**) und 41.5 Gew.-% des Co-Hosts C1 sowie 17 Gew.-% des Emitters enthält. Strukturierte ITO-Substrate (Technoprint) und das Material für die sogenannte Pufferschicht (PEDOT:PSS) (als wässrige Dispersion Clevios Baytron P von H.C. Starck) sind käuflich erhältlich. Die verwendete Zwischenschicht (Interlayer) dient der Lochinjektion; in diesem Fall wird HIL-012 von Merck KGaA verwendet. Die Emissionsschicht wird in einer Inertgasatmosphäre, im vorliegenden Fall Argon, aufgeschleudert und 10 min bei 120°C ausgeheizt. Zuletzt wird eine Kathode aus Barium und Aluminium im Vakuum aufgedampft. Zwischen der emittierenden Schicht und der Kathode können auch eine Lochblockierschicht und/oder eine Elektronentransportschicht per Bedampfung aufgebracht werden, auch kann der Interlayer durch eine oder mehrere Schichten ersetzt werden, die lediglich die Bedingung erfüllen müssen, durch den nachgelagerten Prozessierungsschritt der Abscheidung der emittierenden Schicht aus Lösung nicht wieder abgelöst zu werden.

Die Devices werden standardmäßig mittels Methoden charakterisiert, die dem Fachmann gut bekannt sind. Die genannten OLED-Beispiele sind noch nicht optimiert. Tabelle 2 fasst die erhaltenen Daten zusammen. Bei den prozessierten Devices zeigt sich, dass die erfindungsgemäßen Materialien den zuvor zur Verfügung stehenden in Effizienz und/oder Lebensdauer überlegen sind.

**Tabelle 2: Ergebnisse mit aus Lösung prozessierten erfindungsgemäßen Materialien in der beschriebenen phosphoreszierenden Devicekonfiguration (Ba/Al)/EML/Zwischenschicht/Pufferschicht/ITO.**

| Bsp. | EML 80 nm | Max. Eff. [cd/A] | Spannung [V] bei 1000 cd/m² | CIE (x, y) | Lebensdauer [h], Anfangshelligkeit 1000 cd/m² |
|---|---|---|---|---|---|
| 7 Vergl. | (**XXIX**) : TEG-1 | 12 | 4.7 | 0.33/0.63 | 8000 |
| 6 | (**XXVI**) : TEG-1 | 20 | 4.6 | 0.33/0.63 | 18000 |
| 7 Vergl. | (**XXIX**) : C1 : TEG-1 | 15 | 4.6 | 0.33/0.63 | 12000 |
| 6 | (**XXVI**) : C1 : TEG-1 | 34 | 4.6 | 0.33/0.63 | 37000 |

## Patentansprüche

1. Verbindung gemäß Formel (27) oder (28) 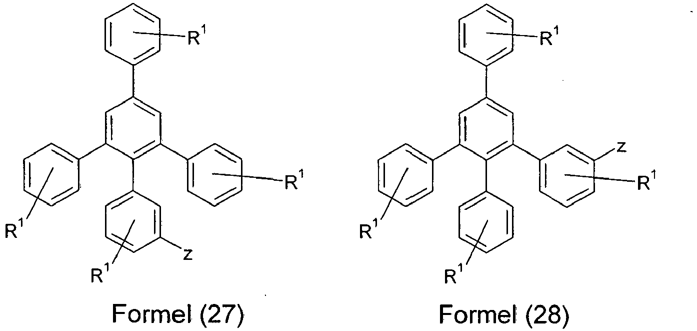 wobei für die verwendeten Symbole und Indizes gilt:
R¹ ist gleich oder verschieden bei jedem Auftreten H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², eine geradkettige Alkyl-, Alkoxy- oder Thioalkoxy-gruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C≡C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy-, Arylalkoxy- oder Heteroaryloxygruppe mit 5 bis 60 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R² substituiert sein kann;
wobei die Reste R¹ an den Ringen maximal einmal auftreten;
R² ist gleich oder verschieden bei jedem Auftreten H, D, F, Cl, Br, I, N(R³)₂, CN, NO₂, Si(R³)₃, B(OR³)₂, C(=O)R³, P(=O)(R³)₂ S(=O)R³, S(=O)₂R³, OSO₂R³, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten R³ substituiert sein kann, wobei eine oder mehrere nicht benachbarte CH₂-Gruppen durch R³C=CR³, C≡C, Si(R³)₂, Ge(R³)₂, Sn(R³)₂, C=O, C=S, C=Se, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R³ substituiert sein kann, oder eine Aryloxy-, Arylalkoxy- oder Heteroaryloxygruppe mit 5 bis 60 aromatischen Ringatomen, die durch einen oder mehrere Reste R³ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R³ substituiert sein kann, oder eine Kombination aus zwei oder mehr dieser Gruppen; dabei können zwei oder mehrere benachbarte Reste R² miteinander ein mono- oder polycyclisches, aliphatisches oder aromatisches Ringsystem bilden;
R³ ist gleich oder verschieden bei jedem Auftreten H, D, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten R³ auch miteinander ein mono- oder polycyclisches, aliphatisches oder aromatisches Ringsystem bilden;
Z ist bei jedem Auftreten gleich oder verschieden ein Rest der folgenden Formeln (99) bis (117) und (121) bis (289), wobei die Reste ihrerseits mit einem oder mehreren Resten R³ substituiert sein können, die bei jedem Auftreten gleich oder verschieden sein können und mit der Maßgabe, dass wenigstens einer der Reste Z eine aromatische oder heteroaromatische Gruppe mit 5 bis 60 aromatischen Ringatomen sein muss

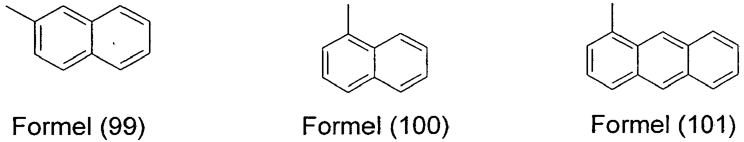

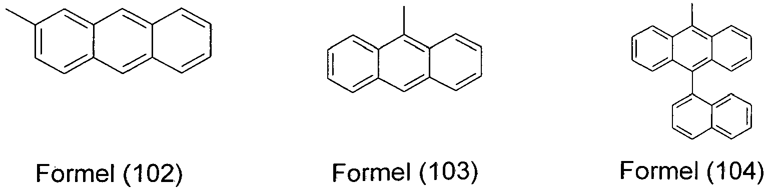

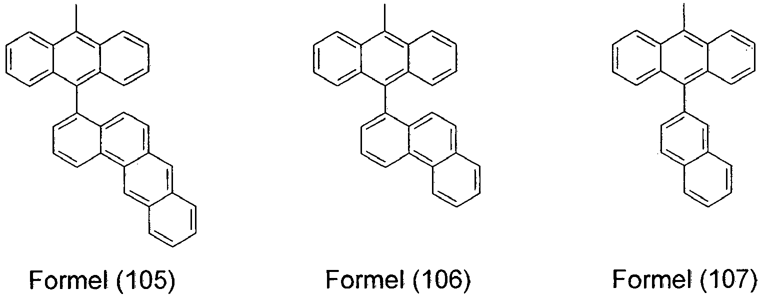

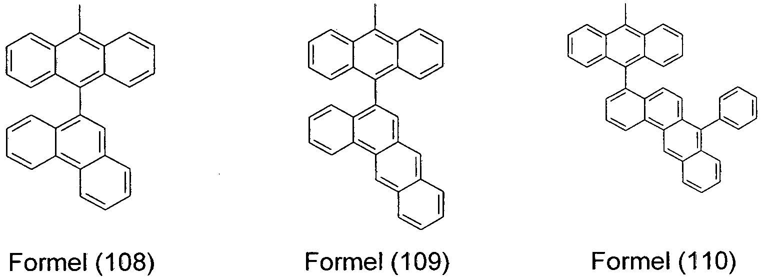

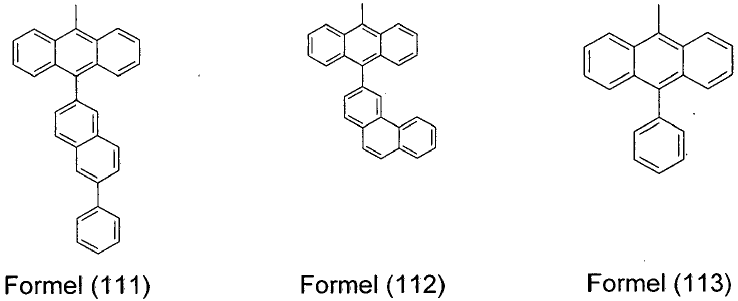

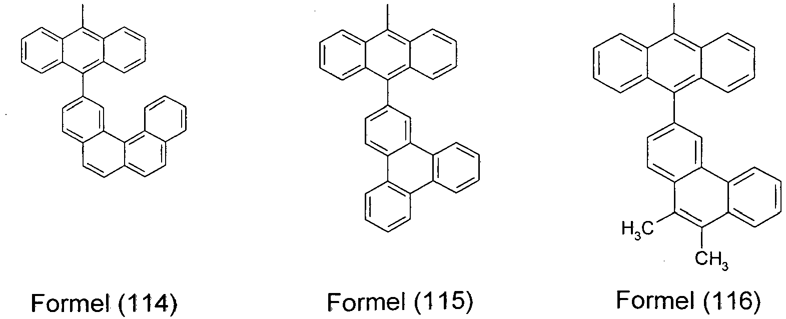

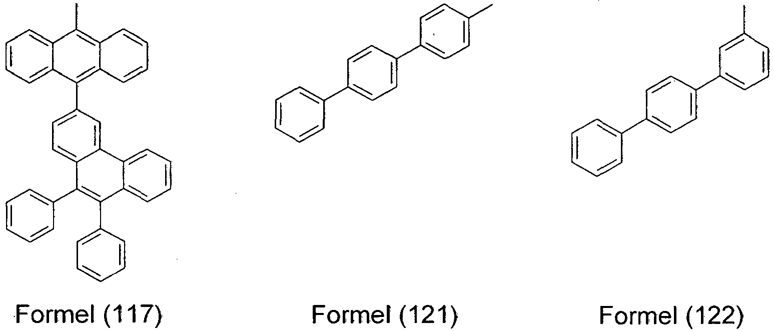

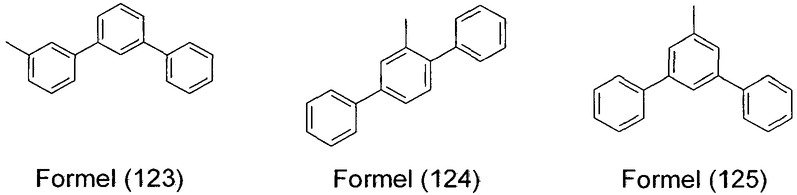

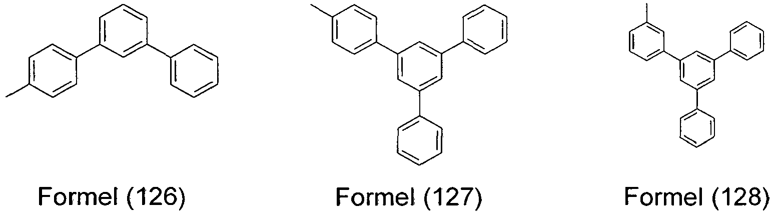

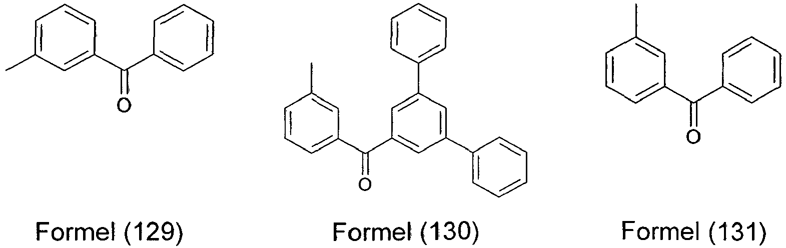

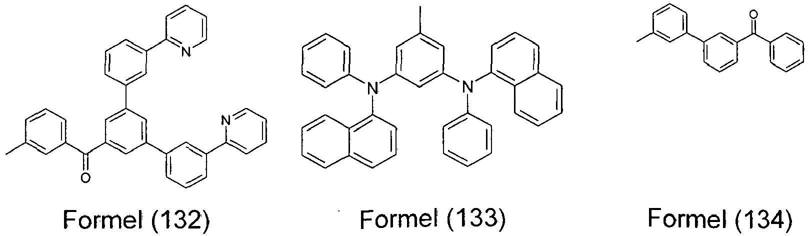

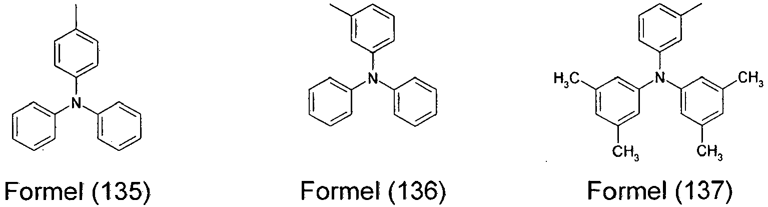

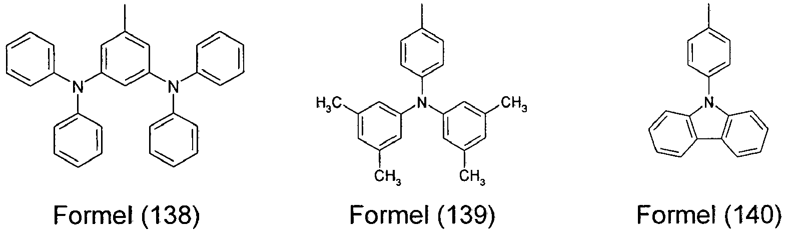

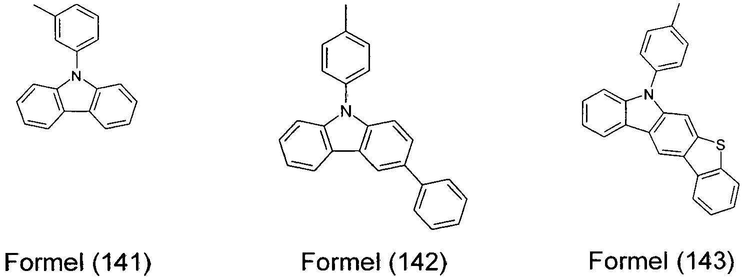

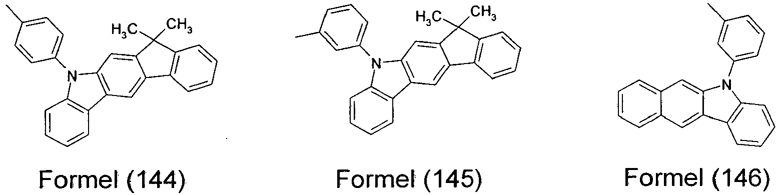

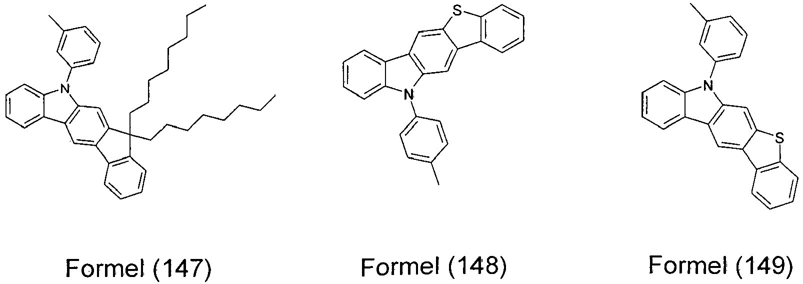

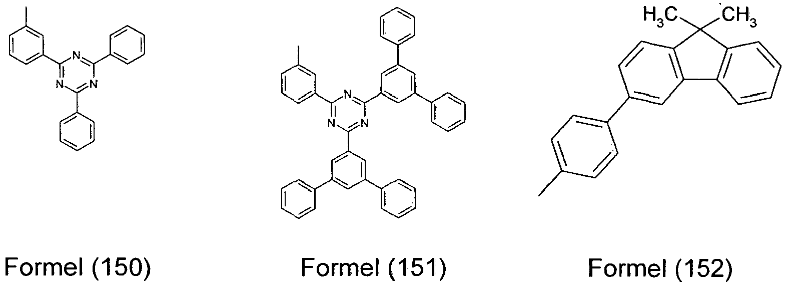

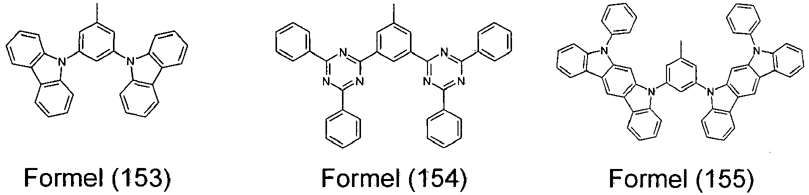

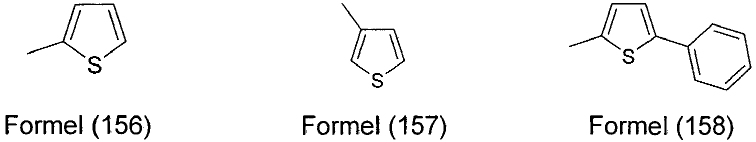

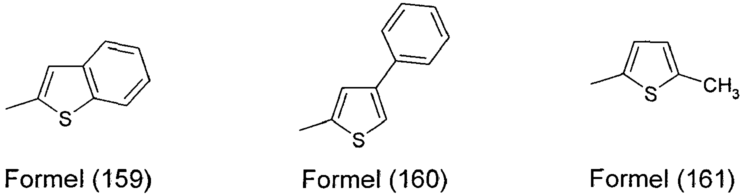

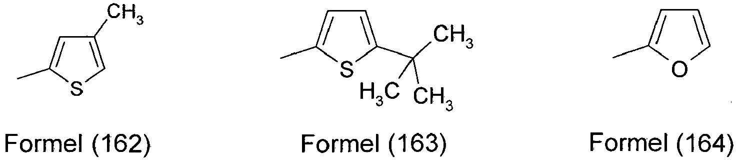

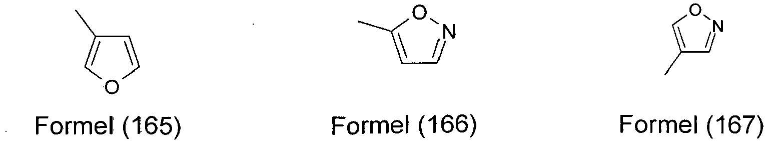

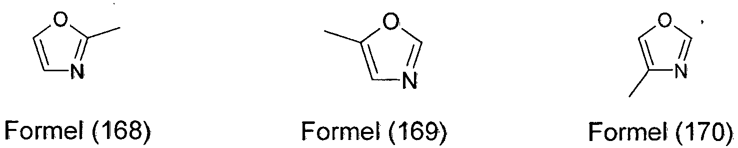

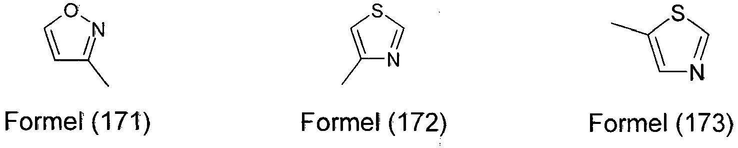

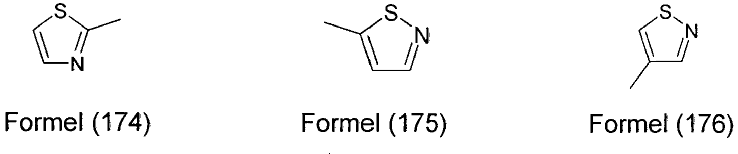

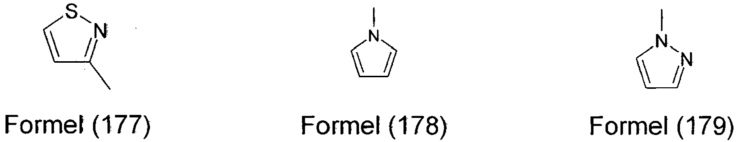

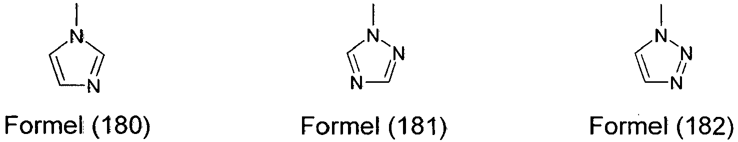

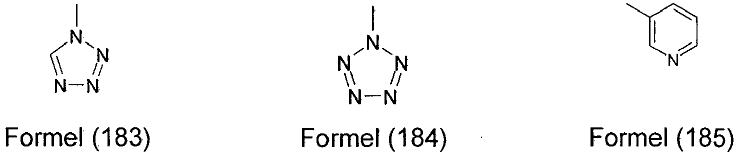

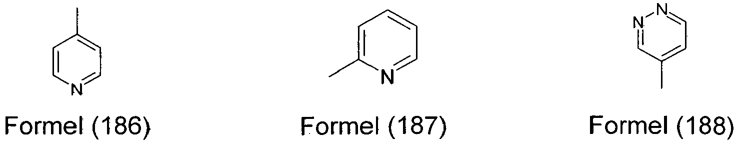

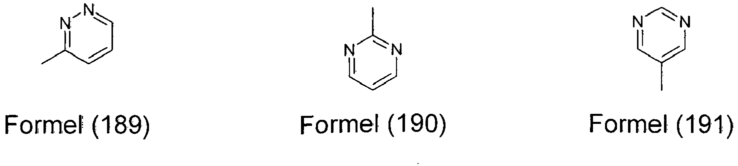

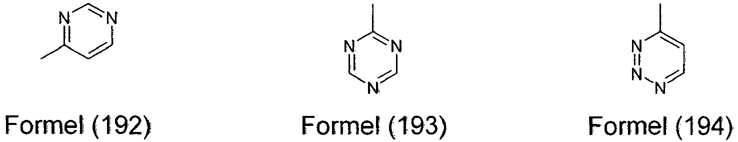

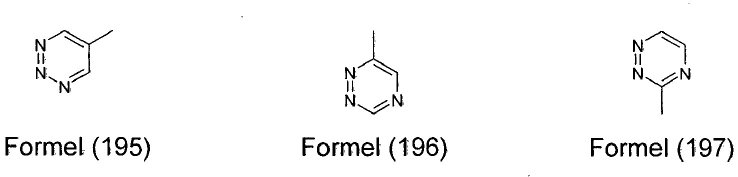

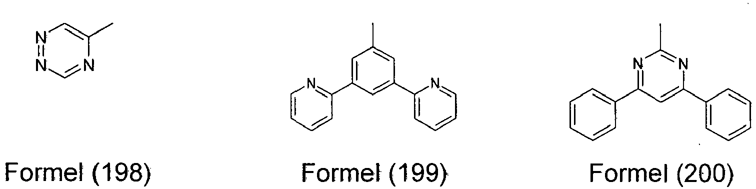

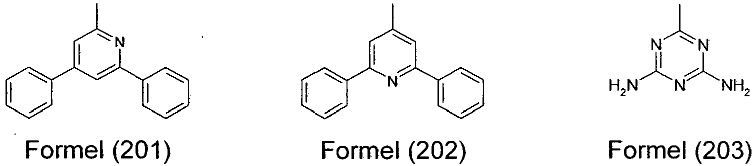

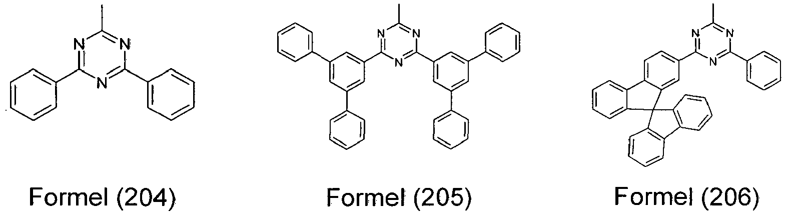

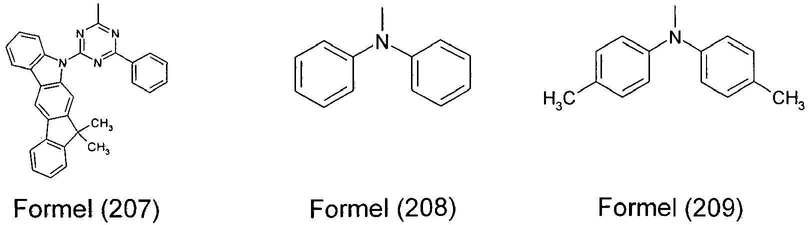

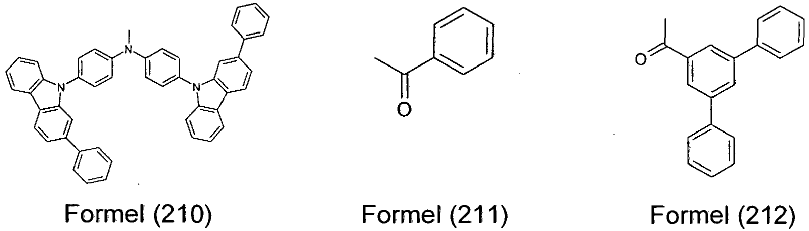

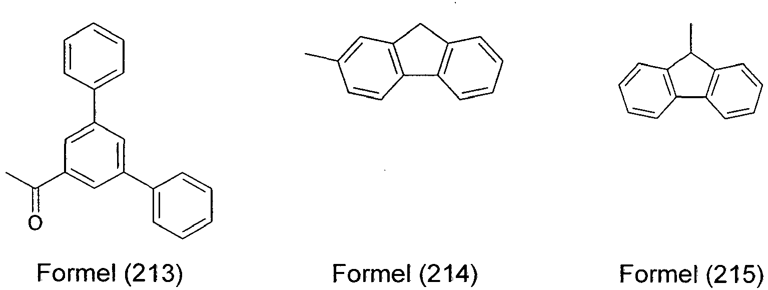

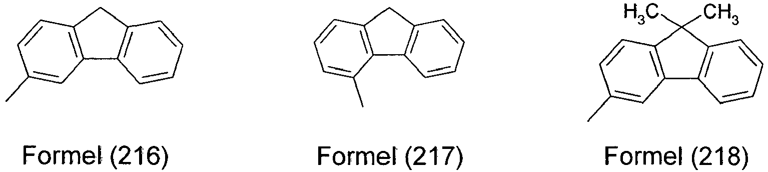

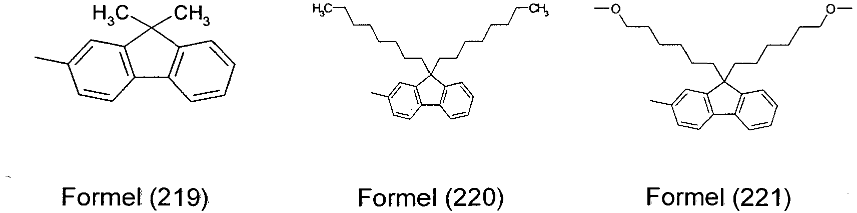

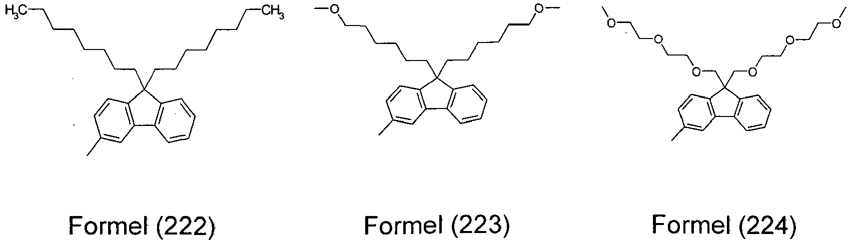

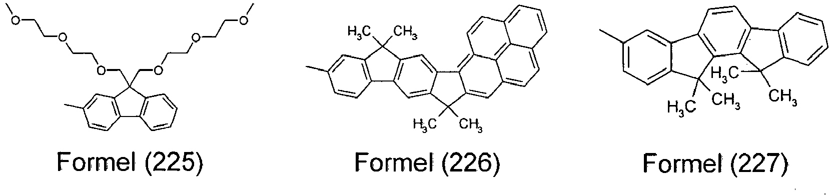

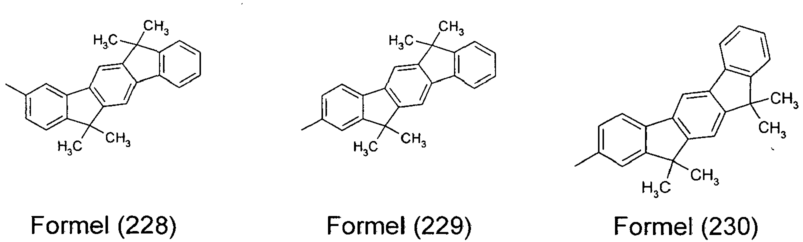

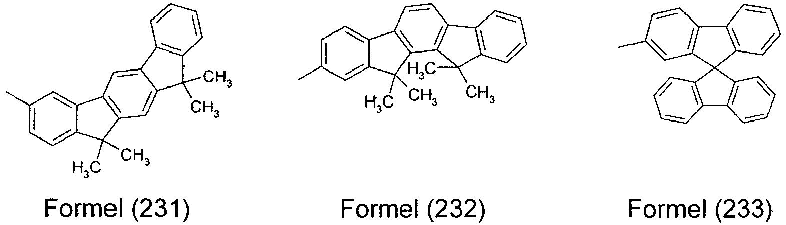

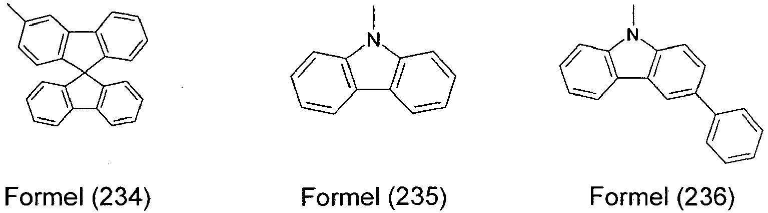

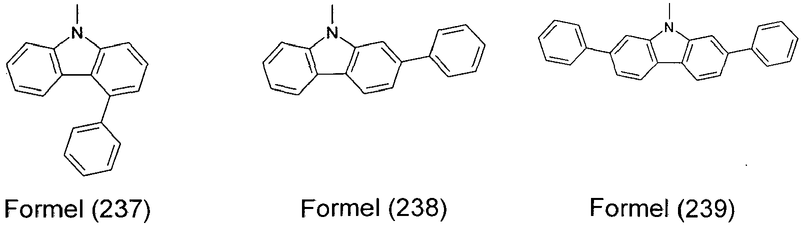

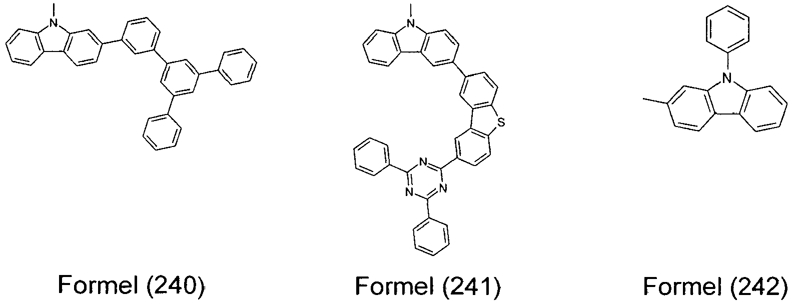

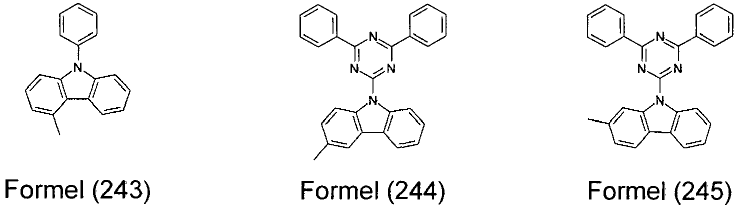

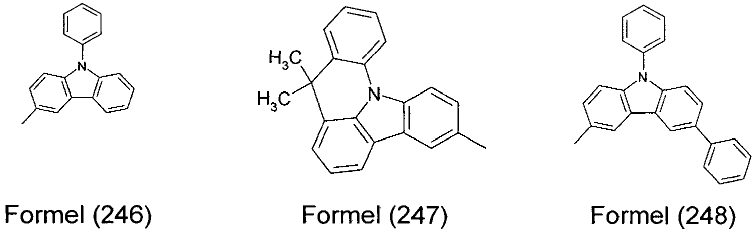

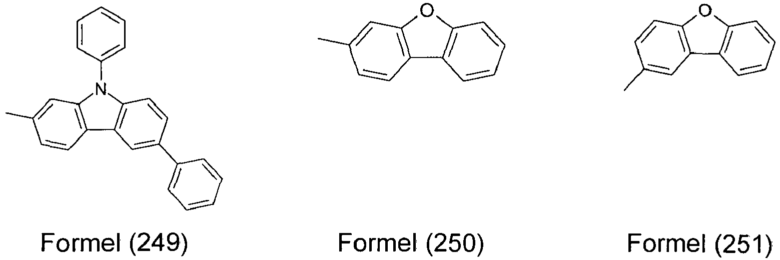

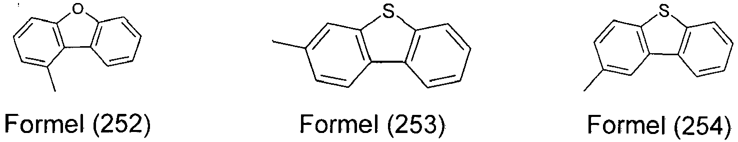

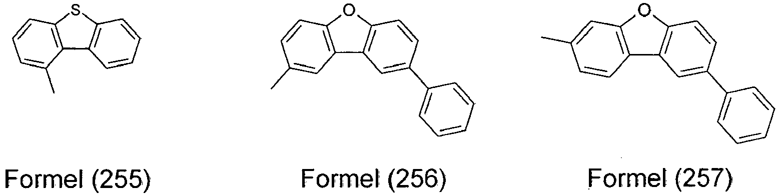

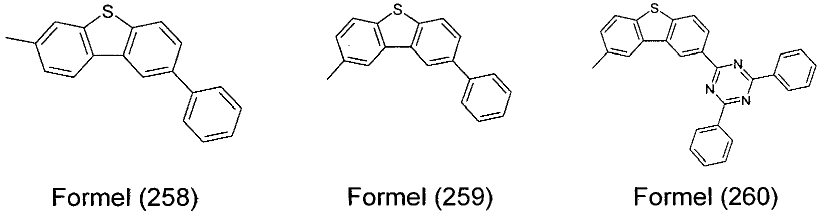

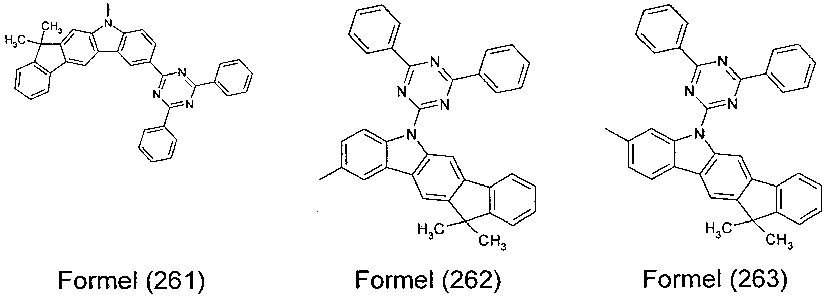

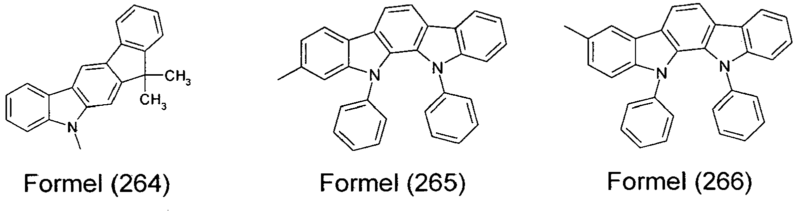

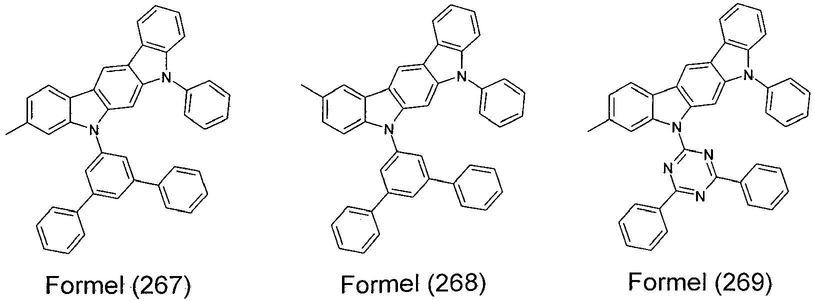

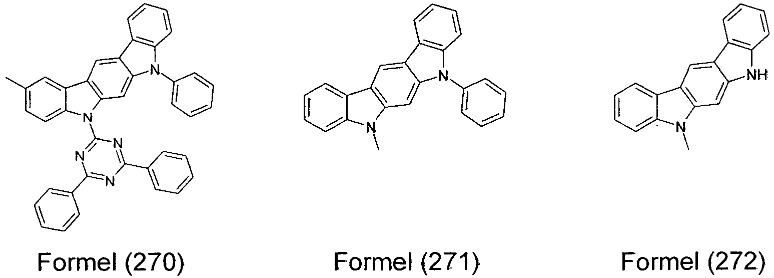

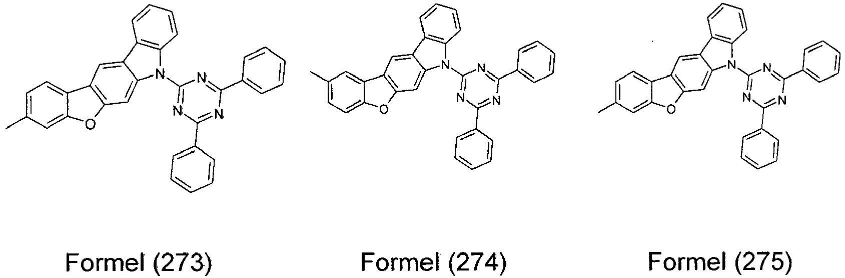

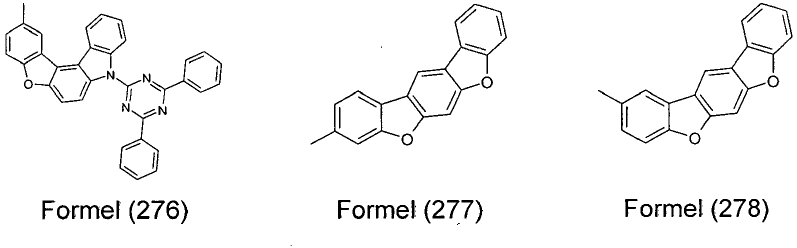

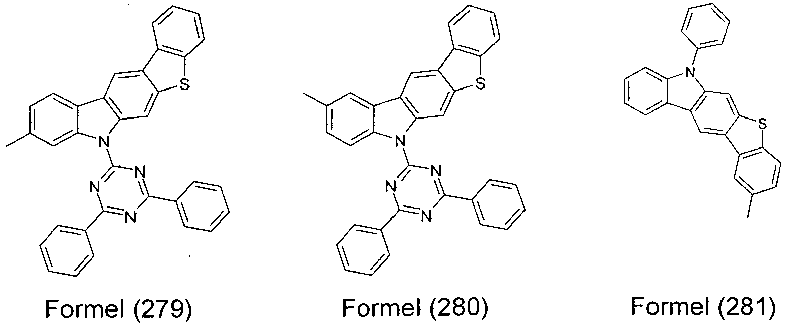

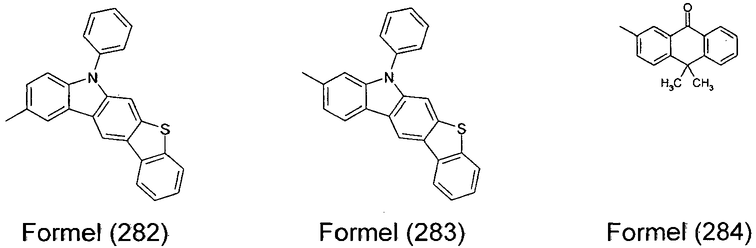

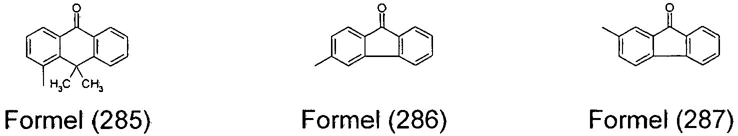

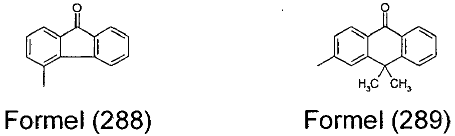

2. Verbindung nach Anspruch 1, **dadurch gekennzeichnet, dass** sie neben dem Rest Z nur einen Rest R¹ enthält.

3. Verbindung nach Anspruch 1 oder 2, **dadurch gekennzeichnet, dass** sie ausgewählt ist aus den Verbindungen der Formeln (30) bis (52).

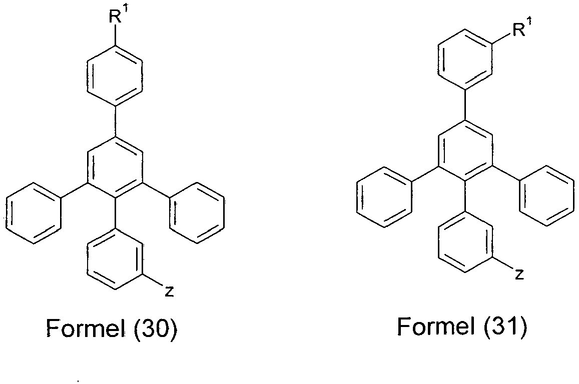

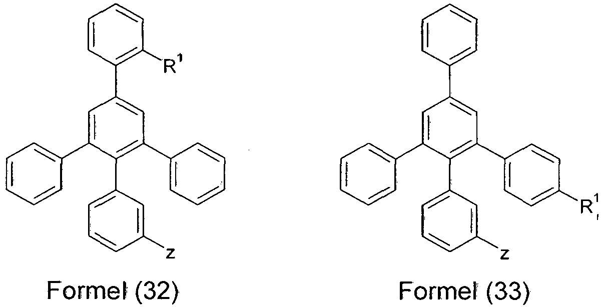

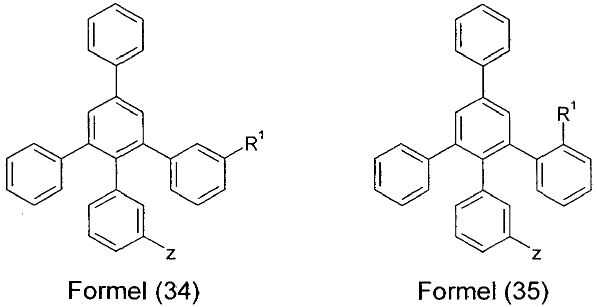

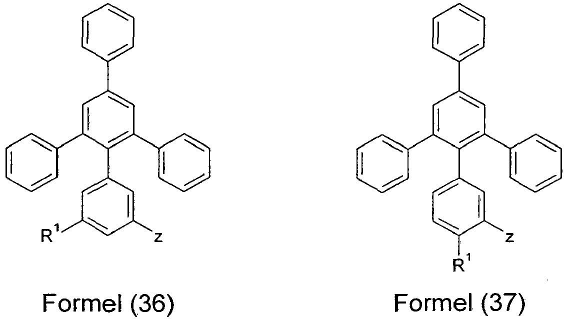

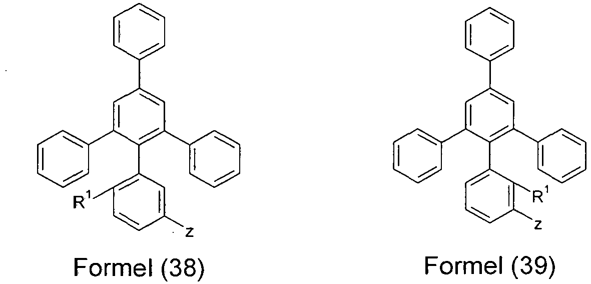

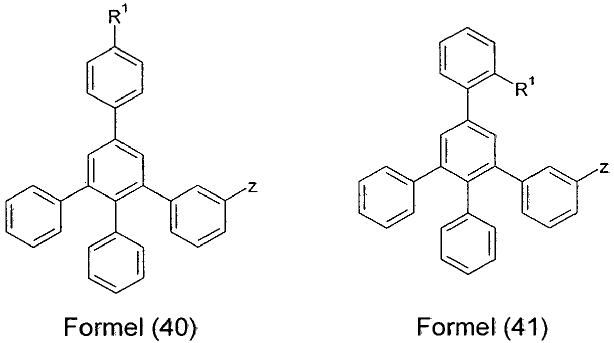

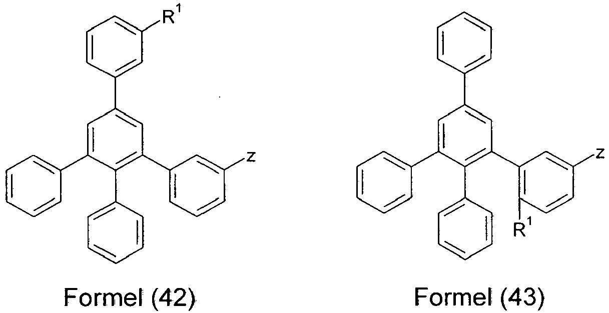

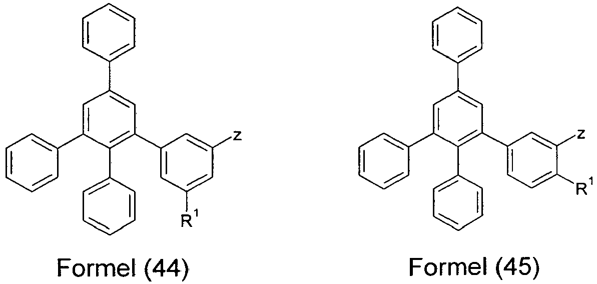

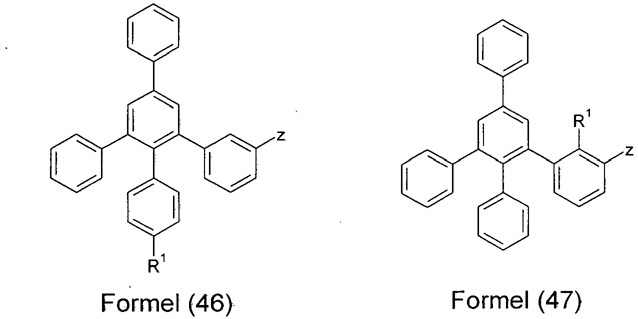

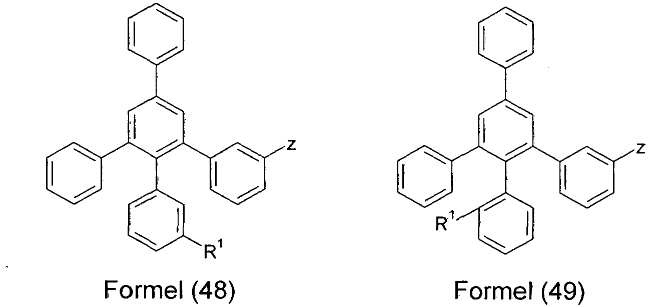

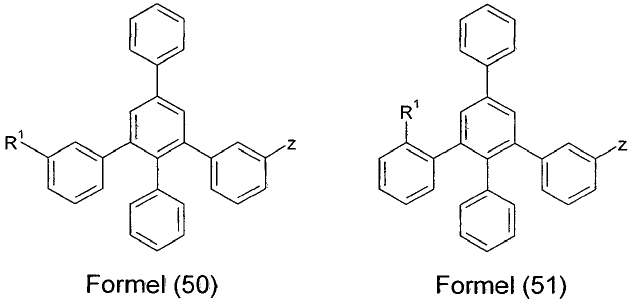

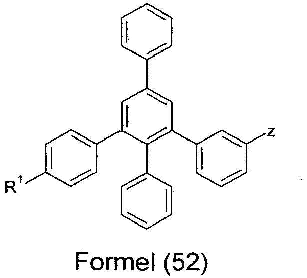

4. Verbindung nach einem oder mehreren der Ansprüche 1 bis 3, **dadurch gekennzeichnet, dass** sie ausgewählt ist aus den Verbindungen der Formeln (63) und (64).

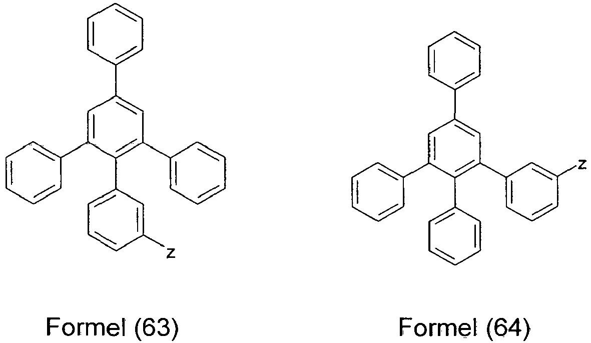

5. Formulierung enthaltend wenigstens eine Verbindung nach einem oder mehreren der Ansprüche 1 bis 4 und wenigstens ein Lösungsmittel.

6. Zusammensetzung enthaltend wenigstens eine der Verbindungen nach einem oder mehreren der Ansprüche 1 bis 4 und wenigstens ein weiteres organisch funktionelles Material ausgewählt aus der Gruppe der Emitter, Host Materialien, Matrix Materialien, Elektronentransportmaterialien (ETM), Elektroneninjektionsmaterialien (EIM), Lochtransportmaterialien (HTM), Lochinjektionsmaterialien (HIM), Elektronenblockiermaterialien (EBM), Lochblockiermaterialien (HBM), Exzitonenblockiermaterialien (ExBM), besonders bevorzugt Emitter und ganz besonders bevorzugt fluoreszierende und/oder phosphoreszierende Emitter.

7. Verwendung einer Verbindung nach einem oder mehreren der Ansprüche 1 bis 4 oder einer Formulierung nach Anspruch 5 oder einer Zusammensetzung nach Anspruch 6 in einer elektronischen Vorrichtung, bevorzugt ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs) oder organischen Laserdioden (O-Laser).

8. Elektronische Vorrichtung, enthaltend mindestens eine Verbindung nach einem oder mehreren der Ansprüche 1 bis 4 oder eine Zusammensetzung nach Anspruch 6, bevorzugt ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs) oder organischen Laserdioden (O-Laser).

9. Organische Elektrolumineszenzvorrichtung nach Anspruch 8, **dadurch gekennzeichnet, dass** die Verbindung nach einem oder mehreren der Ansprüche 1 bis 4 als Host oder Matrix-Material in einer oder mehreren emittierenden Schichten eingesetzt wird, bevorzugt in Kombination mit einem Emitter, wobei der Emitter bevorzugt ausgewählt ist aus der Gruppe der fluoreszierenden und phosphoreszierenden Emitter.

10. Organsche Elektrolumineszenzvorrichtung nach Anspruch 8 oder 9 zur Verwendung in der Medizin zur Phototherapie.

11. Verwendung einer organischen Elektrolumineszenzvorrichtung nach Anspruch 8 oder 9 in der Kosmetik.

## Claims

1. Compound of the formula (27) or (28) 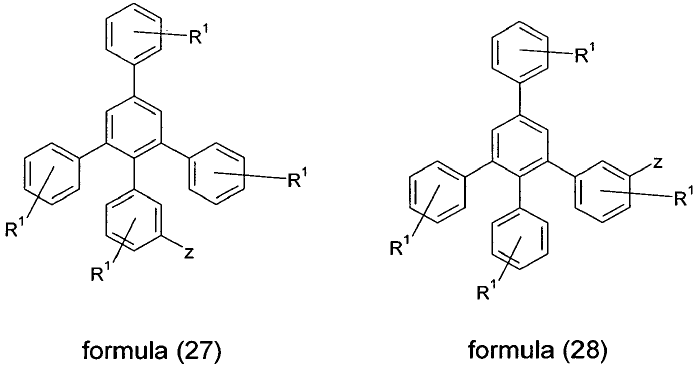 where the following applies to the symbols and indices used:
R¹ is, identically or differently on each occurrence, H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, which may in each case be sub-stituted by one or more radicals R², where one or more non-adjacent CH₂ groups may be replaced by R²C=CR², C≡C, Si(R²)₂, Ge(R²)₂, Sn(R²)₂, C=O, C=S, C=Se, C=NR², P(=O)(R²), SO, SO₂, NR², O, S or CONR² and where one or more H atoms may be replaced by D, F, Cl, Br, I, CN or NO₂, or an aromatic or heteroaromatic ring system having 5 to 60 aromatic ring atoms, which may in each case be substituted by one or more radicals R², or an aryloxy, arylalkoxy or heteroaryloxy group having 5 to 60 aromatic ring atoms, which may be substituted by one or more radicals R², or a diarylamino group, diheteroarylamino group or arylheteroarylamino group having 10 to 40 aromatic ring atoms, which may be substituted by one or more radicals R²;
where the radicals R¹ may occur a maximum of once on the rings;
R² is, identically or differently on each occurrence, H, D, F, Cl, Br, I, N(R³)₂, CN, NO₂, Si(R³)₃, B(OR³)₂, C(=O)R³, P(=O)(R³)₂, S(=O)R³, S(=O)₂R³, OSO₂R³, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, which may in each case be sub-stituted by one or more radicals R³, where one or more non-adjacent CH₂ groups may be replaced by R³C=CR³, C≡C, Si(R³)₂, Ge(R³)₂, Sn(R³)₂, C=O, C=S, C=Se, C=NR³, P(=O)(R³), SO, SO₂, NR³, O, S or CONR³ and where one or more H atoms may be replaced by D, F, Cl, Br, I, CN or NO₂, or an aromatic or heteroaromatic ring system having 5 to 60 aromatic ring atoms, which may in each case be substituted by one or more radicals R³, or an aryloxy, arylalkoxy or heteroaryloxy group having 5 to 60 aromatic ring atoms, which may be substituted by one or more radicals R³, or a diarylamino group, diheteroarylamino group or arylheteroarylamino group having 10 to 40 aromatic ring atoms, which may be substituted by one or more radicals R³, or a combination of two or more of these groups; two or more adjacent radicals R² here may form a mono- or polycyclic, aliphatic or aromatic ring system with one another;
R³ is, identically or differently on each occurrence, H, D, F or an aliphatic, aromatic and/or heteroaromatic hydrocarbon radical having 1 to 20 C atoms, in which, in addition, one or more H atoms may be replaced by F; two or more substituents R³ here may also form a mono- or polycyclic, aliphatic or aromatic ring system with one another;
Z is on each occurrence, identically or differently, a radical of the following formulae (99) to (117) and (121) to (289), where the radicals may themselves be substituted by one or more radicals R³, which may be identical or different on each occurrence and, with the proviso that at least one of the radicals Z must be an aromatic or heteroaromatic group having 5 to 60 aromatic ring atoms

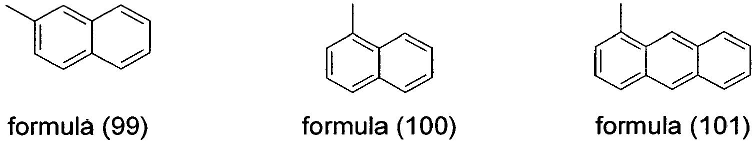

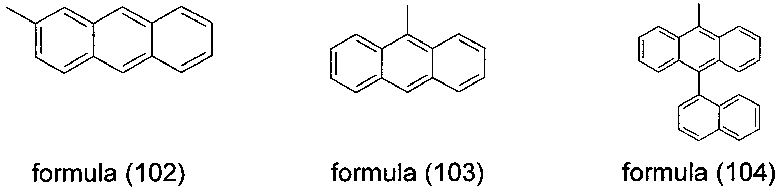

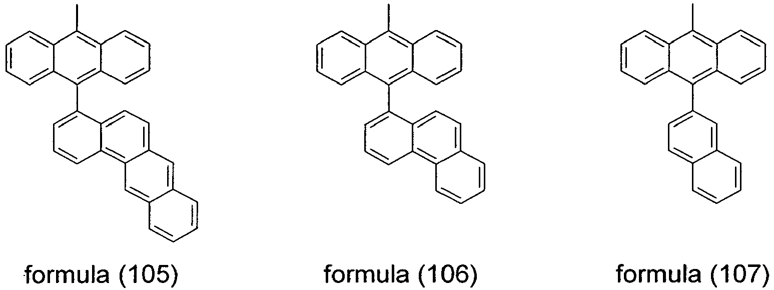

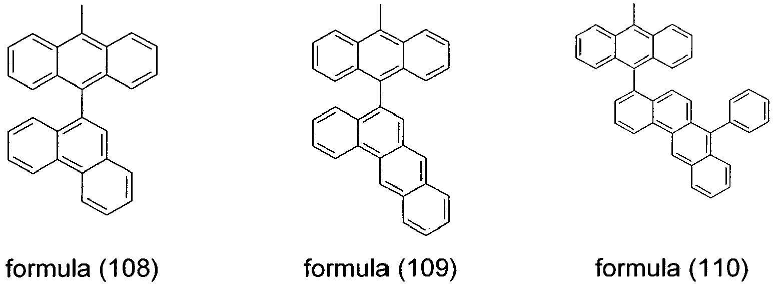

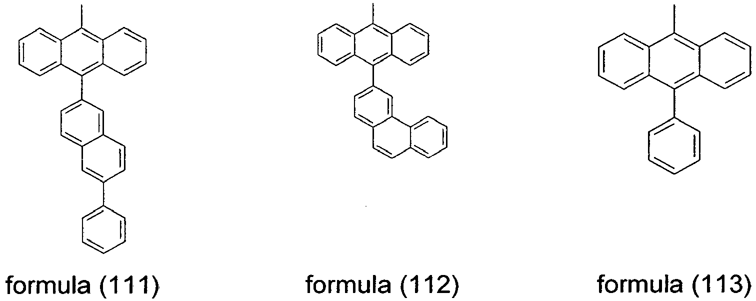

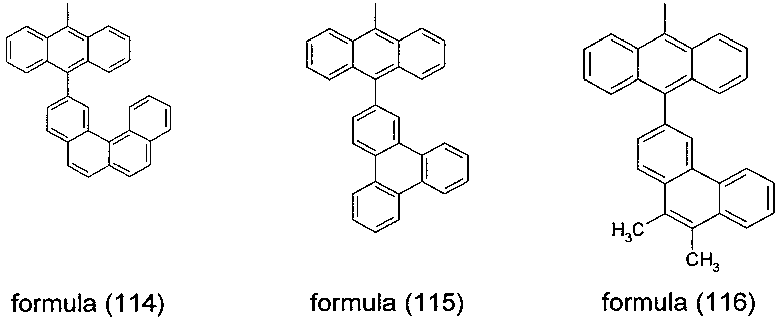

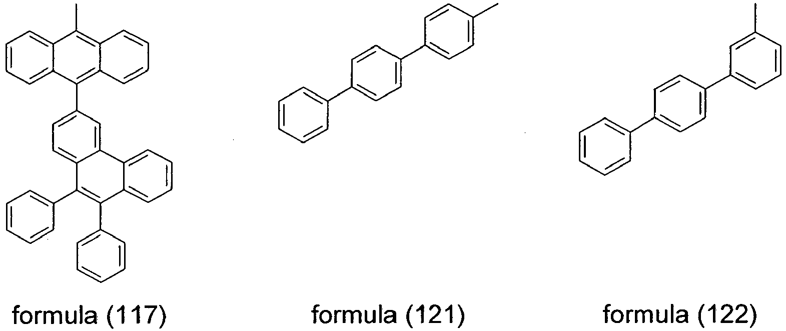

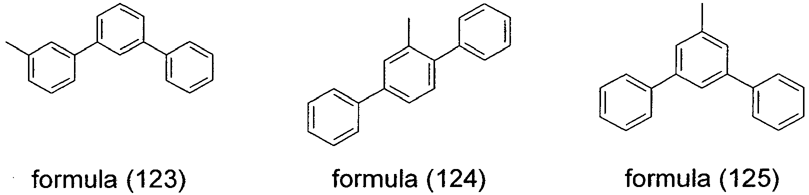

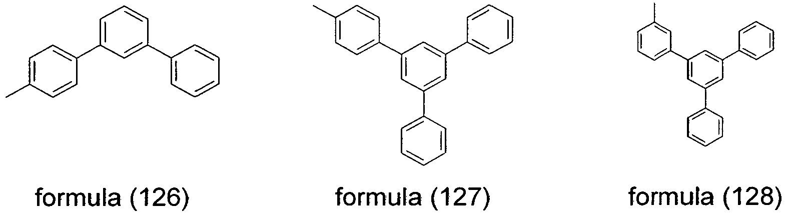

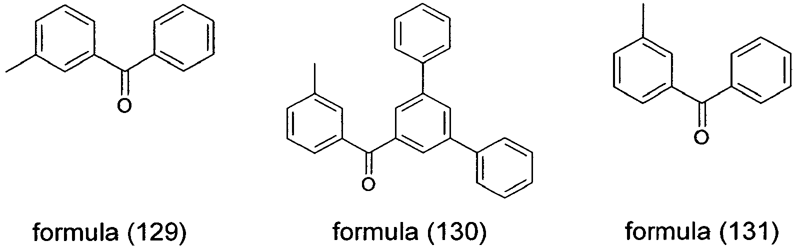

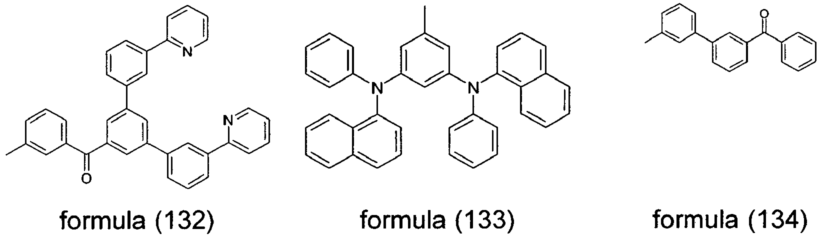

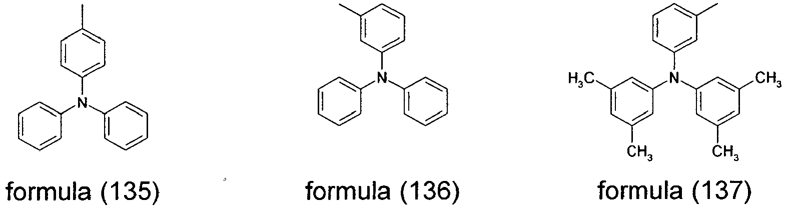

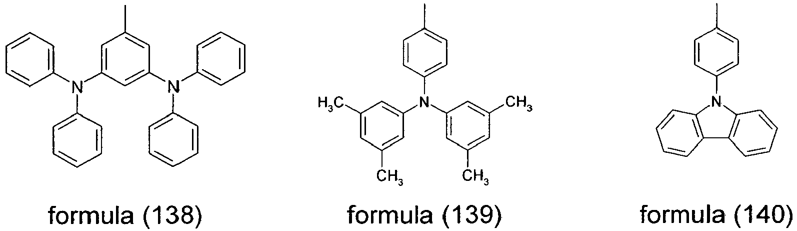

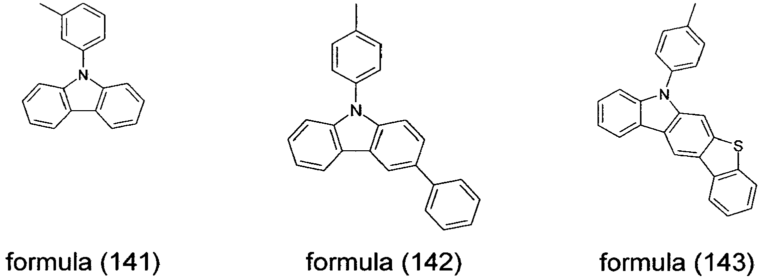

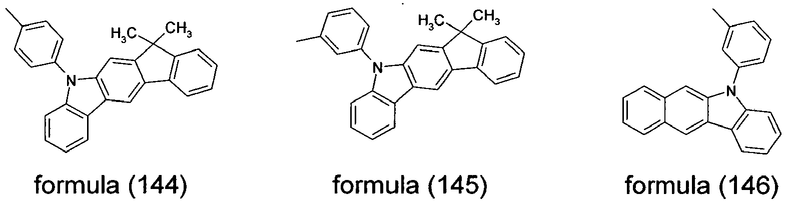

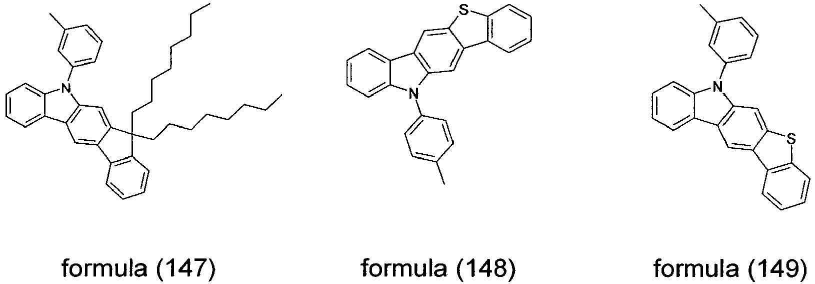

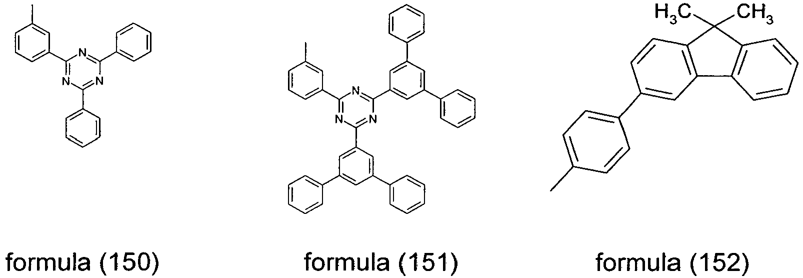

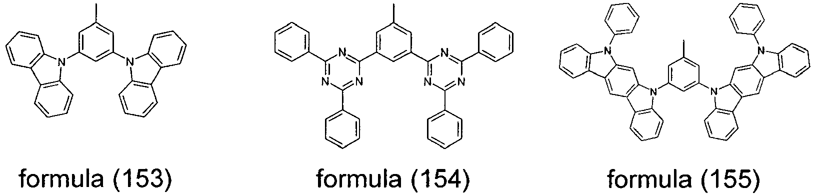

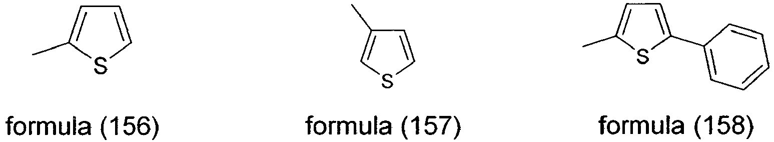

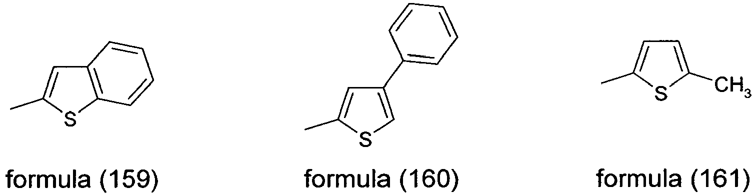

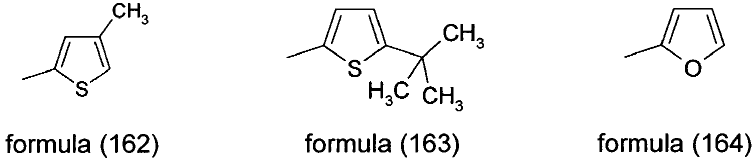

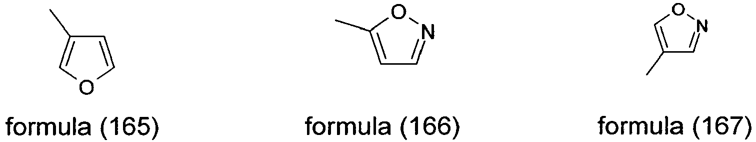

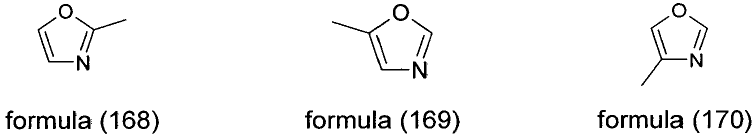

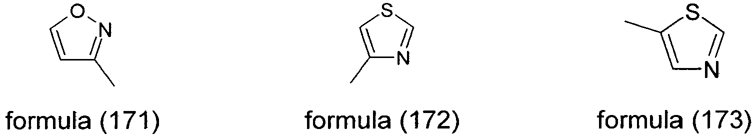

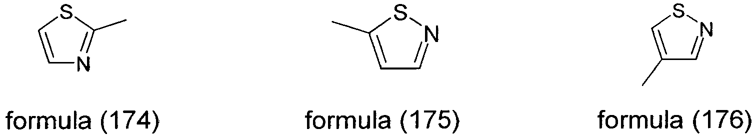

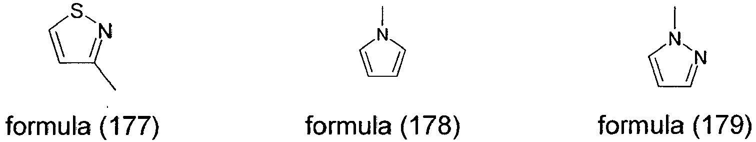

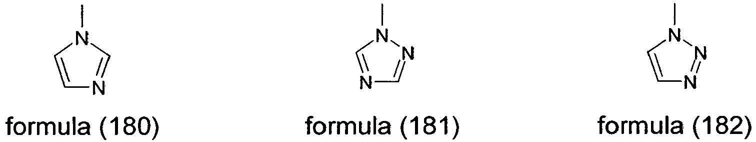

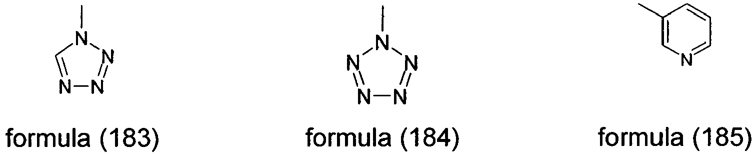

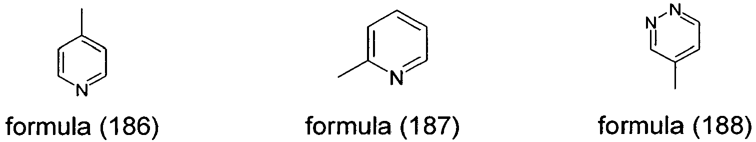

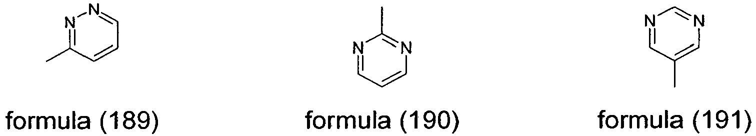

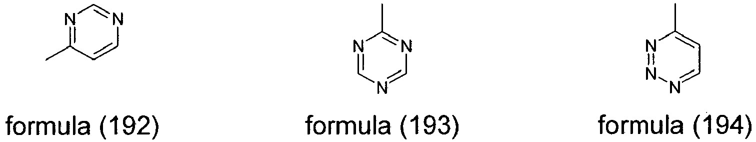

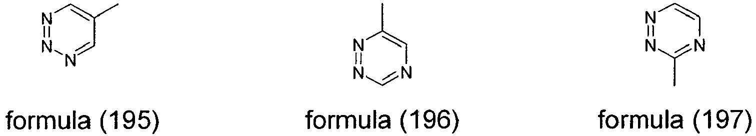

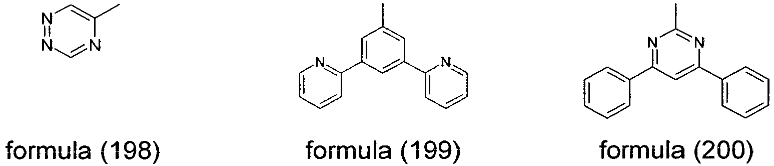

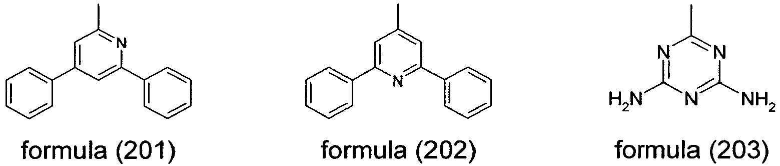

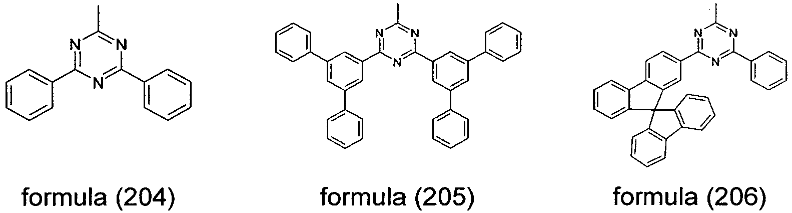

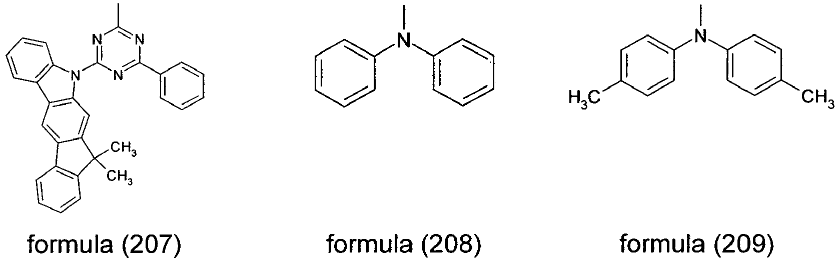

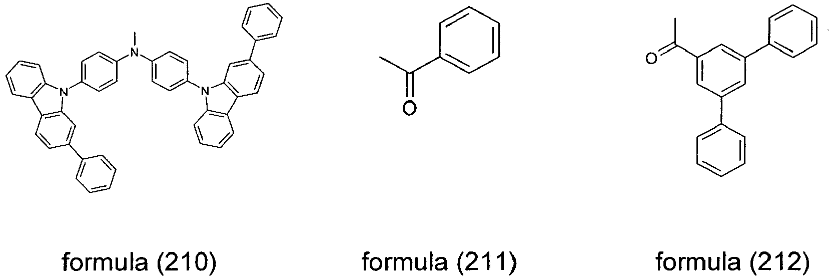

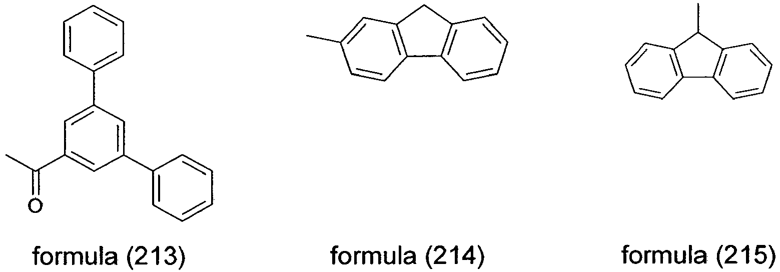

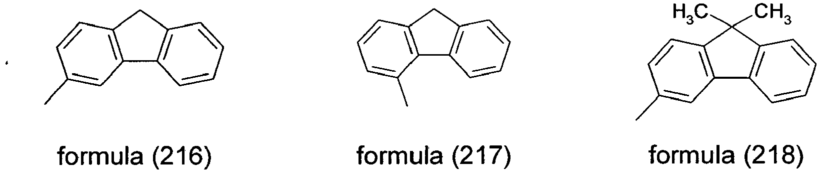

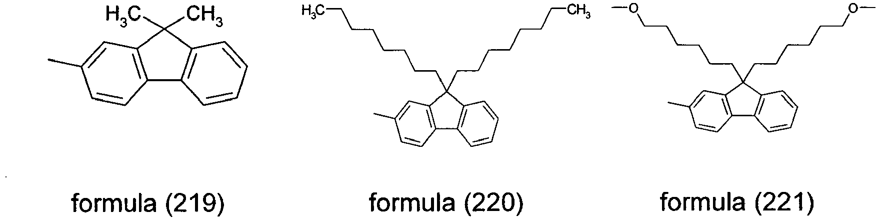

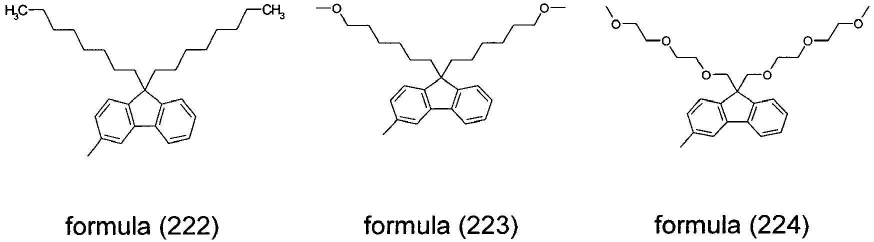

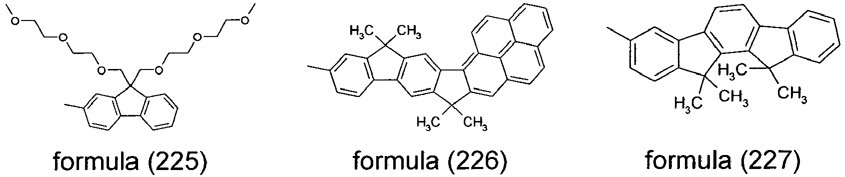

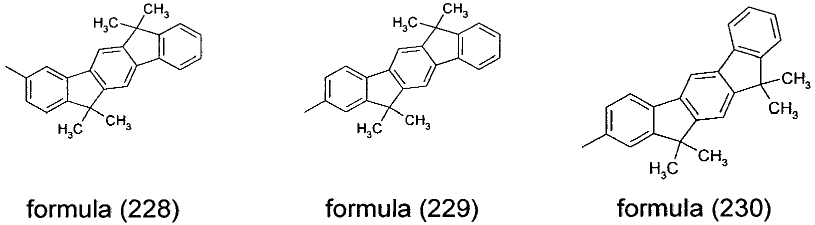

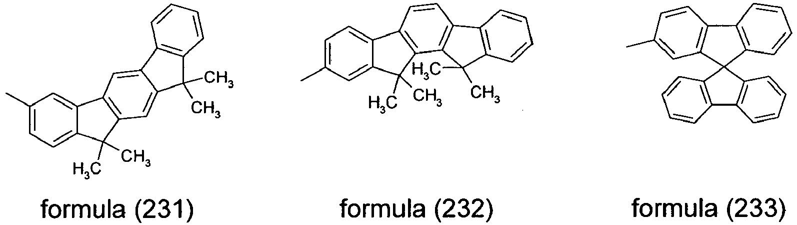

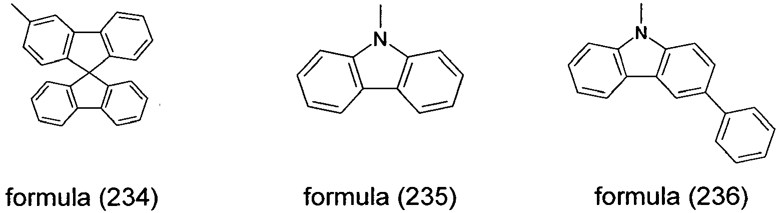

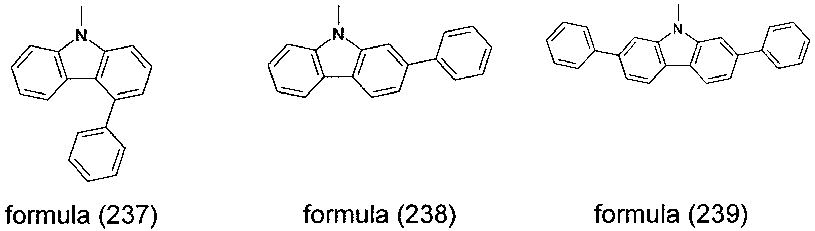

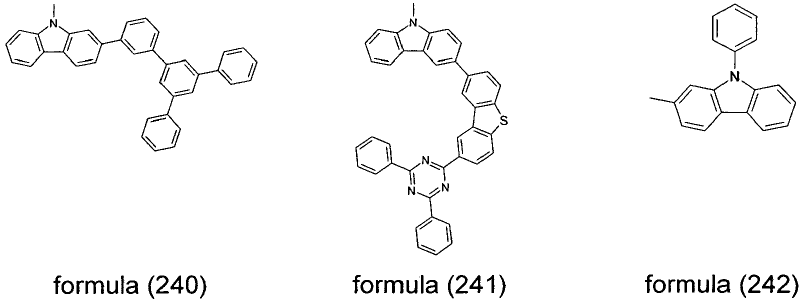

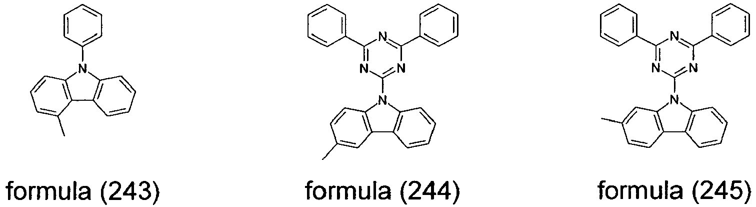

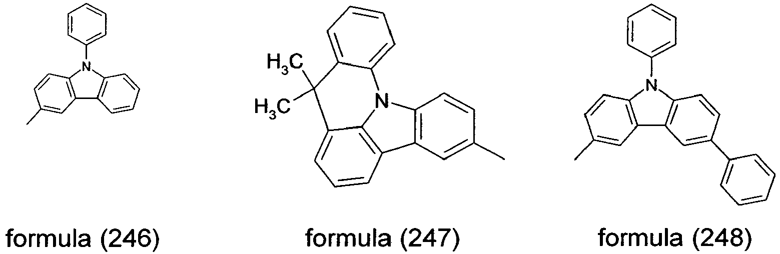

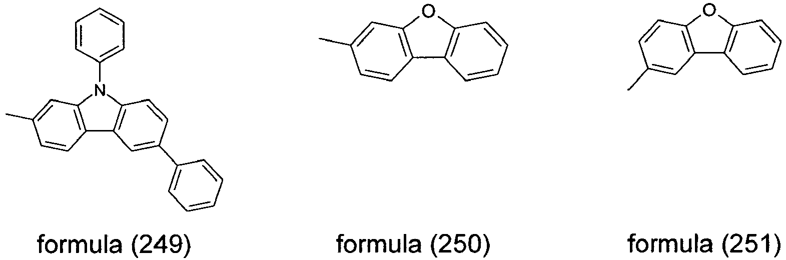

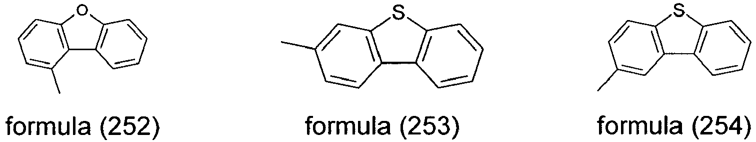

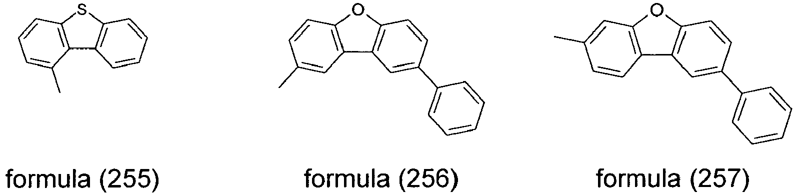

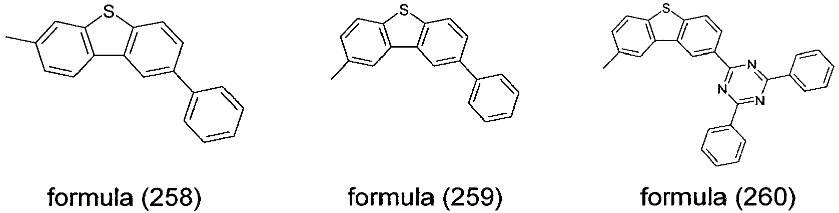

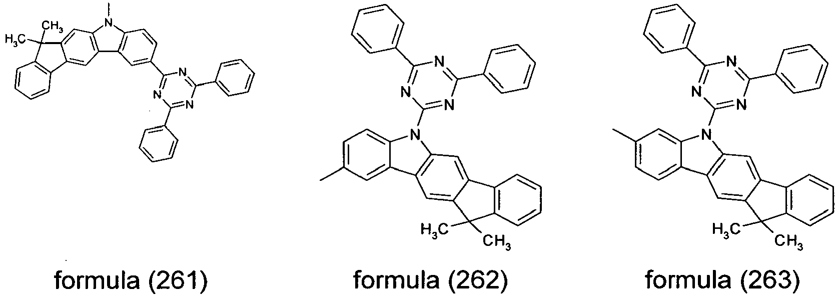

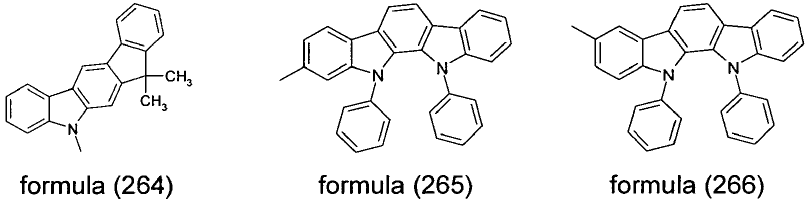

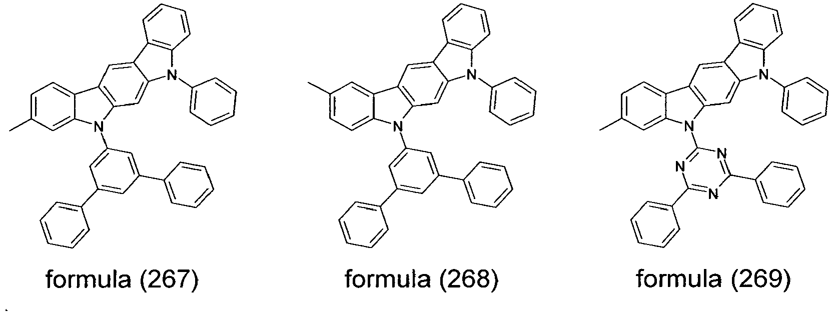

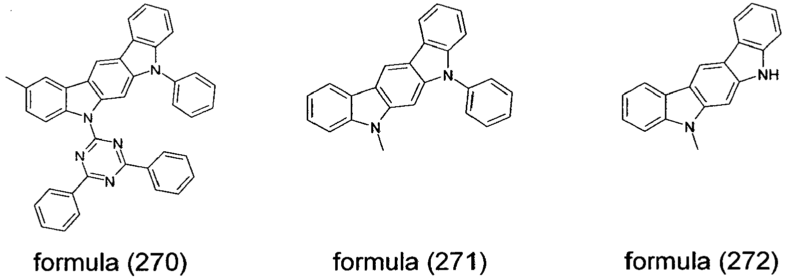

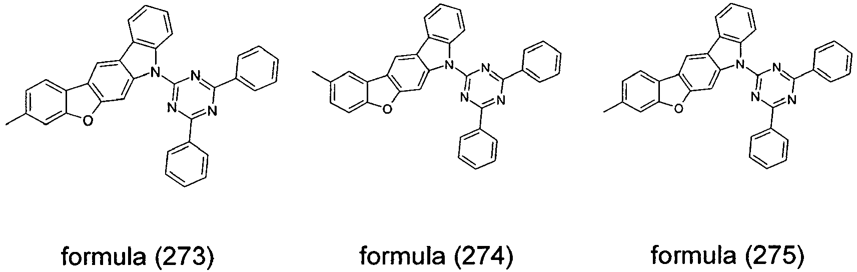

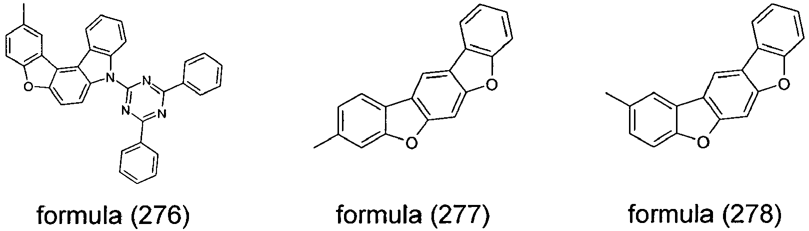

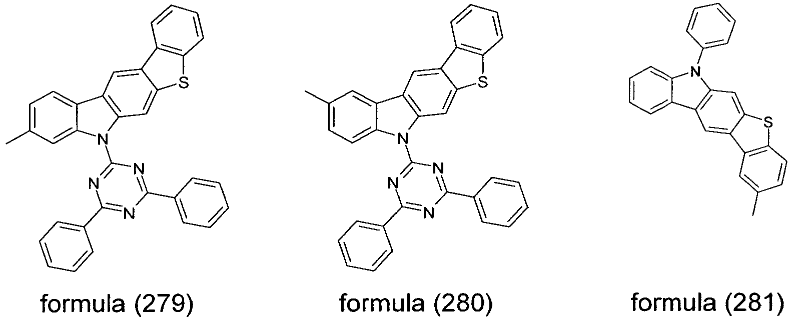

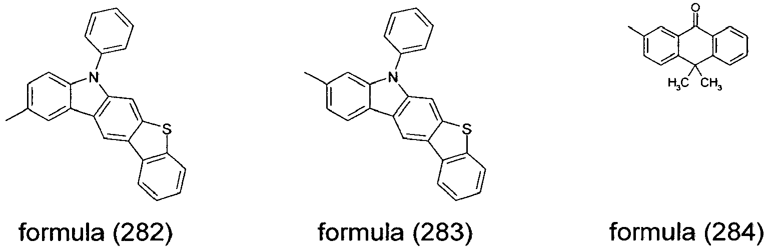

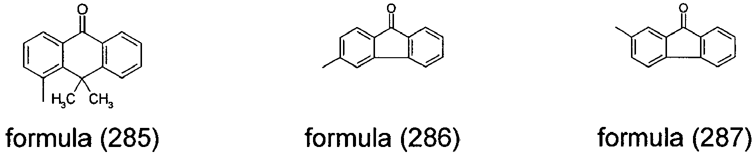

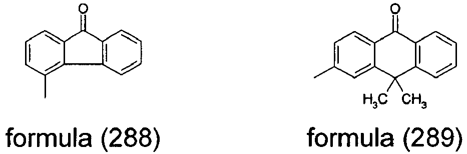

2. Compound according to Claim 1,**characterised in that** it only contains one radical R¹ besides the radical Z.

3. Compound according to Claim 1 or 2, **characterised in that** it is selected from the compounds of the formulae (30) to (52),

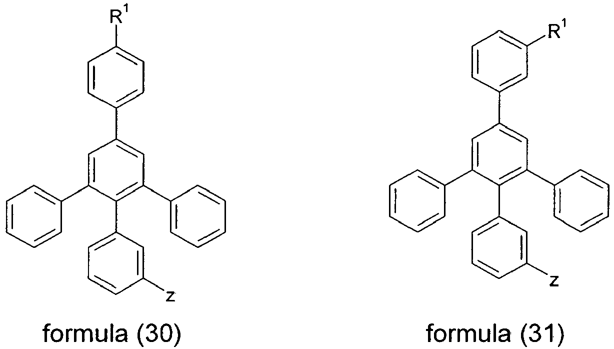

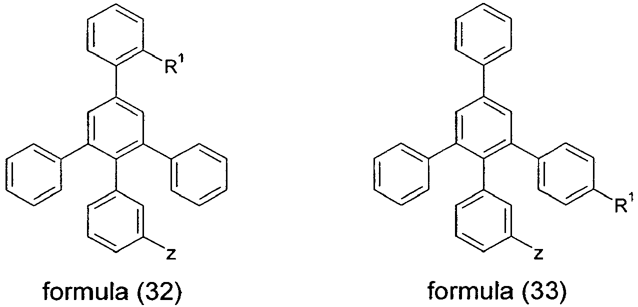

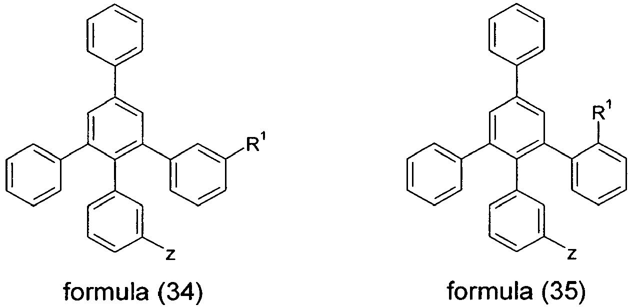

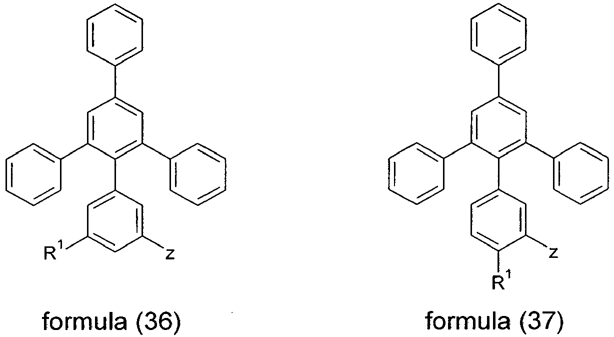

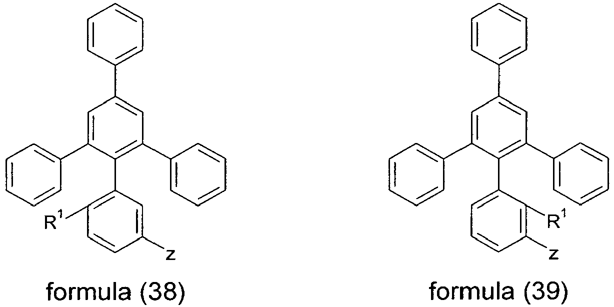

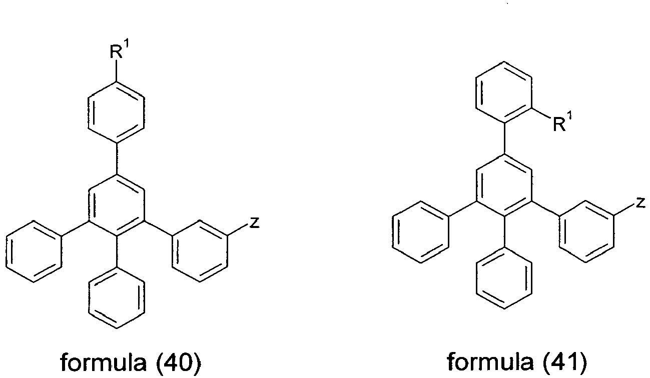

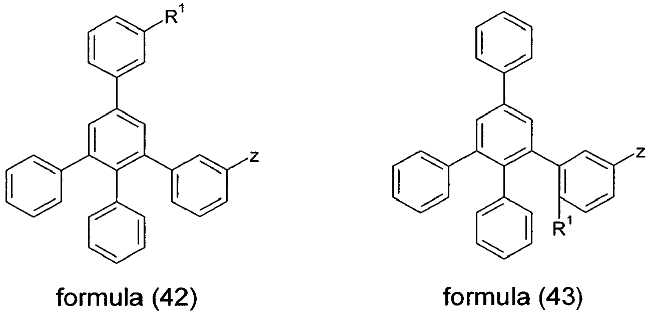

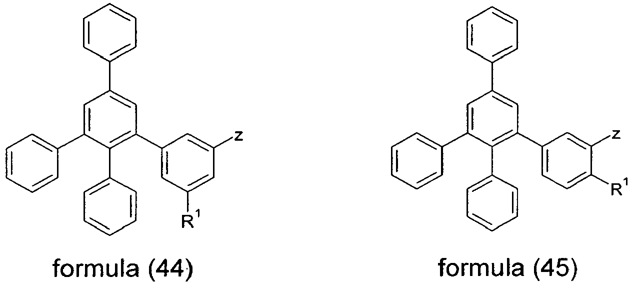

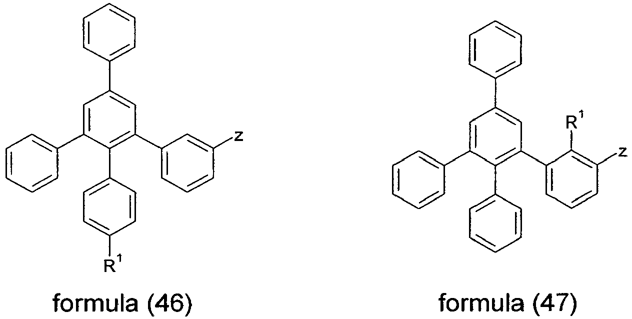

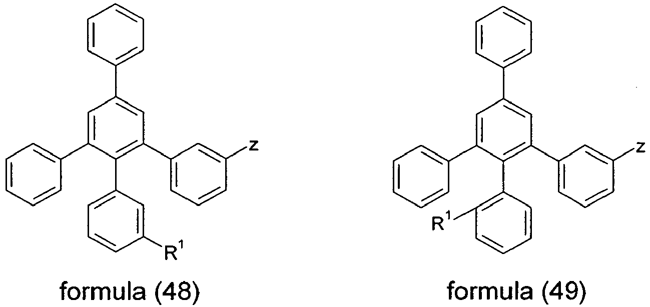

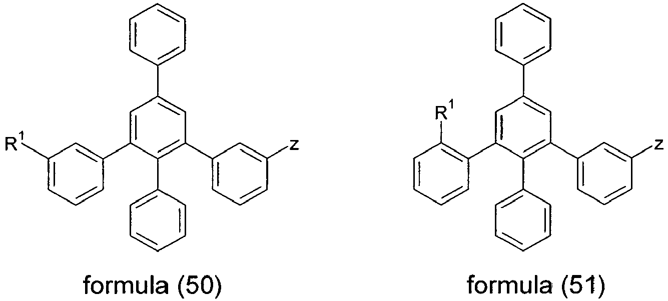

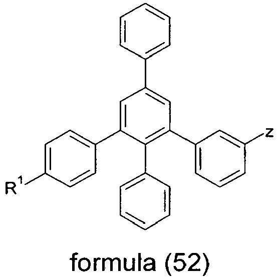

4. Compound according to one or more of Claims 1 to 3, **characterised in that** it is selected from the compounds of the formulae (63) and (64),

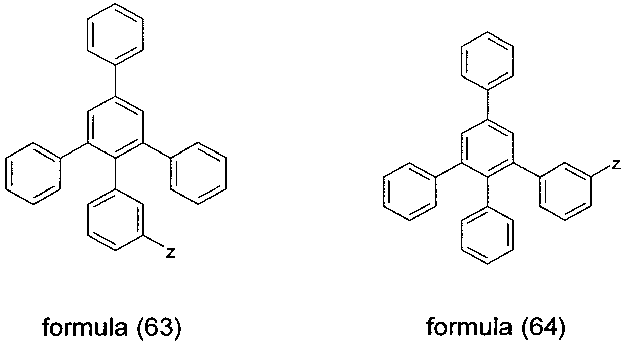

5. Formulation comprising at least one compound according to one or more of Claims 1 to 4 and at least one solvent.

6. Composition comprising at least one of the compounds according to one or more of Claims 1 to 4 and at least one further organically functional material selected from the group of the emitters, host materials, matrix materials, electron-transport materials (ETM), electron-injection materials (EIM), hole-transport materials (HTM), hole-injection materials (HIM), electron-blocking materials (EBM), hole-blocking materials (HBM), exciton-blocking materials (ExBM), particularly preferably emitters and very particularly preferably fluorescent and/or phosphorescent emitters.

7. Use of a compound according to one or more of Claims 1 to 4 or of a formulation according to Claim 5 or of a composition according to Claim 6 in an electronic device, preferably selected from the group consisting of organic electroluminescent devices (OLEDs, PLEDs), organic integrated circuits (O-ICs), organic field-effect transistors (O-FETs), organic thin-film transistors (O-TFTs), organic light-emitting transistors (O-LETs), organic solar cells (O-SCs), organic optical detectors, organic photoreceptors, organic field-quench devices (O-FQDs), light-emitting electrochemical cells (LECs) or organic laser diodes (O-lasers).

8. Electronic device, containing at least one compound according to one or more of Claims 1 to 4 or a composition according to Claim 6, preferably selected from the group consisting of organic electroluminescent devices (OLEDs, PLEDs), organic integrated circuits (O-ICs), organic field-effect transistors (O-FETs), organic thin-film transistors (O-TFTs), organic light-emitting transistors (O-LETs), organic solar cells (O-SCs), organic optical detectors, organic photoreceptors, organic field-quench devices (O-FQDs), light-emitting electrochemical cells (LECs) or organic laser diodes (O-lasers).

9. Organic electroluminescent device according to Claim 8, **characterised in that** the compound according to one or more of Claims 1 to 4 is employed as host or matrix material in one or more emitting layers, preferably in combination with an emitter, where the emitter is preferably selected from the group of the fluorescent and phosphorescent emitters.

10. Organic electroluminescent device according to Claim 8 or 9 for use in medicine for phototherapy.

11. Use of an organic electroluminescent device according to Claim 8 or 9 for use in the cosmetics field.

## Revendications

1. Composé de la formule (27) ou (28) : 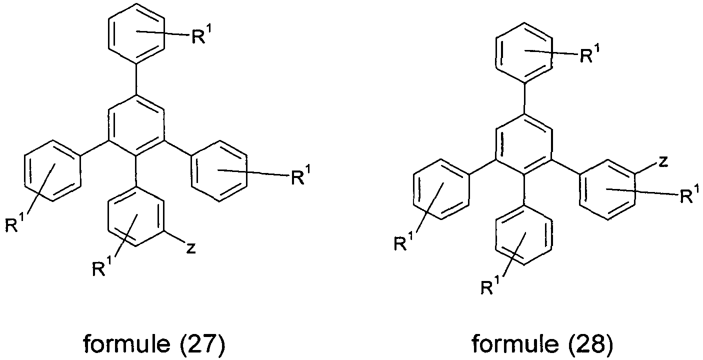 dans lesquelles ce qui suit s'applique aux symboles et indices utilisés :
R¹ est, de manière identique ou différente pour chaque occurrence, H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², un groupe alkyle, alcoxy ou thioalcoxy en chaîne droite qui comporte 1 à 40 atome(s) de C ou un groupe alkényle ou alkynyle en chaîne droite qui comporte 2 à 40 atomes de C ou un groupe alkyle, alkényle, alkynyle, alcoxy, alkylalcoxy ou thioalcoxy ramifié ou cyclique qui comporte 3 à 40 atomes de C, lequel peut, dans chaque cas, être substitué par un radical ou plusieurs radicaux R², où un ou plusieurs groupe(s) CH₂ non adjacents peut/ peuvent être remplacé(s) par R²C=CR², C≡C, Si(R²)₂, Ge(R²)₂, Sn(R²)₂, C=O, C=S, C=Se, C=NR², P(=O)(R²), SO, SO₂, NR², O, S ou CONR² et où un ou plusieurs atome(s) de H peut/ peuvent être remplacé(s) par D, F, Cl, Br, I, CN ou NO₂, ou un système de cycle aromatique ou hétéroaromatique qui comporte 5 à 60 atomes de cycle aromatique, lequel peut, dans chaque cas, être substitué par un radical ou plusieurs radicaux R², ou un groupe aryloxy, arylalcoxy ou hétéroaryloxy qui comporte 5 à 60 atomes de cycle aromatique, lequel peut être substitué par un radical ou plusieurs radicaux R², ou un groupe diarylamino, un groupe dihétéroarylamino ou un groupe aryl-hétéroarylamino qui comporte 10 à 40 atomes de cycle aromatique, lequel peut être substitué par un radical ou plusieurs radicaux R² ;
où les radicaux R¹ peuvent présenter une occurrence maximum d'une fois sur les cycles ;
R² est, de manière identique ou différente pour chaque occurrence, H, D, F, Cl, Br, I, N(R³)₂, CN, NO₂, Si(R³)₃, B(OR³)₂, C(=O)R³, P(=O)(R³)₂, S(=O)R³, S(=O)₂R³, OSO₂R³, un groupe alkyle, alcoxy ou thioalcoxy en chaîne droite qui comporte 1 à 40 atome(s) de C ou un groupe alkényle ou alkynyle en chaîne droite qui comporte 2 à 40 atomes de C ou un groupe alkyle, alkényle, alkynyle, alcoxy, alkylalcoxy ou thioalcoxy ramifié ou cyclique qui comporte 3 à 40 atomes de C, lequel peut, dans chaque cas, être substitué par un radical ou plusieurs radicaux R³, où un ou plusieurs groupe(s) CH₂ non adjacents peut/ peuvent être remplacé(s) par R³C=CR³, C≡C, Si(R³)₂, Ge(R³)₂, Sn(R³)₂, C=O, C=S, C=Se, C=NR³, P(=O)(R³), SO, SO₂, NR³, O, S ou CONR³ et où un ou plusieurs atome(s) de H peut/ peuvent être remplacé(s) par D, F, Cl, Br, I, CN ou NO₂, ou un système de cycle aromatique ou hétéroaromatique qui comporte 5 à 60 atomes de cycle aromatique, lequel peut, dans chaque cas, être substitué par un radical ou plusieurs radicaux R³, ou un groupe aryloxy, arylalcoxy ou hétéroaryloxy qui comporte 5 à 60 atomes de cycle aromatique, lequel peut être substitué par un radical ou plusieurs radicaux R³, ou un groupe diarylamino, un groupe dihétéroarylamino ou un groupe aryl-hétéroarylamino qui comporte 10 à 40 atomes de cycle aromatique, lequel peut être substitué par un radical ou plusieurs radicaux R³, ou une combinaison de deux de ces groupes ou plus; deux radicaux R² adjacents ou plus peuvent ici former un système de cycle aliphatique ou aromatique monocyclique ou polycyclique l'un avec l'autre ou les uns avec les autres ;
R³ est, de manière identique ou différente pour chaque occurrence, H, D, F ou un radical hydrocarbone aromatique et/ou hétéro-aromatique aliphatique qui comporte 1 à 20 atome(s) de C, où, en outre, un ou plusieurs atome(s) de H peut/peuvent être remplacé(s) par F ; deux substituants R³ ou plus peuvent ici également former un système de cycle aliphatique ou aromatique monocyclique ou polycyclique l'un avec l'autre ou les uns avec les autres ;
Z est pour chaque occurrence, de manière identique ou différente, un radical des formules qui suivent (99) à (117) et (121) à (289), où les radicaux peuvent eux-mêmes être substitués par un radical ou plusieurs radicaux R³, lesquels peuvent être identiques ou différents pour chaque occurrence, et étant entendu qu'au moins l'un des radicaux Z doit être un groupe aromatique ou hétéroaromatique qui comporte 5 à 60 atomes de cycle aromatique :

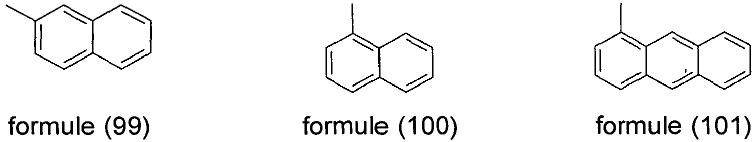

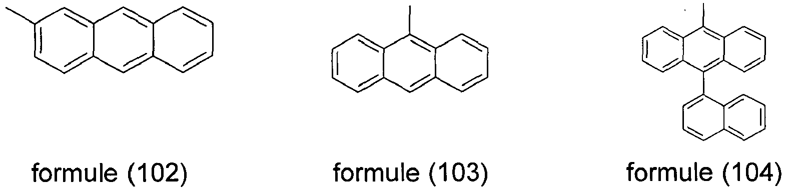

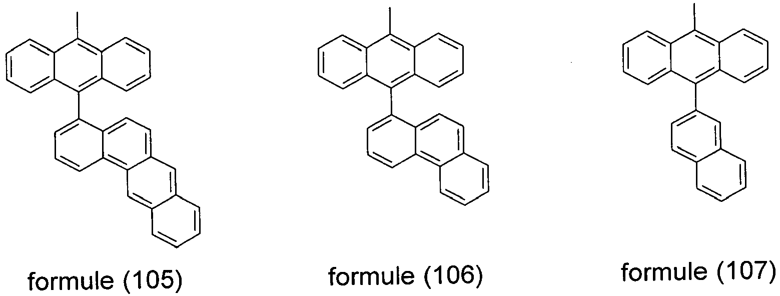

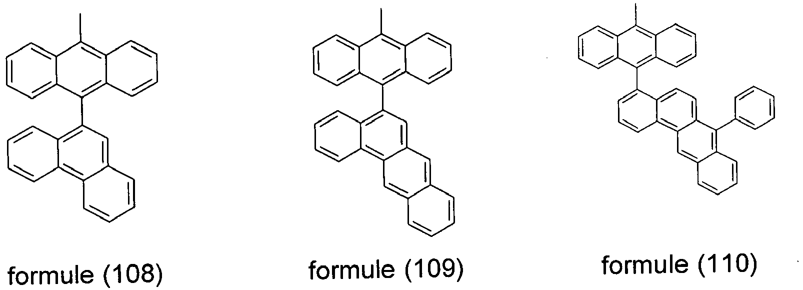

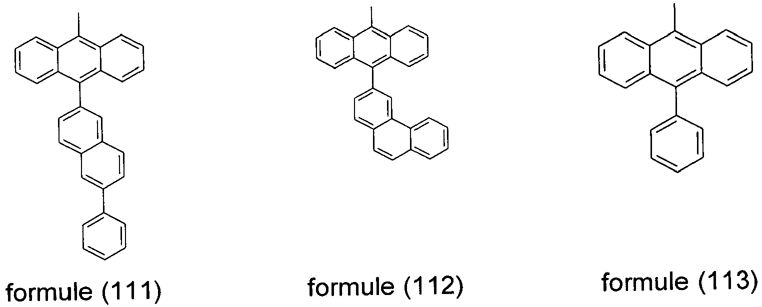

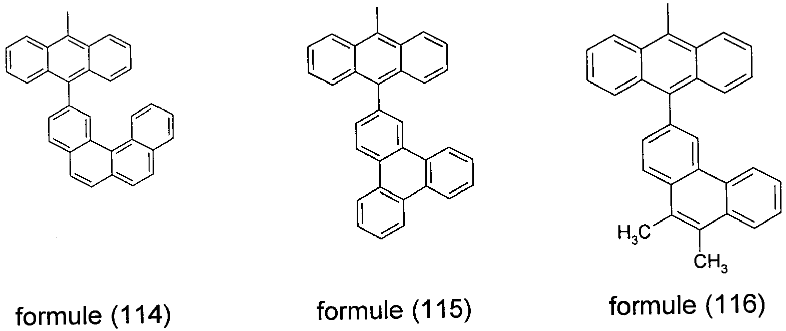

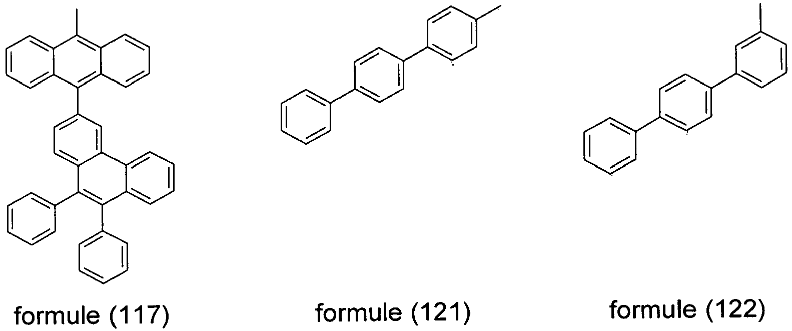

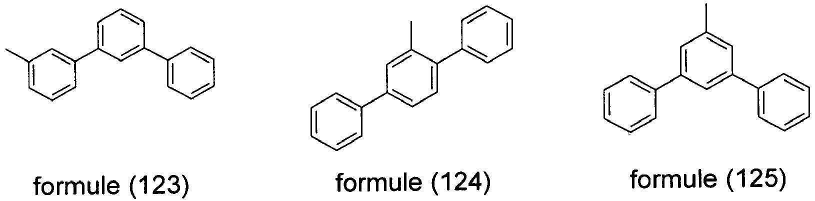

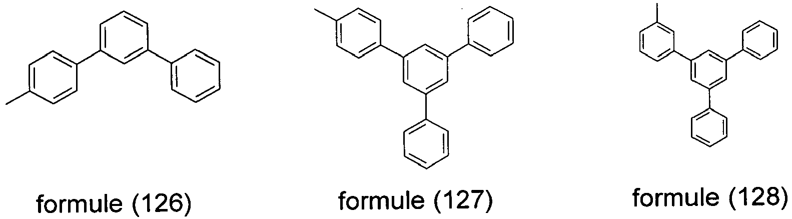

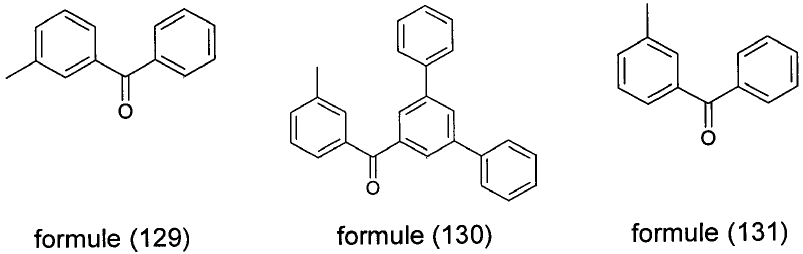

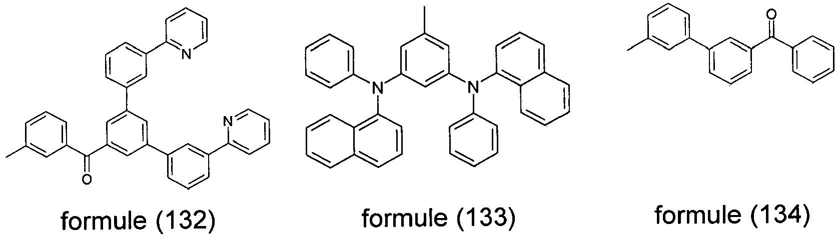

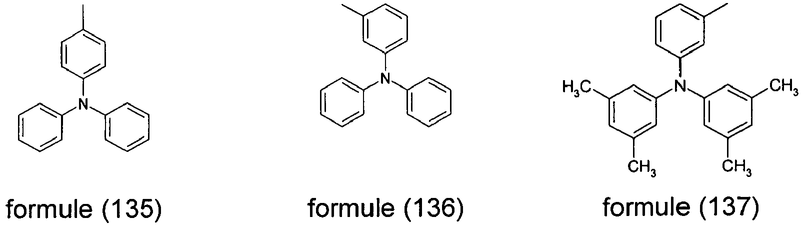

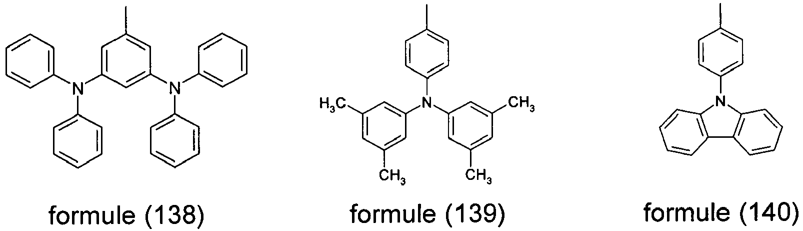

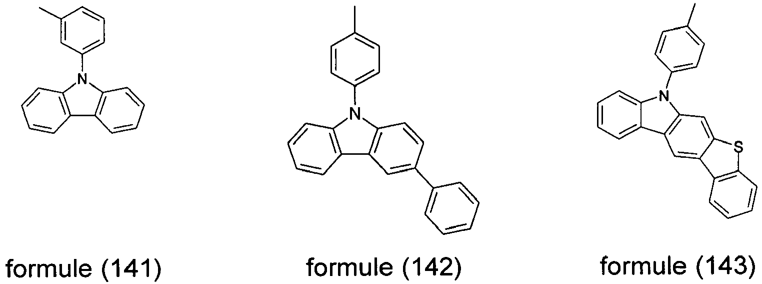

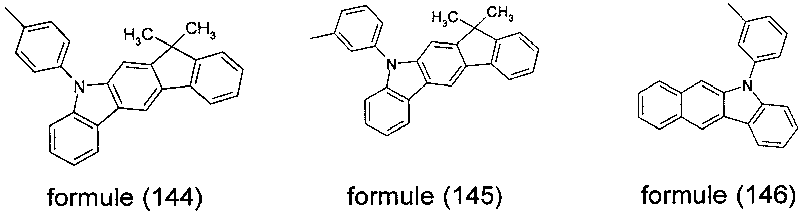

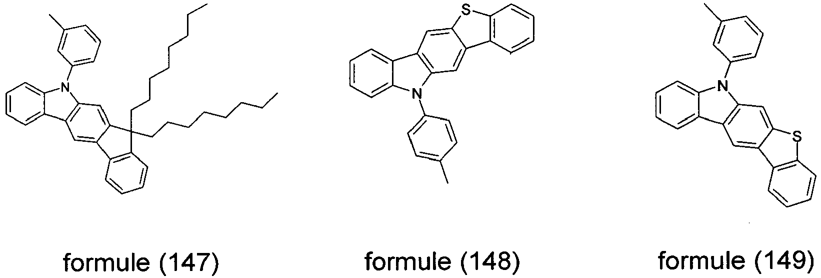

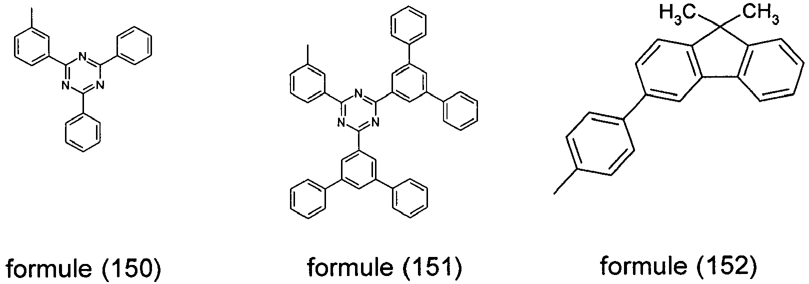

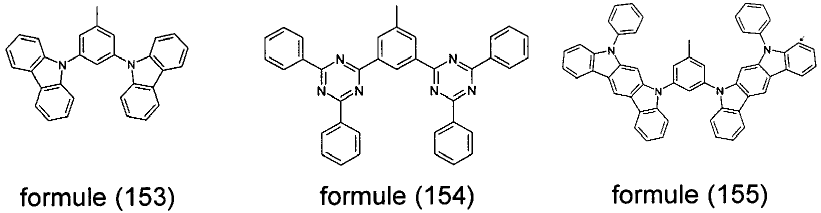

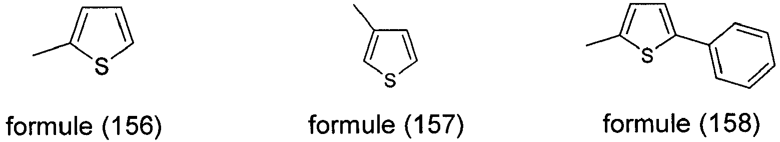

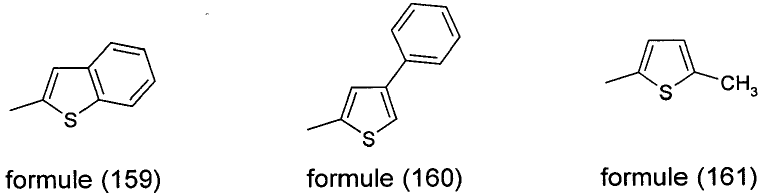

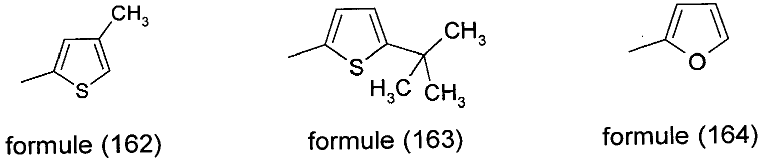

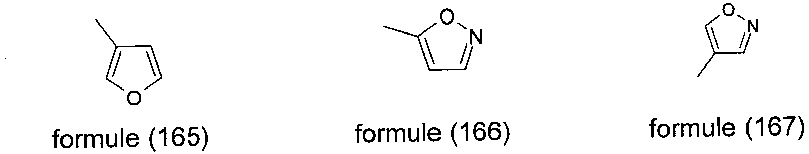

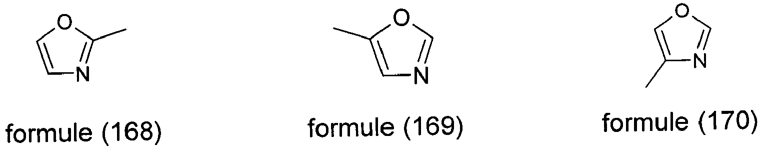

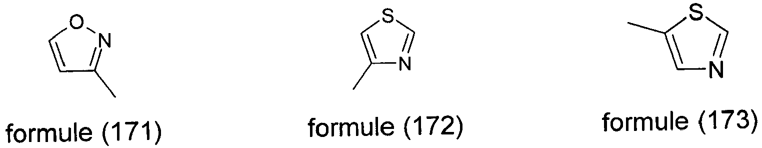

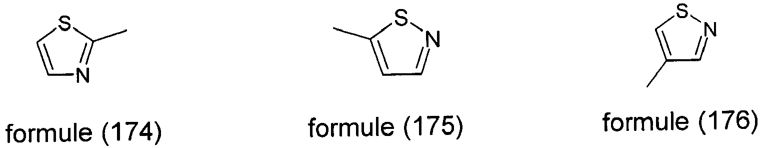

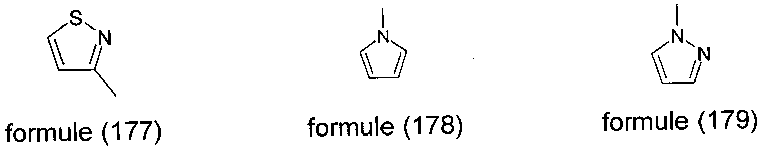

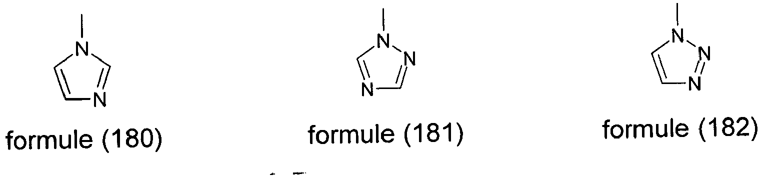

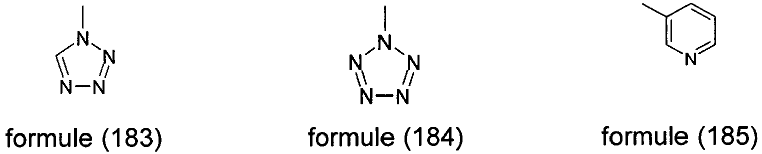

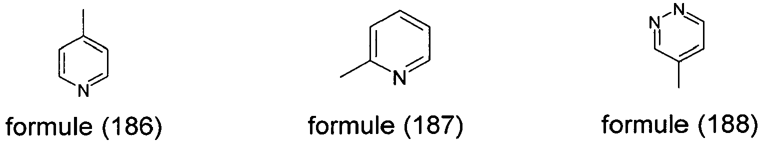

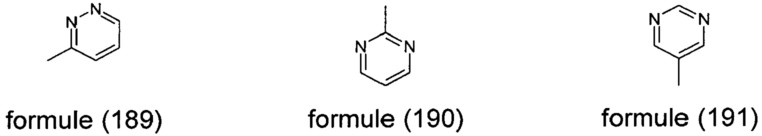

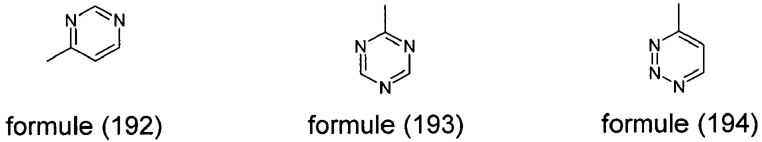

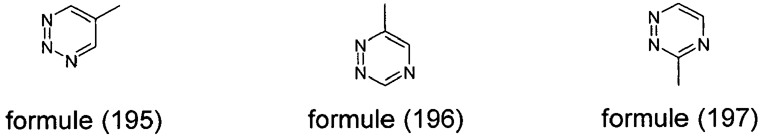

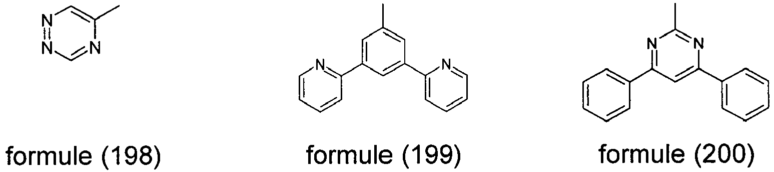

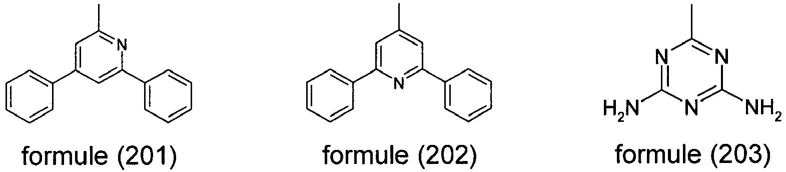

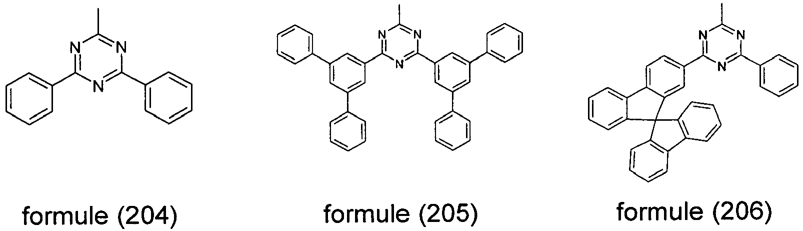

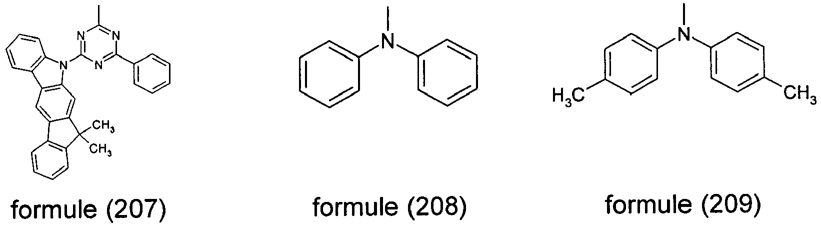

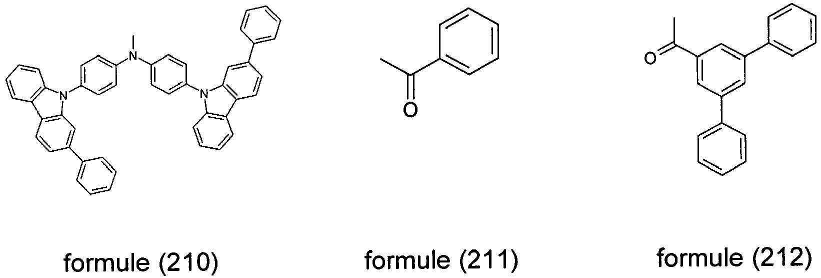

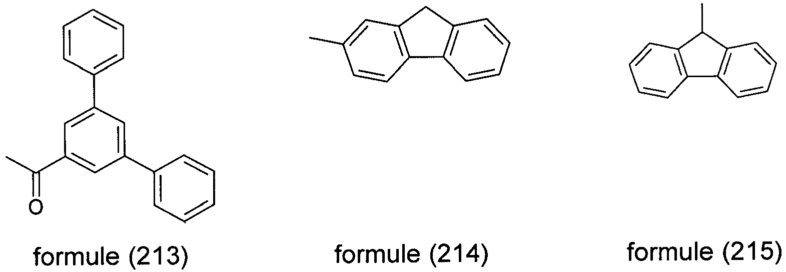

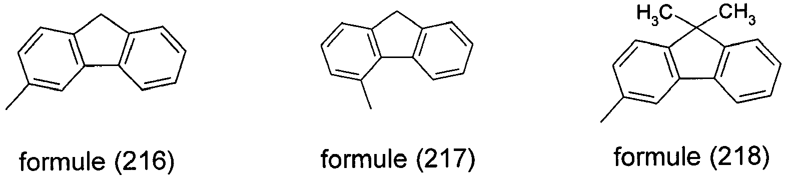

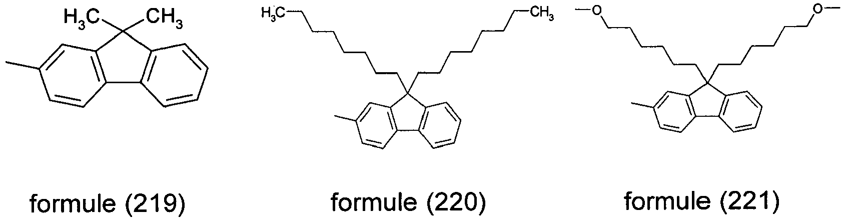

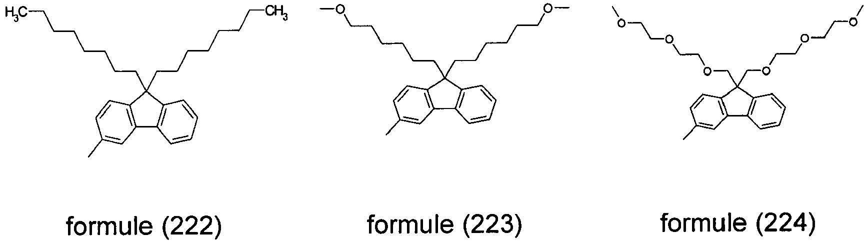

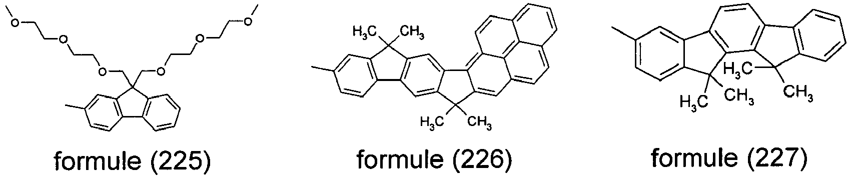

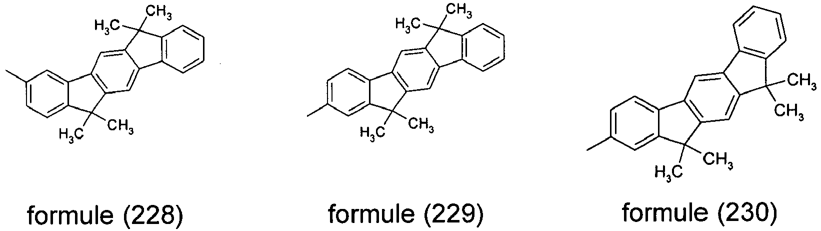

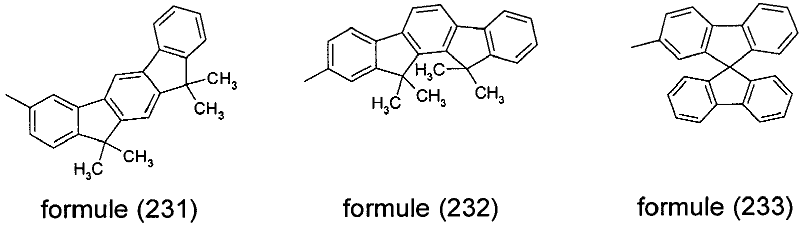

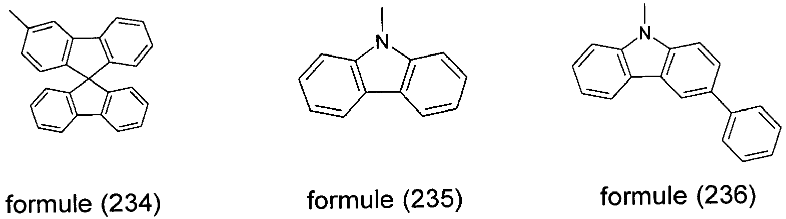

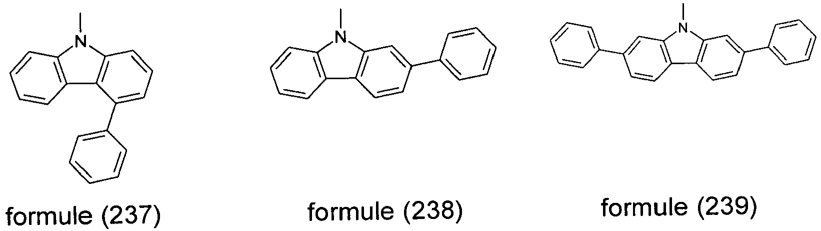

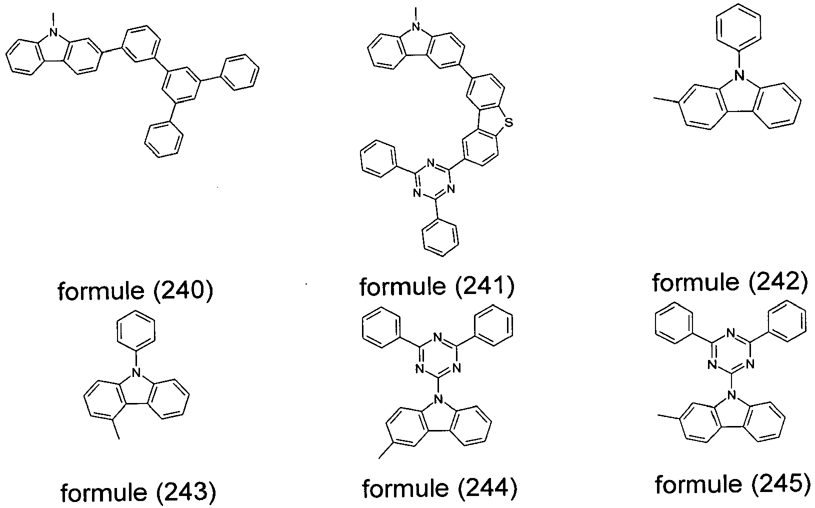

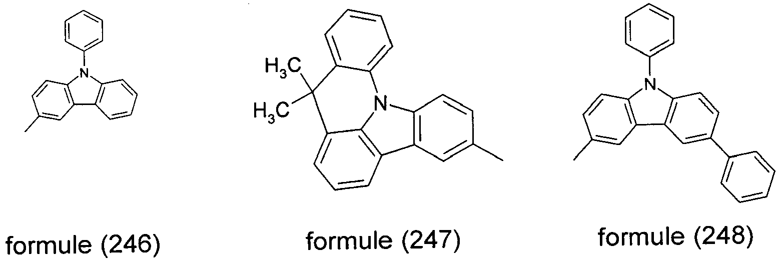

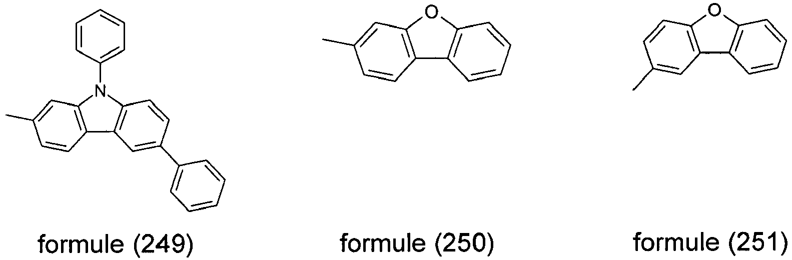

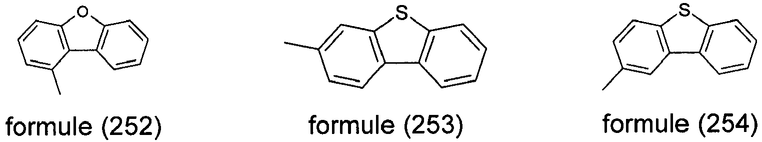

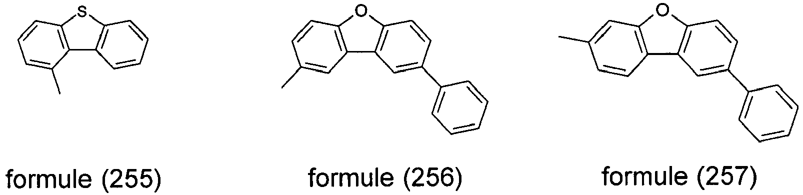

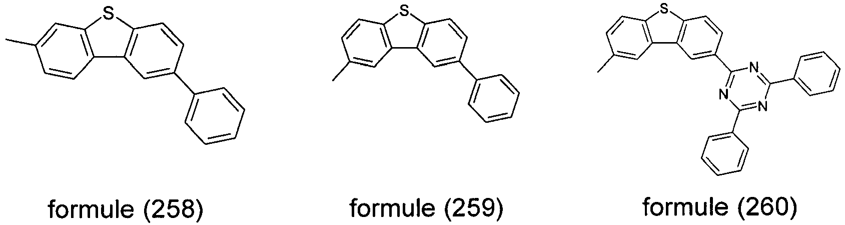

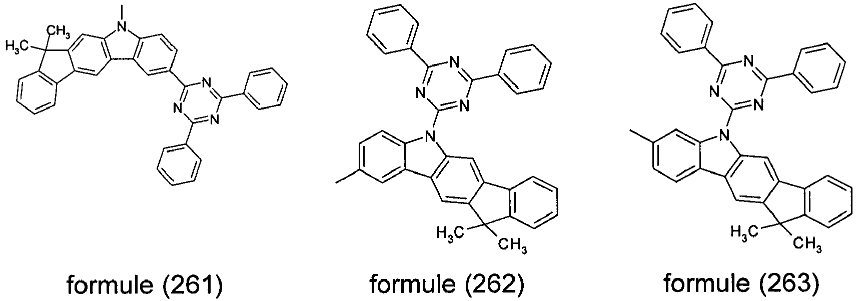

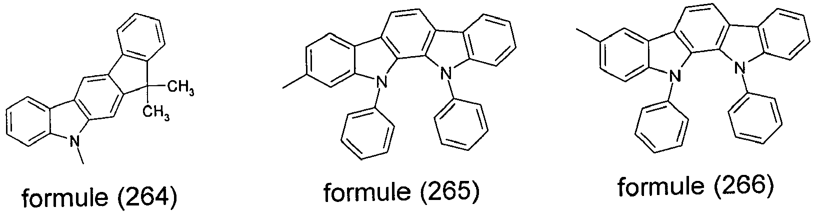

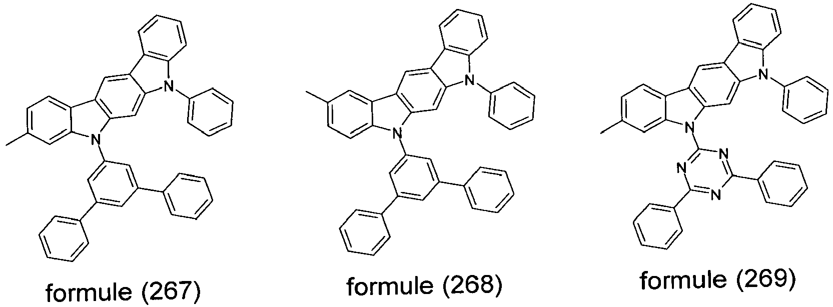

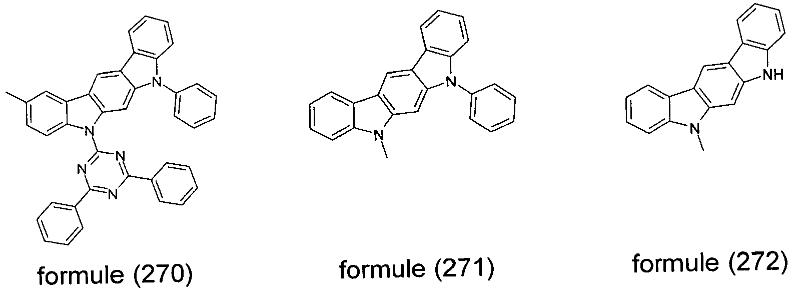

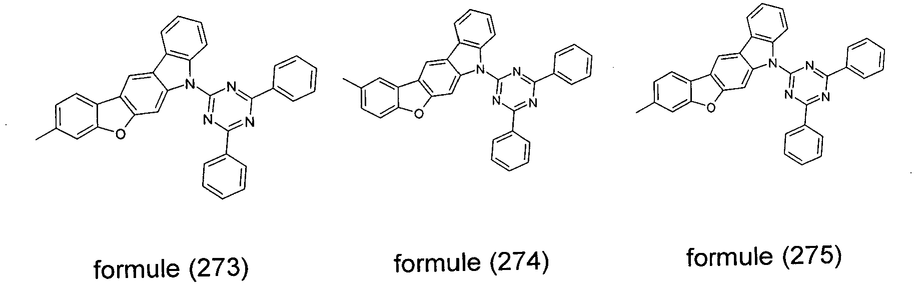

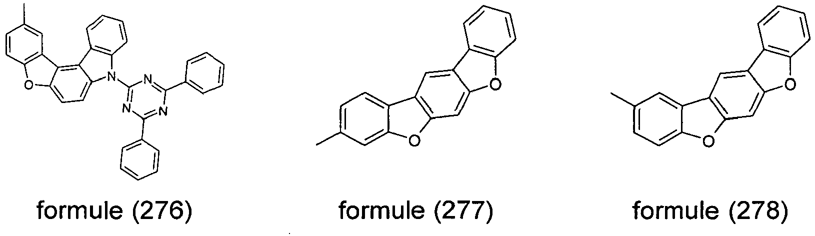

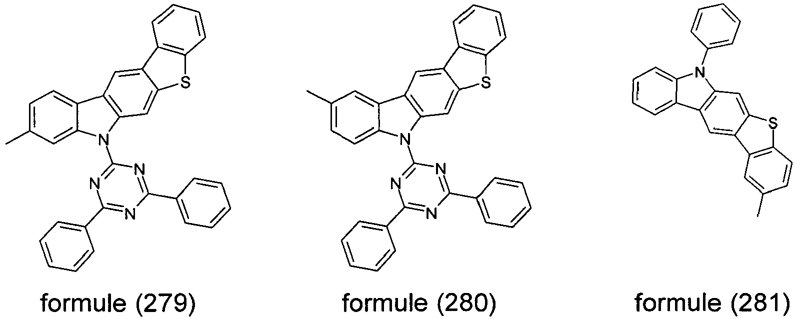

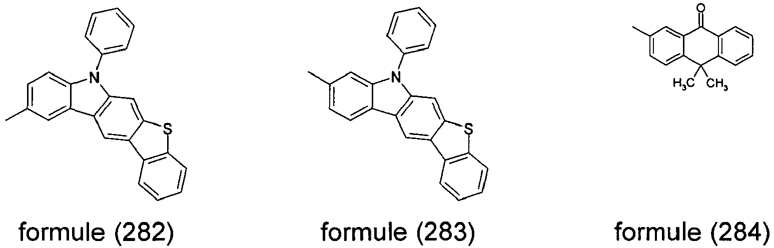

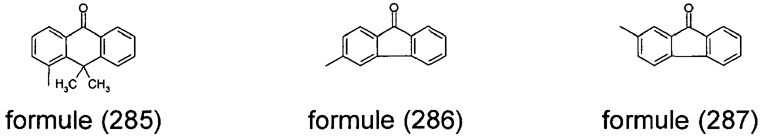

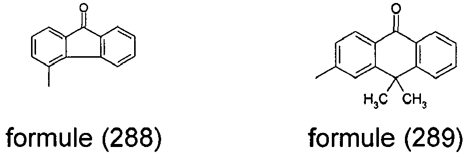

2. Composé selon la revendication 1, **caractérisé en ce qu'**il contient seulement un radical R¹ en plus du radical Z.

3. Composé selon la revendication 1 ou 2, **caractérisé en ce qu'**il est choisi parmi les composés des formules (30) à (52) :

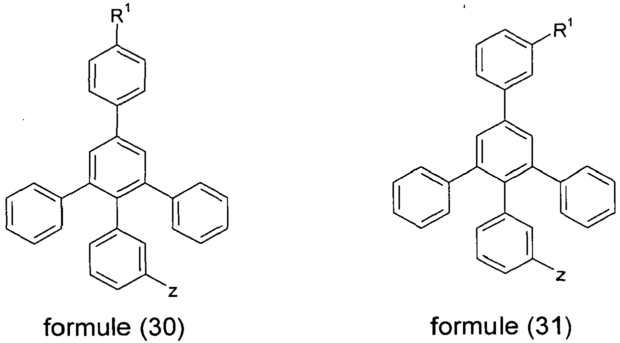

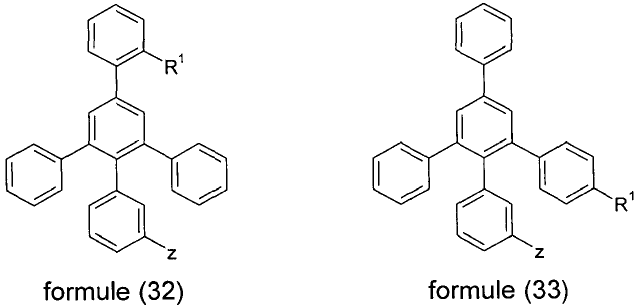

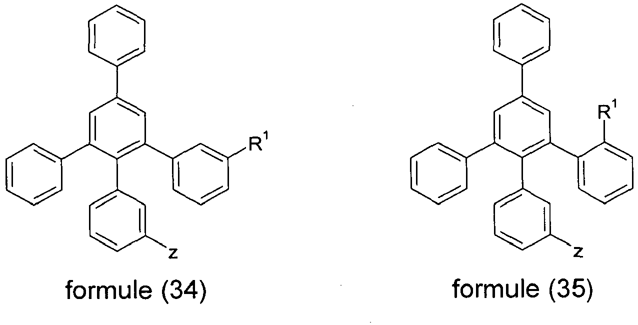

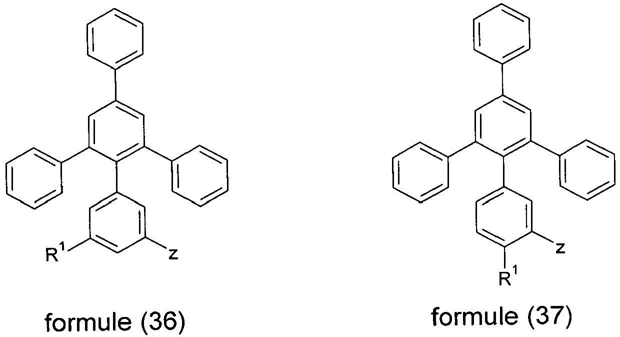

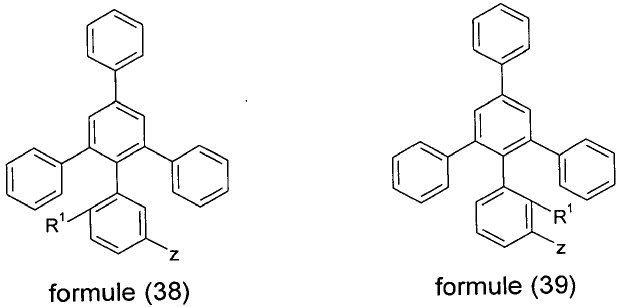

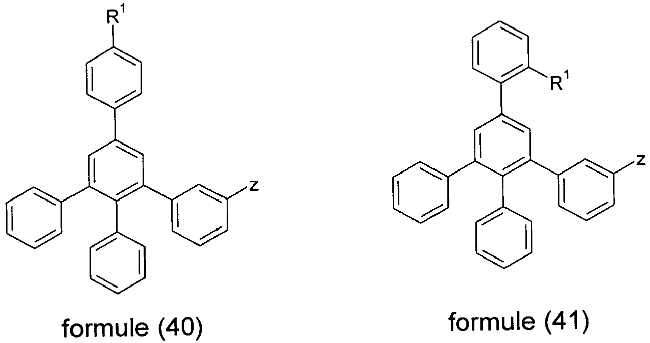

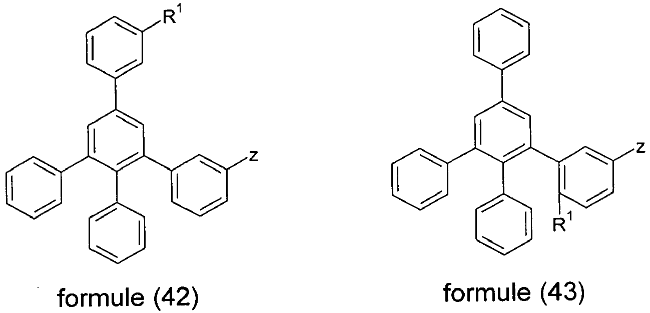

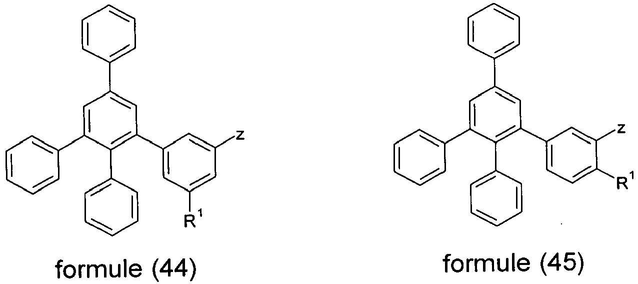

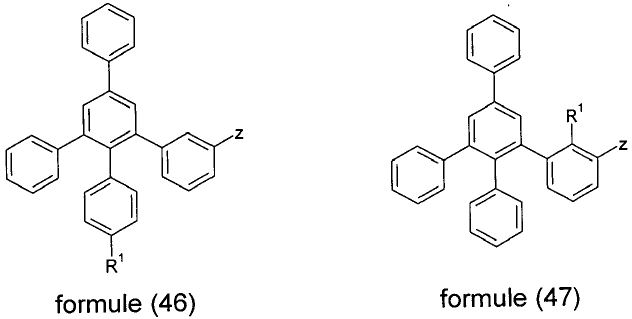

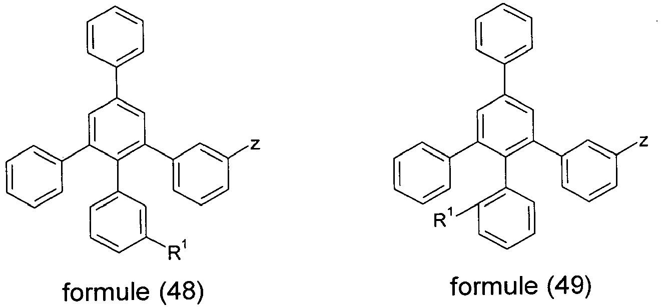

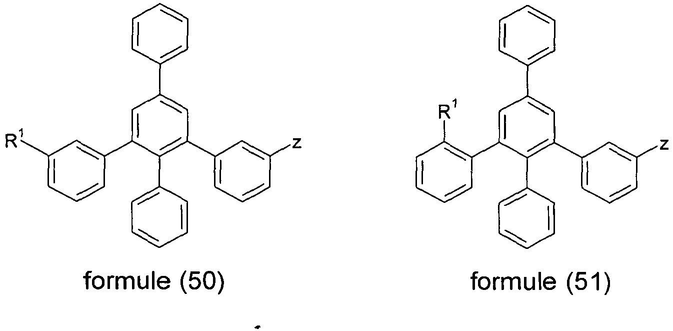

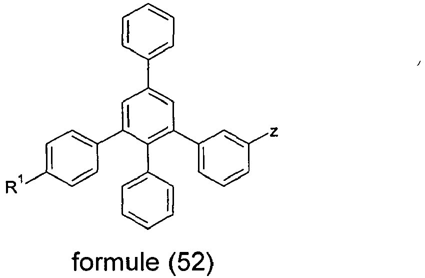

4. Composé selon une ou plusieurs des revendications 1 à 3, **caractérisé en ce qu'**il est choisi parmi les composés des formules (63) et (64) :

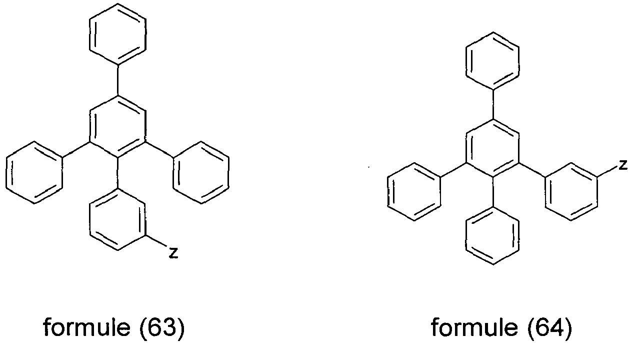

5. Formulation comprenant au moins un composé selon une ou plusieurs des revendications 1 à 4 et au moins un solvant.

6. Composition comprenant au moins l'un des composés selon une ou plusieurs des revendications 1 à 4 et au moins un autre matériau fonctionnel organiquement choisi parmi le groupe constitué par les émetteurs, les matériaux hôtes, les matériaux de matrice, les matériaux de transport d'électrons (ETM), les matériaux d'injection d'électrons (EIM), les matériaux de transport de trous (HTM), les matériaux d'injection de trous (HIM), les matériaux de blocage d'électrons (EBM), les matériaux de blocage de trous (HBM), les matériaux de blocage d'excitons (ExBM), de façon particulièrement préférable, les émetteurs et de façon très particulièrement préférable, les émetteurs fluorescents et/ou phosphorescents.

7. Utilisation d'un composé selon une ou plusieurs des revendications 1 à 4 ou d'une formulation selon la revendication 5 ou d'une composition selon la revendication 6 dans un dispositif électronique, de façon préférable choisi parmi le groupe constitué par les dispositifs électroluminescents organiques (OLED, PLED), les circuits intégrés organiques (O-IC), les transistors à effet de champ organiques (O-FET), les transistors à film mince organiques (O-TFT), les transistors à émission de lumière organiques (O-LET), les cellules solaires organiques (O-SC), les détecteurs optiques organiques, les photorécepteurs organiques, les dispositifs à extinction de champ organiques (O-FQD), les cellules électrochimiques à émission de lumière (LEC) ou les diodes laser organiques (O-lasers).

8. Dispositif électronique, contenant au moins un composé selon une ou plusieurs des revendications 1 à 4 ou une composition selon la revendication 6, de façon préférable choisi parmi le groupe constitué par les dispositifs électroluminescents organiques (OLED, PLED), les circuits intégrés organiques (O-IC), les transistors à effet de champ organiques (O-FET), les transistors à film mince organiques (O-TFT), les transistors à émission de lumière organiques (O-LET), les cellules solaires organiques (O-SC), les détecteurs optiques organiques, les photorécepteurs organiques, les dispositifs à extinction de champ organiques (O-FQD), les cellules électrochimiques à émission de lumière (LEC) ou les diodes laser organiques (O-laser).

9. Dispositif électroluminescent organique selon la revendication 8, **caractérisé en ce que** le composé selon une ou plusieurs des revendications 1 à 4 est utilisé en tant que matériau hôte ou de matrice dans une ou plusieurs couche(s) d'émission, de façon préférable en combinaison avec un émetteur, où l'émetteur est de façon préférable choisi parmi le groupe des émetteurs fluorescents et phosphorescents.

10. Dispositif électroluminescent organique selon la revendication 8 ou 9 pour une utilisation en médecine pour la photothérapie.

11. Utilisation d'un dispositif électroluminescent organique selon la revendication 8 ou 9 pour une utilisation dans le domaine des cosmétiques.
